# EUROPEAN PATENT SPECIFICATION

(11) **EP 2 069 503 B1**
(45) Date of publication and mention of the grant of the patent: **06.01.2016**
(21) Application number: 07845539.1
(22) Date of filing: 15.11.2007
(51) Int. Cl.: C12N 15/62, A61K 35/76, A61K 39/00, A61K 39/002, A61K 39/02, A61K 39/12, A61K 39/385, A61K 39/39, A61K 9/12, A61P 31/04, A61P 31/12

(54) **PAPAYA MOSAIC VIRUS-BASED VACCINES FOR INFLUENZA**
INFLUENZA-IMPFSTOFFE AUF DER BASIS VON PAPMV
VACCINS CONTRE LA GRIPPE A BASE DU VIRUS DE LA MOSAIQUE DU PAPAYER

(30) Priority: 15.11.2006 US 865997 P
(43) Date of publication of application: 17.06.2009
(73) Proprietor: Folia Biotech Inc., Québec City, QC G1P 4R1 (CA)
(72) Inventor: Leclerc, Denis, Fossambault sur le lac, QC G0A 3M0 (CA)
(74) Representative: Litton, Rory Francis
(86) International application number: PCT/CA2007/002069
(87) International publication number: WO 2008/058396

(56) References cited:
- WO-A1-2008/058369
- WO-A2-2004/004761
- WO-A2-2005/055957
- WO-A2-2007/011904
- CANIZARES M C ET AL: "Use of viral vectors for vaccine production in plants" IMMUNOLOGY AND CELL BIOLOGY, CARLTON, AU LNKD- DOI:10.1111/J.1440-1711.2005.01339.X, vol. 83, no. 3, 1 June 2005 (2005-06-01), pages 263-270, XP002344926 ISSN: 0818-9641
- SHORT M N ET AL: "The primary structure of papaya mosaic virus coat protein" VIROLOGY, ACADEMIC PRESS,ORLANDO, US LNKD- DOI:10.1016/0042-6822(86)90395-8, vol. 152, no. 1, 15 July 1986 (1986-07-15) , pages 280-283, XP023055686 ISSN: 0042-6822 [retrieved on 1986-07-15]
- RAJA VARSHA ET AL: "Development of vaccine for hepatitis C (Papaya mosaic virus as an expression vector for foreign epitopes)" RETROVIROLOGY, BIOMED CENTRAL LTD., LONDON, GB LNKD- DOI:10.1186/1742-4690-3-S1-P52, vol. 3, no. Suppl 1, 21 December 2006 (2006-12-21), page P52, XP021025146 ISSN: 1742-4690
- DENIS J ET AL: "Development of a universal influenza A vaccine based on the M2e peptide fused to the papaya mosaic virus (PapMV) vaccine platform" VACCINE, ELSEVIER LTD, GB LNKD- DOI:10.1016/J.VACCINE.2008.04.052, vol. 26, no. 27-28, 25 June 2008 (2008-06-25), pages 3395-3403, XP022710543 ISSN: 0264-410X [retrieved on 2008-05-12]
- DE FILETTE M. ET AL.: 'Improved design and intranasal delivery of an Me2-based human influenza A vaccine' VACCINE vol. 24, no. 44-46, 10 November 2006, pages 6597 - 6601, XP005755539
- LECLERC D. ET AL.: 'Proteasome-independent major histocompatability complex class I cross-presentation mediated by papaya mosaic virus-like particles leads to expansion of specific human T cells' J. VIROL. vol. 81, no. 3, February 2007, pages 1319 - 1326, XP008105174
- LACASSE P. ET AL.: 'Novel plant virus-based vaccine induces protective cytotoxic T-lymphocyte-mediated antiviral immunity through dendritic cell maturation' J. VIROL. vol. 82, no. 2, 08 January 2008, pages 785 - 794, XP008131330
- DENIS J. ET AL.: 'Immunogenicity of papaya mosaic virus-like particles fused to a hepatitis C virus epitope: Evidence for the critical function of multimerization' VIROLOGY vol. 363, no. 1, 20 June 2007, pages 59 - 68, XP022065038
- TREMBLAY M.-H. ET AL.: 'Effect of mutations K97A and E128A on RNA binding and self assembly of papaya mosaic potexvirus coat protein' FEBS J. vol. 273, no. 1, January 2006, pages 14 - 25, XP008105172

### SEQUENCE LISTING

<110> LECLERC, Denis
<120> Papaya Mosaic Virus-Based Vaccines for
   Influenza
<130> 1398-107PCT
<140>
   <141> 2007-11-15
<150> 60/865,997
   <151> 2006-11-15
<160> 66
<170> FastSEQ for Windows Version 4.0
<210> 1
   <211> 215
   <212> PRT
   <213> Papaya mosaic virus
<220>
   <221>
   <222>
   <223> coat protein
<400> 1
   Met Ser Lys Ser Ser Met Ser Thr Pro Asn Ile Ala Phe Pro Ala Ile
    1               5                  10                  15
   Thr Gln Glu Gln Met Ser Ser Ile Lys Val Asp Pro Thr Ser Asn Leu
               20                  25                  30
   Leu Pro Ser Gln Glu Gln Leu Lys Ser Val Ser Thr Leu Met Val Ala
           35                  40                  45
   Ala Lys Val Pro Ala Ala Ser Val Thr Thr Val Ala Leu Glu Leu Val
       50                  55                  60
   Asn Phe Cys Tyr Asp Asn Gly Ser Ser Ala Tyr Thr Thr Val Thr Gly
   65                  70                  75                  80
   Pro Ser Ser Ile Pro Glu Ile Ser Leu Ala Gln Leu Ala Ser Ile Val
                   85                  90                  95
   Lys Ala Ser Gly Thr Ser Leu Arg Lys Phe Cys Arg Tyr Phe Ala Pro
               100                 105                 110
   Ile Ile Trp Asn Leu Arg Thr Asp Lys Met Ala Pro Ala Asn Trp Glu
           115                 120                 125
   Ala Ser Gly Tyr Lys Pro Ser Ala Lys Phe Ala Ala Phe Asp Phe Phe
       130                 135                 140
   Asp Gly Val Glu Asn Pro Ala Ala Met Gln Pro Pro Ser Gly Leu Ile
   145                 150                 155                 160
   Arg Ser Pro Thr Gln Glu Glu Arg Ile Ala Asn Ala Thr Asn Lys Gln
                   165                 170                 175
   Val His Leu Phe Gln Ala Ala Ala Gln Asp Asn Asn Phe Thr Ser Asn
               180                 185                 190
   Ser Ala Phe Ile Thr Lys Gly Gln Ile Ser Gly Ser Thr Pro Thr Ile
           195                 200                 205
   Gln Phe Leu Pro Pro Pro Glu
       210                 215
<210> 2
   <211> 648
   <212> DNA
   <213> Papaya mosaic virus
<400> 2
   atgtctaagt caagtatgtc cacacccaac atagccttcc ccgccatcac ccaggaacag 60
   atgagctcga ttaaggtcga tccaacgtcc aatcttctgc cctcccaaga gcagttaaag 120
   tcagtgtcca ccctcatggt agctgctaag gttccagcag ccagtgttac aactgtggca 180
   ttggagttgg tcaacttctg ctatgacaat gggtccagcg cgtacaccac agtgactggc 240
   ccatcatcaa taccggagat atcactggca caattggcta gtattgtcaa agcttccggc 300
   acttccctta gaaaattctg ccggtacttc gcgccaataa tctggaatct gaggacggac 360
   aaaatggctc ctgccaattg ggaggcttca ggatacaagc caagcgccaa atttgccgcg 420
   ttcgacttct tcgacggggt ggagaatccg gcggccatgc aacccccttc gggactaatc 480
   aggtcgccga cccaggaaga gcggattgcc aatgctacca acaaacaggt gcatctcttc 540
   caagccgcgg cacaggacaa caactttacc agcaactccg ccttcatcac caaaggccaa 600
   atttctgggt caaccccaac catccaattc cttccacccc ccgaataa             648
<210> 3
   <211> 211
   <212> PRT
   <213> Artificial Sequence
<220>
   <223> mutant PapMV coat protein CPdeltaN5
<400> 3
   Met Ala Ser Thr Pro Asn Ile Ala Phe Pro Ala Ile Thr Gln Glu Gln
    1               5                  10                  15
   Met Ser Ser Ile Lys Val Asp Pro Thr Ser Asn Leu Leu Pro Ser Gln
               20                  25                  30
   Glu Gln Leu Lys Ser Val Ser Thr Leu Met Val Ala Ala Lys Val Pro
           35                  40                  45
   Ala Ala Ser Val Thr Thr Val Ala Leu Glu Leu Val Asn Phe Cys Tyr
       50                  55                  60
   Asp Asn Gly Ser Ser Ala Tyr Thr Thr Val Thr Gly Pro Ser Ser Ile
   65                  70                  75                  80
   Pro Glu Ile Ser Leu Ala Gln Leu Ala Ser Ile Val Lys Ala Ser Gly
                   85                  90                  95
   Thr Ser Leu Arg Lys Phe Cys Arg Tyr Phe Ala Pro Ile Ile Trp Asn
               100                 105                 110
   Leu Arg Thr Asp Lys Met Ala Pro Ala Asn Trp Glu Ala Ser Gly Tyr
           115                 120                 125
   Lys Pro Ser Ala Lys Phe Ala Ala Phe Asp Phe Phe Asp Gly Val Glu
       130                 135                 140
   Asn Pro Ala Ala Met Gln Pro Pro Ser Gly Leu Thr Arg Ser Pro Thr
   145                 150                 155                 160
   Gln Glu Glu Arg Ile Ala Asn Ala Thr Asn Lys Gln Val His Leu Phe
                   165                 170                 175
   Gln Ala Ala Ala Gln Asp Asn Asn Phe Ala Ser Asn Ser Ala Phe Ile
               180                 185                 190
   Thr Lys Gly Gln Ile Ser Gly Ser Thr Pro Thr Ile Gln Phe Leu Pro
           195                 200                 205
   Pro Pro Glu
       210
<210> 4
   <211> 244
   <212> PRT
   <213> Artificial Sequence
<220>
   <223> CPdeltaNS-SM fused at its C-terminus to the M2e peptide from influenza virus strain H1N1
<400> 4
   Met Ala Ser Thr Pro Asn Ile Ala Phe Pro Ala Ile Thr Gln Glu Gln
    1               5                  10                  15
   Met Ser Ser Ile Lys Ala Asp Pro Thr Ser Asn Leu Leu Pro Ser Gln
               20                  25                  30
   Glu Gln Leu Lys Ser Val Ser Thr Leu Met Val Ala Ala Lys Val Pro
           35                  40                  45
   Ala Ala Ser Val Thr Thr Val Ala Leu Glu Leu Val Asn Phe Cys Tyr
       50                  55                  60
   Asp Asn Gly Ser Ser Ala Tyr Thr Thr Val Thr Gly Pro Ser Ser Ile
   65                  70                  75                  80
   Gln Glu Ile Ser Leu Ala Gln Leu Ala Ser Ile Val Trp Asn Leu Arg
                   85                  90                  95
   Thr Asp Lys Ala Ser Gly Thr Ser Leu Arg Lys Phe Cys Arg Tyr Phe
               100                 105                 110
   Ala Pro Ile Ile Lys Met Ala Pro Ala Asn Trp Glu Ala Ser Gly Tyr
           115                 120                 125
   Lys Pro Ser Ala Lys Phe Ala Ala Phe Asp Phe Phe Asp Gly Val Glu
       130                 135                 140
   Asn Pro Ala Ala Met Gln Pro Pro Ser Gly Leu Thr Arg Ser Pro Thr
   145                 150                 155                 160
   Gln Glu Glu Arg Ile Ala Asn Ala Thr Asn Lys Gln Val His Leu Phe
                   165                 170                 175
   Gln Ala Ala Ala Gln Asp Asn Asn Phe Ala Ser Asn Ser Ala Phe Ile
               180                 185                 190
   Thr Lys Gly Gln Ile Ser Gly Ser Thr Pro Thr Ile Gln Phe Leu Pro
           195                 200                 205
   Pro Pro Glu Thr Ser Ser Leu Leu Thr Glu Val Glu Thr Pro Ile Arg
       210                 215                 220
   Asn Glu Trp Gly Cys Arg Cys Asn Asp Ser Ser Asp Thr Arg His His
   225                 230                 235                 240
   His His His His
<210> 5
   <211> 744
   <212> DNA
   <213> Artificial Sequence
<220>
   <223> nucleotide sequence encoding CPdeltaN5-SM fused at its C-terminus to the M2e peptide from influenza virus strain H1N1
<400> 5
   atggcatcca cacccaacat agccttcccc gccatcaccc aggaacaaat gagctcgatt 60
   aaggccgatc caacgtccaa tcttctgccc tcccaagagc agttaaagtc agtgtccacc 120
   ctcatggtag ctgctaaggt tccagcagcc agtgttacaa ctgtggcatt ggagttggtt 180
   aacttctgct atgacaatgg gtccagcgcg tacaccacag tgactggccc atcatcaata 240
   caggagatat cactggcaca attggccagc attgtctgga atctgaggac ggacaaagct 300
   tccggcactt cccttaggaa attctgccgg tacttcgcgc caataatcaa aatggctcct 360
   gccaattggg aggcctcagg atacaagcca agcgccaaat ttgccgcgtt cgacttcttc 420
   gacggggtgg agaatccggc ggccatgcaa cccccttcgg gactaaccag gtcgccgacc 480
   caggaagagc ggattgccaa tgccaccaac aaacaggtgc atctcttcca agccgcggca 540
   caggacaaca actttgccag caactccgcc ttcatcacca aaggccaaat ttctgggtca 600
   acccccacca tccaattcct tccacccccc gaaactagtt ccctgctgac cgaagtggaa 660
   accccgattc gcaacgaatg gggctgccgc tgcaacgatt cctccgatac gcgtcaccat 720
   caccatcacc attagtaagg atcc                                        744
<210> 6
   <211> 23
   <212> PRT
   <213> Artificial Sequence
<220>
   <223> M2e peptide from influenza strain Human H1N1 A/USRR/90/77 and A/WSN/33
<400> 6.
   Ser Leu Leu Thr Glu Val Glu Thr Pro Ile Arg Asn Glu Trp Gly Cys
    1              5                   10                  15
   Arg Cys Asn Asp Ser Ser Asp
               20
<210> 7
   <211> 23
   <212> PRT
   <213> Artificial Sequence
<220>
   <223> M2e peptide from influenza virus
<400> 7
   Ser Leu Leu Thr Glu Val Glu Thr Pro Ile Arg Asn Glu Trp Gly Cys
    1              5                   10                  15
   Arg Cys Asn Gly Ser Ser Asp
               20
<210> 8
   <211> 23
   <212> PRT
   <213> Artificial Sequence
<220>
   <223> M2e peptide from influenza virus
<400> 8
   Ser Leu Leu Thr Glu Val Glu Thr Pro Thr Lys Asn Glu Trp Asp Cys
    1              5                   10                  15
   Arg Cys Asn Asp Ser Ser Asp
               20
<210> 9
   <211> 23
   <212> PRT
   <213> Artificial Sequence
<220>
   <223> M2e peptide from influenza strain Equine H3N8 A/equine/Massachussetts/213/2003
<400> 9
   Ser Leu Leu Thr Glu Val Glu Thr Pro Thr Arg Asn Gly Trp Glu Cys
    1              5                   10                  15
   Lys Cys Ser Asp Ser Ser Asp
               20
<210> 10
   <211> 23
   <212> PRT
   <213> Artificial Sequence
<220>
   <223> M2e peptide from influenza strain H5N1 A/Vietnam/1196/04
<400> 10
   Ser Leu Leu Thr Glu Val Glu Thr Pro Thr Arg Asn Glu Trp Glu Cys
   1               5                   10                  15
   Arg Cys Ser Asp Ser Ser Asp
               20
<210> 11
   <211> 8
   <212> PRT
   <213> Artificial Sequence
<220>
   <223> M2e epitope from influenza virus
<400> 11
   Glu Val Glu Thr Pro Ile Arg Asn
<210> 12
   <211> 8
   <212> PRT
   <213> Artificial Sequence
<220>
   <223> M2e epitope from influenza virus
<400> 12
   Glu Val Glu Thr Leu Thr Arg Asn
   1               5
<210> 13
   <211> 8
   <212> PRT
   <213> Artificial Sequence
<220>
   <223> M2e epitope from influenza virus
<400> 13
   Glu Val Glu Thr Pro Ile Arg Ser
   1               5
<210> 14
   <211> 8
   <212> PRT
   <213> Artificial Sequence
<220>
   <223> M2e epitope from influenza virus
<400> 14
   Glu Val Glu Thr Pro Thr Arg Asn
   1               5
<210> 15
   <211> 8
   <212> PRT
   <213> Artificial Sequence
<220>
   <223> M2e epitope from influenza virus
<400> 15
   Glu Val Glu Thr Pro Thr Lys Asn
   1               5
<210> 16
   <211> 8
   <212> PRT
   <213> Artificial Sequence
<220>
   <223> M2e epitope from influenza virus
<400> 16
   Glu Val Asp Thr Leu Thr Arg Asn
   1               5
<210> 17
   <211> 8
   <212> PRT
   <213> Artificial Sequence
<220>
   <223> M2e epitope from influenza virus
<400> 17
   Glu Val Glu Thr Pro Ile Arg Lys
   1               5
<210> 18
   <211> 8
   <212> PRT
   <213> Artificial Sequence
<220>
   <223> M2e epitope from influenza virus
<400> 18
   Glu Val Glu Thr Leu Thr Lys Asn
   1               5
<210> 19
   <211> 30
   <212> DNA
   <213> Artificial Sequence
<220>
   <223> primer
<400> 19
   agtcccatgg atccaacgtc caatcttctg 30
<210> 20
   <211> 49
   <212> DNA
   <213> Artificial Sequence
<220>
   <223> primer
<400> 20
   atgcggatcc ttactaatgg tgatggtgat ggtgttcggg gggtggaag 49
<210> 21
   <211> 9
   <212> PRT
   <213> Artificial Sequence
<220>
   <223> HLA-A*0201 epitope from tumor antigen gp100
<400> 21
   Ile Met Asp Gln Val Pro Phe Ser Val
   1               5
<210> 22
   <211> 9
   <212> PRT
   <213> Artificial Sequence
<220>
   <223> HLA-A*0201 epitope from influenza M1 protein
<400> 22
   Gly Ile Leu Gly Phe Val Phe Thr Leu
   1 5
<210> 23
   <211> 63
   <212> DNA
   <213> Artificial Sequence
<220>
   <223> oligonucleotide
<400> 23
   ctagttcttc tgcgttcacc atcatggacc aggttccgtt ctctgtttct gtttctcagc 60
   tga 63
<210> 24
   <211> 63
   <212> DNA
   <213> Artificial Sequence
<220>
   <223> oligonucleotide
<400> 24
   ctagtcagct gagaaacaga aacagagaac ggaacctggt ccatgatggt gaacgcagaa 60
   gaa 63
<210> 25
   <211> 63
   <212> DNA
   <213> Artificial Sequence
<220>
   <223> oligonucleotide
<400> 25
   ctagttctcc gctgaccaaa ggtatcctgg gtttcgtttt caccctgacc gttccgtctg 60
   aaa 63
<210> 26
   <211> 63
   <212> DNA
   <213> Artificial Sequence
<220>
   <223> oligonucleotide
<400> 26
   ctagtttcag acggaacggt cagggtgaaa acgaaaccca ggataccttt ggtcagcgga 60
   gaa 63
<210> 27
   <211> 9
   <212> PRT
   <213> Artificial Sequence
<220>
   <223> HLA-A*0201-restricted epitope from tumour antigen gp100
<400> 27
   Ile Thr Asp Gln Val Pro Phe Ser Val
   1 5
<210> 28
   <211> 33
   <212> DNA
   <213> Artificial Sequence
<220>
   <223> primer
<400> 28
   agtcccatgg catccacacc caacatagcc ttc 33
<210> 29
   <211> 79
   <212> DNA
   <213> Artificial Sequence
<220>
   <223> primer
<400> 29
   gatcggatcc ttactaatgg tgatggtgat ggtgacgcgt ggtactagtt tcggggggtg 60
   gaaggaattg gatggttgg 79
<210> 30
   <211> 75
   <212> DNA
   <213> Artificial Sequence
<220>
   <223> oligonucleotide
<400> 30
   ctagttccct gctgaccgaa gtggaaaccc cgattcgcaa cgaatggggc tgccgctgca 60
   acgattcctc cgata 75
<210> 31
   <211> 75
   <212> DNA
   <213> Artificial Sequence
<220>
   <223> oligonucleotide
<400> 31
   cgcgtatcgg aggaatcgtt gcagcggcag ccccattcgt tgcgaatcgg ggtttccact 60
   tcggtcagca gggaa                                                  75
<210> 32
   <211> 80
   <212> DNA
   <213> Artificial Sequence
<220>
   <223> oligonucleotide
<400> 32
   gatcactagt gtggtggtgg gtaccaccga tcgtagcggt gcgccgacct acagctgggg 60
   tgcgaacgat acgcgtcatg                                             80
<210> 33
   <211> 80
   <212> DNA
   <213> Artificial Sequence
<220>
   <223> oligonucleotide
<400> 33
   catgacgcgt atcgttcgca ccccagctgt aggtcggcgc accgctacga tcggtggtac 60
   ccaccaccac actagtgatc                                             80
<210> 34
   <211> 33
   <212> DNA
   <213> Artificial Sequence
<220>
   <223> primer
<400> 34
   agtcccatgg ccgatccaac gtccaatctt ctg 33
<210> 35
   <211> 28
   <212> DNA
   <213> Artificial Sequence
<220>
   <223> primer
<400> 35
   acgtccatgg tatatctcct tcttaaag 28
<210> 36
   <211> 7
   <212> PRT
   <213> Artificial Sequence
<220>
   <223> phage display peptide
<400> 36
   Asp Pro Ala Pro Arg Pro Arg
   1 5
<210> 37
   <211> 7
   <212> PRT
   <213> Artificial Sequence
<220>
   <223> phage display peptide
<400> 37
   Leu Leu Asn Ser His Ala Val
   1 5
<210> 38
   <211> 7
   <212> PRT
   <213> Artificial Sequence
<220>
   <223> phage display peptide
<400> 38
   Asn His Ala His Ser Thr Pro
   1 5
<210> 39
   <211> 7
   <212> PRT
   <213> Artificial Sequence
<220>
   <223> phage display peptide
<400> 39
   Lys Ala Leu Gly Asp Asn Gly
   1 5
<210> 40
   <211> 24
   <212> PRT
   <213> Artificial Sequence
<220>
   <223> M2e peptide from influenza strain Human H1N1 A/USRR/90/77 and A/WSN/33
<400> 40
   Met Ser Leu Leu Thr Glu Val Glu Thr Pro Ile Arg Asn Glu Trp Gly
   1 5 10 15
   Cys Arg Cys Asn Asp Ser Ser Asp
   20
<210> 41
   <211> 24
   <212> PRT
   <213> Artificial Sequence
<220>
   <223> M2e peptide from influenza strain Avian H5N1 A/Vietnam/1196/04
<400> 41
   His Ser Leu Leu Thr Glu Val Glu Thr Pro Thr Arg Asn Glu Trp Glu
   1 5 10 15
   Cys Arg Cys Ser Asp Ser Ser Asp
   20
<210> 42
   <211> 24
   <212> PRT
   <213> Artificial Sequence
<220>
   <223> M2e peptide from influenza strain H3N8, Horse-Dog A/equine/Massachussetts/213/2003
<400> 42
   Met Ser Leu Leu Thr Glu Val Glu Thr Pro Thr Arg Asn Gly Trp Glu
   1 5 10 15
   Cys Lys Cys Ser Asp Ser Ser Asp
   20
<210> 43
   <211> 24
   <212> PRT
   <213> Artificial Sequence
<220>
   <223> M2e peptide from influenza strain H9N2, A/chicken/Osaka/aq69/2001
<400> 43
   Met Ser Leu Leu Thr Glu Val Glu Thr Pro Thr Arg Asn Gly Trp Gly
    1               5                  10                  15
   Cys Arg Cys Ser Asp Ser Ser Asp
               20
<210> 44
   <211> 24
   <212> PRT
   <213> Artificial Sequence
<220>
   <223> M2e peptide from mutant influenza strain H1N1 I/T
<400> 44
   Met Ser Leu Leu Thr Glu Val Glu Thr Pro Thr Arg Asn Glu Trp Gly
    1               5                  10                  15
   Cys Arg Cys Ser Asp Ser Ser Asp
               20
<210> 45
   <211> 79
   <212> DNA
   <213> Artificial Sequence
<220>
   <223> primer
<400> 45
   gatcggatcc ttactaatgg tgatggtgat ggtgacgcgt ggtactagtt tcggggggtg 60
   gaaggaattg gatggttgg                                              79
<210> 46
   <211> 222
   <212> PRT
   <213> Artificial Sequence
<220>
   <223> PapMV coat protein CPdeltaN5 containing the restriction sites SpeI-MluI at the C-terminus (CPdeltaN5-SM)
<400> 46
   Met Ala Ser Thr Pro Asn Ile Ala Phe Pro Ala Ile Thr Gln Glu Gln
    1               5                  10                  15
   Met Ser Ser Ile Lys Val Asp Pro Thr Ser Asn Leu Leu Pro Ser Gln
               20                  25                  30
   Glu Gln Leu Lys Ser Val Ser Thr Leu Met Val Ala Ala Lys Val Pro
           35                  40                  45
   Ala Ala Ser Val Thr Thr Val Ala Leu Glu Leu Val Asn Phe Cys Tyr
       50                  55                  60
   Asp Asn Gly Ser Ser Ala Tyr Thr Thr Val Thr Gly Pro Ser Ser Ile
   65                  70                  75                  80
   Pro Glu Ile Ser Leu Ala Gln Leu Ala Ser Ile Val Lys Ala Ser Gly
                   85                  90                  95
   Thr Ser Leu Arg Lys Phe Cys Arg Tyr Phe Ala Pro Ile Ile Trp Asn
               100                 105                 110
   Leu Arg Thr Asp Lys Met Ala Pro Ala Asn Trp Glu Ala Ser Gly Tyr
           115                 120                 125
   Lys Pro Ser Ala Lys Phe Ala Ala Phe Asp Phe Phe Asp Gly Val Glu
       130                 135                 140
   Asn Pro Ala Ala Met Gln Pro Pro Ser Gly Leu Thr Arg Ser Pro Thr
   145                 150                 155                 160
   Gln Glu Glu Arg Ile Ala Asn Ala Thr Asn Lys Gln Val His Leu Phe
                   165                 170                 175
   Gln Ala Ala Ala Gln Asp Asn Asn Phe Ala Ser Asn Ser Ala Phe Ile
               180                 185                 190
   Thr Lys Gly Gln Ile Ser Gly Ser Thr Pro Thr Ile Gln Phe Leu Pro
           195                 200                 205
   Pro Pro Glu Thr Ser Thr Thr Arg His His His His His His
       210                 215                 220
<210> 47
   <211> 244
   <212> PRT
   <213> Artificial Sequence
<220>
   <223> CPdeltaN5-SM fused at its C-terminus to the M2e peptide from influenza virus strain H9N2
<400> 47
   Met Ala Ser Thr Pro Asn Ile Ala Phe Pro Ala Ile Thr Gln Glu Gln
    1               5                  10                  15
   Met Ser Ser Ile Lys Val Asp Pro Thr Ser Asn Leu Leu Pro Ser Gln
               20                  25                  30
   Glu Gln Leu Lys Ser Val Ser Thr Leu Met Val Ala Ala Lys Val Pro
           35                  40                  45
   Ala Ala Ser Val Thr Thr Val Ala Leu Glu Leu Val Asn Phe Cys Tyr
       50                  55                  60
   Asp Asn Gly Ser Ser Ala Tyr Thr Thr Val Thr Gly Pro Ser Ser Ile
   65                  70                  75                  80
   Pro Glu Ile Ser Leu Ala Gln Leu Ala Ser Ile Val Lys Ala Ser Gly
                   85                  90                  95
   Thr Ser Leu Arg Lys Phe Cys Arg Tyr Phe Ala Pro Ile Ile Trp Asn
               100                 105                 110
   Leu Arg Thr Asp Lys Met Ala Pro Ala Asn Trp Glu Ala Ser Gly Tyr
           115                 120                 125
   Lys Pro Ser Ala Lys Phe Ala Ala Phe Asp Phe Phe Asp Gly Val Glu
       130                 135                 140
   Asn Pro Ala Ala Met Gln Pro Pro Ser Gly Leu Thr Arg Ser Pro Thr
   145                 150                 155                 160
   Gln Glu Glu Arg Ile Ala Asn Ala Thr Asn Lys Gln Val His Leu Phe
                   165                 170                 175
   Gln Ala Ala Ala Gln Asp Asn Asn Phe Ala Ser Asn Ser Ala Phe Ile
               180                 185                 190
   Thr Lys Gly Gln Ile Ser Gly Ser Thr Pro Thr Ile Gln Phe Leu Pro
           195                 200                 205
   Pro Pro Glu Thr Ser Ser Leu Leu Thr Glu Val Glu Thr Pro Thr Arg
       210                 215                 220
   Asn Gly Trp Gly Cys Arg Cys Ser Asp Ser Ser Asp Thr Arg His His
   225                 230                 235                 240
   His His His His
<210> 48
   <211> 252
   <212> PRT
   <213> Artificial Sequence
<220>
   <223> CPdeltaN5-SM fused at its C-terminus to a glycine spacer and the M2e peptide from influenza virus strain H9N2
<400> 48
   Met Ala Ser Thr Pro Asn Ile Ala Phe Pro Ala Ile Thr Gln Glu Gln
    1              5                   10                  15
   Met Ser Ser Ile Lys Val Asp Pro Thr Ser Asn Leu Leu Pro Ser Gln
               20                  25                  30
   Glu Gln Leu Lys Ser Val Ser Thr Leu Met Val Ala Ala Lys Val Pro
           35                  40                  45
   Ala Ala Ser Val Thr Thr Val Ala Leu Glu Leu Val Asn Phe Cys Tyr
       50                  55                  60
   Asp Asn Gly Ser Ser Ala Tyr Thr Thr Val Thr Gly Pro Ser Ser Ile
   65                  70                  75                  80
   Pro Glu Ile Ser Leu Ala Gln Leu Ala Ser Ile Val Lys Ala Ser Gly
                   85                  90                  95
   Thr Ser Leu Arg Lys Phe Cys Arg Tyr Phe Ala Pro Ile Ile Trp Asn
               100                 105                 110
   Leu Arg Thr Asp Lys Met Ala Pro Ala Asn Trp Glu Ala Ser Gly Tyr
           115                 120                 125
   Lys Pro Ser Ala Lys Phe Ala Ala Phe Asp Phe Phe Asp Gly Val Glu
       130                 135                 140
   Asn Pro Ala Ala Met Gln Pro Pro Ser Gly Leu Thr Arg Ser Pro Thr
   145                 150                 155                 160
   Gln Glu Glu Arg Ile Ala Asn Ala Thr Asn Lys Gln Val His Leu Phe
                   165                 170                 175
   Gln Ala Ala Ala Gln Asp Asn Asn Phe Ala Ser Asn Ser Ala Phe Ile
               180                 185                 190
   Thr Lys Gly Gln Ile Ser Gly Ser Thr Pro Thr Ile Gln Phe Leu Pro
           195                 200                 205
   Pro Pro Glu Thr Ser Gly Gly Gly Leu Leu Leu Thr Ser Ser Leu Leu
       210                 215                 220
   Thr Glu Val Glu Thr Pro Thr Arg Asn Glu Trp Gly Cys Arg Cys Asn
   225                 230                 235                 240
   Asp Ser Ser Asp Thr Arg His His His His His His
                   245                 250
<210> 49
   <211> 669
   <212> DNA
   <213> Artificial Sequence
<220>
   <223> nucleotide sequence encoding PapMV coat protein CPdeltaN5 containing the restriction sites SpeI-MluI at the C-terminus (CPdeltaN5-SM)
<400> 49
   atggcatcca cacccaacat agccttcccc gccatcaccc aggaacaaat gagctcgatt 60
   aaggtcgatc caacgtccaa tcttctgccc tcccaagagc agttaaagtc agtgtccacc 120
   ctcatggtag ctgctaaggt tccagcagcc agtgttacaa ctgtggcatt ggagttggtt 180
   aacttctgct atgacaatgg gtccagcgcg tacaccacag tgactggccc atcatcaata 240
   ccggagatat cactggcaca attggccagc attgtcaaag cttccggcac ttcccttagg 300
   aaattctgcc ggtacttcgc gccaataatc tggaatctga ggacggacaa aatggctcct 360
   gccaattggg aggcctcagg atacaagcca agcgccaaat ttgccgcgtt cgacttcttc 420
   gacggggtgg agaatccggc ggccatgcaa cccccttcgg gactaaccag gtcgccgacc 480
   caggaagagc ggattgccaa tgccaccaac aaacaggtgc atctcttcca agccgcggca 540
   caggacaaca actttgccag caactccgcc ttcatcacca aaggccaaat ttctgggtca 600
   accccaacca tccaattcct tccacccccc gaaactagta ccacgcgtca ccatcaccat 660
   caccattag                                                         669
<210> 50
   <211> 75
   <212> DNA
   <213> Artificial Sequence
<220>
   <223> oligonucleotide
<400> 50
   ctagttccct gctgaccgaa gtggaaaccc cgacccgcaa cggttggggc tgccgctgct 60
   ctgattcctc cgata                                                   75
<210> 51
   <211> 75
   <212> DNA
   <213> Artificial Sequence
<220>
   <223> oligonucleotide
<400> 51
   cgcgtatcgg aggaatcaga gcagcggcag ccccaaccgt tgcgggtcgg ggtttccact 60
   tcggtcagca gggaa                                                  75
<210> 52
   <211> 24
   <212> DNA
   <213> Artificial Sequence
<220>
   <223> oligonucleotide
<400> 52
   ctagtggtgg cggtctgctg ctga 24
<210> 53
   <211> 24
   <212> DNA
   <213> Artificial Sequence
<220>
   <223> oligonucleotide
<400> 53
   ctagtcagca gcagaccgcc acca 24
<210> 54
   <211> 75
   <212> DNA
   <213> Artificial Sequence
<220>
   <223> oligonucleotide
<400> 54
   ctagttccct gctgaccgaa gtggaaaccc cgacccgcaa cggttgggaa tgcaaatgct 60
   ctgattcctc cgata                                                  75
<210> 55
   <211> 75
   <212> DNA
   <213> Artificial Sequence
<220>
   <223> oligonucleotide
<400> 55
   cgcgtatcgg aggaatcaga gcatttgcat tcccaaccgt tgcgggtcgg ggtttccact 60
   tcggtcagca gggaa                                                  75
<210> 56
   <211> 735
   <212> DNA
   <213> Artificial Sequence
<220>
   <223> nucleotide sequence encoding PapMV coat protein fused at its C-terminus to the M2e peptide from influenza virus strain H3N8
<400> 56
   atggcatcca cacccaacat agccttcccc gccatcaccc aggaacaaat gagctcgatt 60
   aaggtcgatc caacgtccaa tcttctgccc tcccaagagc agttaaagtc agtgtccacc 120
   ctcatggtag ctgctaaggt tccagcagcc agtgttacaa ctgtggcatt ggagttggtt 180
   aacttctgct atgacaatgg gtccagcgcg tacaccacag tgactggccc atcatcaata 240
   ccggagatat cactggcaca attggccagc attgtcaaag cttccggcac ttcccttagg 300
   aaattctgcc ggtacttcgc gccaataatc tggaatctga ggacggacaa aatggctcct 360
   gccaattggg aggcctcagg atacaagcca agcgccaaat ttgccgcgtt cgacttcttc 420
   gacggggtgg agaatccggc ggccatgcaa cccccttcgg gactaaccag gtcgccgacc 480
   caggaagagc ggattgccaa tgccaccaac aaacaggtgc atctcttcca agccgcggca 540
   caggacaaca actttgccag caactccgcc ttcatcacca aaggccaaat ttctgggtca 600
   accccaacca tccaattcct tccacccccc gaaactagtt ccctgctgac cgaagtggaa 660
   accccgaccc gcaacggttg ggaatgcaaa tgctctgatt cctccgatac gcgtcaccat 720
   caccatcacc attag                                                  735
<210> 57
   <211> 244
   <212> PRT
   <213> Artificial Sequence
<220>
   <223> PapMV coat protein fused at its C-terminus to the M2e peptide from influenza virus strain H3N8
<400> 57
   Met Ala Ser Thr Pro Asn Ile Ala Phe Pro Ala Ile Thr Gln Glu Gln
    1               5                  10                  15
   Met Ser Ser Ile Lys Val Asp Pro Thr Ser Asn Leu Leu Pro Ser Gln
               20                  25                  30
   Glu Gln Leu Lys Ser Val Ser Thr Leu Met Val Ala Ala Lys Val Pro
           35                  40                  45
   Ala Ala Ser Val Thr Thr Val Ala Leu Glu Leu Val Asn Phe Cys Tyr
       50                  55                  60
   Asp Asn Gly Ser Ser Ala Tyr Thr Thr Val Thr Gly Pro Ser Ser Ile
   65                  70                  75                  80
   Pro Glu Ile Ser Leu Ala Gln Leu Ala Ser Ile Val Lys Ala Ser Gly
                   85                  90                  95
   Thr Ser Leu Arg Lys Phe Cys Arg Tyr Phe Ala Pro Ile Ile Trp Asn
               100                 105                 110
   Leu Arg Thr Asp Lys Met Ala Pro Ala Asn Trp Glu Ala Ser Gly Tyr
           115                 120                 125
   Lys Pro Ser Ala Lys Phe Ala Ala Phe Asp Phe Phe Asp Gly Val Glu
       130                 135                 140
   Asn Pro Ala Ala Met Gln Pro Pro Ser Gly Leu Thr Arg Ser Pro Thr
   145                 150                 155                 160
   Gln Glu Glu Arg Ile Ala Asn Ala Thr Asn Lys Gln Val His Leu Phe
                   165                 170                 175
   Gln Ala Ala Ala Gln Asp Asn Asn Phe Ala Ser Asn Ser Ala Phe Ile
               180                 185                 190
   Thr Lys Gly Gln Ile Ser Gly Ser Thr Pro Thr Ile Gln Phe Leu Pro
           195                 200                 205
   Pro Pro Glu Thr Ser Ser Leu Leu Thr Glu Val Glu Thr Pro Thr Arg
       210                 215                 220
   Asn Gly Trp Glu Cys Lys Cys Ser Asp Ser Ser Asp Thr Arg His His
   225                 230                 235                 240
   His His His His
<210> 58
   <211> 735
   <212> DNA
   <213> Artificial Sequence
<220>
   <223> nucleotide sequence encoding CPdeltaN5-SM fused at its C-terminus to the M2e peptide from influenza virus strain H5N1
<400> 58
   atggcatcca cacccaacat agccttcccc gccatcaccc aggaacaaat gagctcgatt 60
   aaggtcgatc caacgtccaa tcttctgccc tcccaagagc agttaaagtc agtgtccacc 120
   ctcatggtag ctgctaaggt tccagcagcc agtgttacaa ctgtggcatt ggagttggtt 180
   aacttctgct atgacaatgg gtccagcgcg tacaccacag tgactggccc atcatcaata 240
   ccggagatat cactggcaca attggccagc attgtcaaag cttccggcac ttcccttagg 300
   aaattctgcc ggtacttcgc gccaataatc tggaatctga ggacggacaa aatggctcct 360
   gccaattggg aggcctcagg atacaagcca agcgccaaat ttgccgcgtt cgacttcttc 420
   gacggggtgg agaatccggc ggccatgcaa cccccttcgg gactaaccag gtcgccaacc 480
   caggaagagc ggattgccaa tgccaccaac aaacaggtgc atctcttcca agccgcggca 540
   caggacaaca actttgccag caactccgcc ttcatcacca aaggccaaat ttctgggtca 600
   accccaacca tccaattcct tccacccccc gaaactagtt ccctgctgac cgaagtggaa 660
   accccgaccc gcaacgaatg ggaatgccgc tgctctgatt cctccgatac gcgtcaccat 720
   caccatcacc attag                                                  735
<210> 59
   <211> 244
   <212> PRT
   <213> Artificial Sequence
<220>
   <223> CPdeltaN5-SM fused at its C-terminus to the M2e peptide from influenza virus strain H5N1
<400> 59
   Met Ala Ser Thr Pro Asn Ile Ala Phe Pro Ala Ile Thr Gln Glu Gln
    1               5                  10                  15
   Met Ser Ser Ile Lys Val Asp Pro Thr Ser Asn Leu Leu Pro Ser Gln
           20                  25                  30
   Glu Gln Leu Lys Ser Val Ser Thr Leu Met Val Ala Ala Lys Val Pro
           35                  40                  45
   Ala Ala Ser Val Thr Thr Val Ala Leu Glu Leu Val Asn Phe Cys Tyr
       50                  55                  60
   Asp Asn Gly Ser Ser Ala Tyr Thr Thr Val Thr Gly Pro Ser Ser Ile
   65                  70                  75                  80
   Pro Glu Ile Ser Leu Ala Gln Leu Ala Ser Ile Val Lys Ala Ser Gly
                   85                  90                  95
   Thr Ser Leu Arg Lys Phe Cys Arg Tyr Phe Ala Pro Ile Ile Trp Asn
               100                 105                 110
   Leu Arg Thr Asp Lys Met Ala Pro Ala Asn Trp Glu Ala Ser Gly Tyr
           115                 120                 125
   Lys Pro Ser Ala Lys Phe Ala Ala Phe Asp Phe Phe Asp Gly Val Glu
       130                 135                 140
   Asn Pro Ala Ala Met Gln Pro Pro Ser Gly Leu Thr Arg Ser Pro Thr
   145                 150                 155                 160
   Gln Glu Glu Arg Ile Ala Asn Ala Thr Asn Lys Gln Val His Leu Phe
                   165                 170                 175
   Gln Ala Ala Ala Gln Asp Asn Asn Phe Ala Ser Asn Ser Ala Phe Ile
               180                 185                 190
   Thr Lys Gly Gln Ile Ser Gly Ser Thr Pro Thr Ile Gln Phe Leu Pro
           195                 200                 205
   Pro Pro Glu Thr Ser Ser Leu Leu Thr Glu Val Glu Thr Pro Thr Arg
       210                 215                 220
   Asn Glu Trp Glu Cys Arg Cys Ser Asp Ser Ser Asp Thr Arg His His
   225                 230                 235                 240
   His His His His
<210> 60
   <211> 75
   <212> DNA
   <213> Artificial Sequence
<220>
   <223> oligonucleotide
<400> 60
   ctagttccct gctgaccgaa gtggaaaccc cgacccgcaa cgaatgggaa tgccgctgct 60
   ctgattcctc cgata                                                  75
<210> 61
   <211> 75
   <212> DNA
   <213> Artificial Sequence
<220>
   <223> oligonucleotide
<400> 61
   cgcgtatcgg aggaatcaga gcagcggcat tcccattcgt tgcgggtcgg ggtttccact 60
   tcggtcagca gggaa                                                  75
<210> 62
   <211> 23
   <212> PRT
   <213> Artificial Sequence
<220>
   <223> M2e peptide fragment from mutant H1N1 influenza strain I/T
<400> 62
   Ser Leu Leu Thr Glu Val Glu Thr Pro Thr Arg Asn Glu Trp Gly Cys
   1               5                   10                  15
   Arg Cys Ser Asp Ser Ser Asp
               20
<210> 63
   <211> 1497
   <212> DNA
   <213> Artificial Sequence
<220>
   <223> nucleotide sequence encoding NP protein from influenza virus strain A/WSN/33
<400> 63
   atggcgacca aaggcaccaa acgatcttac gaacagatgg agactgatgg agaacgccag 60
   aatgccactg aaatcagagc atctgtcgga aaaatgattg gtggaattgg acgattctac 120
   atccaaatgt gcaccgaact taaactcagt gattatgagg gacggctgat tcagaacagc 180
   ttaacaatag agagaatggt gctctctgct tttgacgaga ggaggaataa atatctagaa 240
   gaacatccca gtgcggggaa agatcctaag aaaactggag gacctatata caggagagta 300
   gatggaaagt ggatgagaga actcatcctt tatgacaaag aagaaataag acgaatctgg 360
   cgccaagcta ataatggtga cgatgcaacg gctggtctga ctcacatgat gatctggcac 420
   tccaatttga atgatgcaac ttaccagagg acaagagctc ttgttcgcac aggaatggat 480
   cccaggatgt gctcactgat gcagggttca accctcccta ggaggtctgg ggccgcaggt 540
   gctgcagtca aaggagttgg aacaatggtg atggaattga tcagaatgat caaacgtggg 600
   atcaatgatc ggaacttctg gaggggtgag aatggacgga gaacaaggat tgcttatgaa 660
   agaatgtgca acattctcaa agggaaattt caaacagctg cacaaagagc aatggtggat 720
   caagtgagag agagccggaa tccaggaaat gctgagttcg aagatctcat ctttctagca 780
   cggtctgcac tcatattgag agggtcagtt gctcacaagt cctgcctgcc tgcctgtgtg 840
   tatggacctg ccgtagccag tggatacgac tttgaaagag agggatactc tctagtcgga 900
   atagaccctt tcagactgct tcaaaacagc caagtataca gcctaatcag accaaatgag 960
   aatccagcac acaagagtca actggtgtgg atggcatgcc attctgctgc atttgaagat 1020
   ctaagagtat caagcttcat cagagggacg aaagtggtcc caagagggaa gctttccact 1080
   agaggagttc aaattgcttc caatgaaaac atggagacta tggaatcaag tacccttgaa 1140
   ctgagaagca gatactgggc cataaggacc agaagtggag ggaacaccaa tcaacagagg 1200
   gcttcctcgg gccaaatcag catacaacct acgttctcag tacagagaaa tctccctttt 1260
   gacagaccaa ccattatggc agcattcact gggaatacag aggggagaac atctgacatg 1320
   agaaccgaaa tcataaggct gatggaaagt gcaagaccag aagatgtgtc tttccagggg 1380
   cggggagtct tcgagctctc ggacgaaaag gcagcgagcc cgatcgtgcc ctcctttgac 1440
   atgagtaatg aaggatctta tttcttcgga gacaatgcag aggagtacga caattaa    1497
<210> 64
   <211> 498
   <212> PRT
   <213> Artificial Sequence
<220>
   <223> NP protein from influenza virus strain A/WSN/33
<400> 64
   Met Ala Thr Lys Gly Thr Lys Arg Ser Tyr Glu Gln Met Glu Thr Asp
   1               5                   10                  15
   Gly Glu Arg Gln Asn Ala Thr Glu Ile Arg Ala Ser Val Gly Lys Met
               20                  25                  30
   Ile Gly Gly Ile Gly Arg Phe Tyr Ile Gln Met Cys Thr Glu Leu Lys
           35                  40                  45
   Leu Ser Asp Tyr Glu Gly Arg Leu Ile Gln Asn Ser Leu Thr Ile Glu
       50                  55                  60
   Arg Met Val Leu Ser Ala Phe Asp Glu Arg Arg Asn Lys Tyr Leu Glu
   65                  70                  75                  80
   Glu His Pro Ser Ala Gly Lys Asp Pro Lys Lys Thr Gly Gly Pro Ile
                   85                  90                  95
   Tyr Arg Arg Val Asp Gly Lys Trp Met Arg Glu Leu Ile Leu Tyr Asp
               100                 105                 110
   Lys Glu Glu Ile Arg Arg Ile Trp Arg Gln Ala Asn Asn Gly Asp Asp
           115                 120                 125
   Ala Thr Ala Gly Leu Thr His Met Met Ile Trp His Ser Asn Leu Asn
       130                 135                 140
   Asp Ala Thr Tyr Gln Arg Thr Arg Ala Leu Val Arg Thr Gly Met Asp
   145                 150                 155                 160
   Pro Arg Met Cys Ser Leu Met Gln Gly Ser Thr Leu Pro Arg Arg Ser
                   165                 170                 175
   Gly Ala Ala Gly Ala Ala Val Lys Gly Val Gly Thr Met Val Met Glu
               180                 185                 190
   Leu Ile Arg Met Ile Lys Arg Gly Ile Asn Asp Arg Asn Phe Trp Arg
           195                 200                 205
   Gly Glu Asn Gly Arg Arg Thr Arg Ile Ala Tyr Glu Arg Met Cys Asn
       210                 215                 220
   Ile Leu Lys Gly Lys Phe Gln Thr Ala Ala Gln Arg Ala Met Val Asp
   225                 230                 235                 240
   Gln Val Arg Glu Ser Arg Asn Pro Gly Asn Ala Glu Phe Glu Asp Leu
                   245                 250                 255
   Ile Phe Leu Ala Arg Ser Ala Leu Ile Leu Arg Gly Ser Val Ala His
               260                 265                 270
   Lys Ser Cys Leu Pro Ala Cys Val Tyr Gly Pro Ala Val Ala Ser Gly
           275                 280                 285
   Tyr Asp Phe Glu Arg Glu Gly Tyr Ser Leu Val Gly Ile Asp Pro Phe
       290                 295                 300
   Arg Leu Leu Gln Asn Ser Gln Val Tyr Ser Leu Ile Arg Pro Asn Glu
   305                 310                 315                 320
   Asn Pro Ala His Lys Ser Gln Leu Val Trp Met Ala Cys His Ser Ala
                   325                 330                 335
   Ala Phe Glu Asp Leu Arg Val Ser Ser Phe Ile Arg Gly Thr Lys Val
               340                 345                 350
   Val Pro Arg Gly Lys Leu Ser Thr Arg Gly Val Gln Ile Ala Ser Asn
           355                 360                 365
   Glu Asn Met Glu Thr Met Glu Ser Ser Thr Leu Glu Leu Arg Ser Arg
       370                 375                 380
   Tyr Trp Ala Ile Arg Thr Arg Ser Gly Gly Asn Thr Asn Gln Gln Arg
   385                 390                 395                 400
   Ala Ser Ser Gly Gln Ile Ser Ile Gln Pro Thr Phe Ser Val Gln Arg
                   405                 410                 415
   Asn Leu Pro Phe Asp Arg Pro Thr Ile Met Ala Ala Phe Thr Gly Asn
               420                 425                 430
   Thr Glu Gly Arg Thr Ser Asp Met Arg Thr Glu Ile Ile Arg Leu Met
           435                 440                 445
   Glu Ser Ala Arg Pro Glu Asp Val Ser Phe Gln Gly Arg Gly Val Phe
       450                 455                 460
   Glu Leu Ser Asp Glu Lys Ala Ala Ser Pro Ile Val Pro Ser Phe Asp
   465                 470                 475                 480
   Met Ser Asn Glu Gly Ser Tyr Phe Phe Gly Asp Asn Ala Glu Glu Tyr
                   485                 490                 495
   Asp Asn
<210> 65
   <211> 30
   <212> DNA
   <213> Artificial Sequence
<220>
   <223> primer
<400> 65
   gactccatgg cgaccaaagg caccaaacga 30
<210> 66
   <211> 46
   <212> DNA
   <213> Artificial Sequence
<220>
   <223> primer
<400> 66
   gatcctcgag ttagtggtgg tggtggtggt gattgtcgta ctcctc 46

## Description

### FIELD OF THE INVENTION

The present invention relates to the field of vaccine formulations and adjuvants and, in particular, to influenza vaccines based on plant virus particles.

### BACKGROUND OF THE INVENTION

There are three genera of influenza virus, A, B and C with the last mentioned causing only mild respiratory illness, which can be identified by antigenic differences in their nucleocapsid and matrix proteins. In general, influenza A strains are capable of infecting a large number of vertebrates including humans, domestic and farm animals, marine mammals, and various birds. Influenza B and C, however, are largely restricted to humans, with influenza B infections also having been observed in pigs, and influenza C infections in seals. Influenza types A and B are the most prevalent types in humans and cause 36,000 deaths and 114,000 hospitalizations per year in the USA alone. Most modem influenza vaccines are targeted to type A or B viruses, and predominantly to type A.

The physical structure of all influenza A viruses is similar. The virus particles are enveloped and can be either spherical or filamentous in form. The influenza A virus is an RNA virus and its genome consists of eight single RNA strands: HA which encodes haemagglutinin, NA which encodes neuraminidase, NP which encodes nucleoprotein, M which encodes two matrix proteins (M1 and M2), NS which encodes two non-structural proteins (NS1 and NEP), PA which encodes an RNA polymerase, PB1 which encodes an RNA polymerase and the PB1-F2 protein, and PB2 which encodes another RNA polymerase. This segmented nature of the influenza virus genome allows entire genes to be exchanged between different strains of the virus, which leads to the emergence of new virulent strains and thus provides a challenge to prophylaxis and/or treatment. The multiplicity of influenza virus strains confers the need for annual vaccination according to the strains that are more prevalent at the time, which is determined each year by the World Health Organization.

Existing human vaccines contain three killed or attenuated virus strains - one A (H3N2) virus, one A (H1N1) virus, and one B virus. The hemagglutinin (HA) and the neuraminidase (NA) proteins, which are accessible large glycoproteins at the surface of the virus, are the major target of the immune response during infection which has in turn induced a drift and shift in these proteins (Fier et al., 2004, Virus Research 103:173-176). The selection pressure of the immune system on the surface glycoproteins favours the emergence of mutated viruses that propagate efficiently and cause new epidemics. The newly emerged strain is usually selected as a component of the next vaccine, but this may not come onto the market until 6-8 months later. During this time, the circulating virus has time to evolve which results in a partial efficiency of the vaccine. The reassortment of the viruses in pig and bird reservoirs complicates the cycle and can be the source of pandemics.

H9N2 influenza viruses, for example, have become established in terrestrial poultry in different Asian countries over the last 2 decades and have also become endemic in different types of terrestrial poultry in multiple countries on the Eurasian continent (Xu et al., 2007, J. Virol. 81:10389-10401). Recently, it was demonstrated that the long-term co-circulation of H5N1 and H9N2 viruses in different types of poultry has facilitated frequent reassortment events that are responsible for the great genetic diversity in H9N2 and H5N1 influenza viruses. This situation favours the emergence of influenza viruses with pandemic potential (Xu et al., 2007, *ibid*.). As such, vaccination of poultry may be effective in preventing the dissemination of the H9N2 strains and decrease the risks of emergence of a new virus that could also be virulent in humans.

A live attenuated nasal vaccine of Influenza (FluMist® from MedImmune Vaccines, Inc.) may provide a certain level of cross protection to other strains of influenza through induction of a cytotoxic T lymphocyte (CTL) response toward highly conserved protein found inside the virus particle (Kaiser, 2006, Science 312:380-383). Another approach to creating a vaccine that provides immune protection against influenza strains is to target several universal influenza antigens. The ectodomain of the ion channel protein M2e, a 25 amino acid long peptide, is one possible target (Neirynck et al., 1999, Nat Med 5:1157-1163). The M2e peptide has been conjugated to various carriers to increase its stability and immunogenicity, including keyhole limpet hemocyanin and *Neisseria meninigitidis* outer membrane protein, and was shown to provide partial protection to influenza infection (Fan et al., 2004, Vaccine 22:2993-3003). The protection capacity of the M2e peptide is believed to be only partial and, as such, it has been suggested that it could be included as an additional component in annual flu shots (Kaiser, 2006, Science 312:380-383). Chemical cross-linking of the M2e peptide to a hydrophobic protein for incorporation into a lipidic complex, however, has been employed and the resulting liposome based vaccine was successful in protecting mice against challenge with various influenza virus strains (Ernst et al., 2006, Vaccine 24:5152-5158).

Addition of commercial adjuvants, such as alum (De Filette et al., 2005, Virology 337:149-161), commercial cholera toxin ((De Filette et al., 2006, Vaccine 24:544-551), or monophosphoryl lipid A, reduced morbidity (loss of body weight, decrease in body temperature) and symptoms observed compared to M2 mediated protection without adjuvants (Neirynck et al., *ibid*.).

Another approach to a universal flu vaccine is to use conserved internal proteins such as the matrix protein (M1) or the nucleocapsid (NP) to elicit immunity based on CTL rather than neutralizing antibodies to HA and NA. CTLs will eliminate the infected cells by specific recognition of influenza peptides loaded on the MHC class I complex. The MHC class I complex, located at the surface of the infected cells, efficiently presents peptides derived from highly conserved proteins like M1 and NP. The injection of purified NP or M1 is unlikely to mount alone a protective CTL response, but rather the target proteins must be associated with an adjuvant or a delivery system that is aimed at developing a CTL response to a conserved epitope, such as adenoviral vectors (Bangari and Mittal, 2006, Curr Gene Ther 6:215-226; Ghosh et al., 2006, Appl Biochem Biotechnol 133:9-29) and DNA vaccines (Laddy and Weiner, 2006, Int Rev Immunol 25:99-123; Stan et al., 2006, Hematol Oncl Clin North Am 20:613-636). Adenoviral vectors, however, can be neutralized by antibodies that inhibit their entry to APC (Palker et al., 2004, Virus Res 105:183-194) and DNA vaccines are not immunogenic in large animals and require addition of an adjuvant, such as CpG, to increase their immunogenicity (Klinman D.M., (2006) Int Rev Immunol. 25; 135-154).

Among the numerous new approaches to vaccine development, virus-like-particles (VLPs) made of viral nucleocapsids have emerged as a promising strategy. To date, two VLP vaccines, hepatitis B virus (HBV) and Human Papilloma Virus (HPV), have been shown to function efficiently in humans (Fagan et al., 1987, J. Med. Virol., 21:49-56; Harper et al., 2004, Lancet, 364:1757-1765). VLPs made from the human papillomavirus (HPV) major capsid protein L1, for example, were shown to provide 100% protection in woman against development of cervical cancers (Ault, K.A., 2006, Obstet. Gynecol. Surv. 61:S26-S31; Harper et al., 2004, Lancet 364:1757-1765, see also International Patent Application PCT/US01/18701 (WO 02/04007)). Platforms such as bacteriophage Qβ (Maurer et al., 2005, Eur. J. Immunol. 35:2031-2040), hepatitis B virus VLPs made of the viral core protein (Mihailova et al., 2006, Vaccine 24:4369-4377; Pumpens et al., 2002, Intervirology 45:24-32), and parvovirus VLPs (Antonis et al., 2006, Vaccine 24:5481-5490; Ogasawara et al., 2006, In Vivo 20:319-324) have also shown capacity to carry epitopes and induce a strong antibody response.

The ability of porcine parvovirus (PPV) VLPs to act as an adjuvant when independently administered to mice with exogenous antigen has also been described (Biosgérault et al., 2005, J. Immunol. 174:3432-3439).

Influenza virus VLPs have also been described in the art (see International Patent Application No. PCT/US2004/022001 (WO 2005/020889); and U.S. Patent Application Nos. 2005/0186621 and 2005/0009008). Chemical cross-linking of M2e peptide to Hepatitis B Virus (HBV) (Jegerlehner et al., 2002, Vaccine 20:3104-3112) or to Human Papillomavirus (HPV) (Ionescu et al., J. Pharm. Sci. 95:70-79) and an in-frame fusion of M2e peptide with HBV coat protein (Neirynck et al., 1999, Nat Med 5:1157-1163; Jegerlehner et al., 2004, Vaccine 22:3104-3112; and U.S. Patent Application No. 2003/0202982) were all successful at protecting mice against challenge with influenza virus. A chimeric VLP made from the yeast retrotransposon Ty p1 protein fused to an influenza virus haemagglutinin or nucleoprotein epitope has also been described (U.S. Patent No. 6,060,064).

The use of VLPs from plant viruses as epitope presentation systems has been described. Plant viruses are comprised mainly of proteins that are highly immunogenic, and possess a complex, repetitive and crystalline organisation. In addition, they are phylogenetically distant from the animal immune system, which makes them good candidates for the development of vaccines. For example, cowpea mosaic virus (CPMV), tobacco mosaic virus (TMV), and alfalfa mosaic virus (AIMV) have been modified for the presentation of epitopes of interest (Canizares, M. C. et al., 2005, Immunol. Cell. Biol. 83:263-270; Brennan et al., 2001, Molec. Biol. 17:15-26). International Patent Application PCT/GB97/01065 (WO 97/39134) describes chimaeric virus-like particles that comprise a coat protein and a non-viral protein, which can be used, for example, for presentation of peptide epitopes. International Patent Application PCT/US01/07355 (WO 01/66778) describes a plant virus coat protein, and specifically a tobamovirus coat protein, fused via a linker at the N-terminus to a polypeptide of interest, which may include an epitope of a pathogenic micororganism. International Patent Application PCT/US01/20272 (WO 02/00169) describes vaccines comprising either potato virus Y coat protein or a truncated bean yellow mosaic virus coat protein fused to a foreign peptide, and specifically a Newcastle Disease Virus or human immunodeficiency virus (HIV) epitope. Also, U.S. Patent No. 6,042,832 describes methods of administering fusions of polypeptides, such as pathogen epitopes, with alfalfa mosaic virus or ilarvirus capsid proteins to an animal in order to raise an immune response.

VLPs derived from the coat protein of papaya mosaic virus (PapMV) and their use as immunopotentiators has been described (International Patent Application No. PCT/CA03/00985 (WO 2004/004761)). Expression of the PapMV coat protein in *E. coli* leads to the self-assembly of VLPs composed of several hundred CP subunits organised in a repetitive and crystalline manner (Tremblay et al., 2006, FEBS J 273:14). Studies of the expression and purification of PapMV CP deletion constructs further indicate that self-assembly (or multimerization) of the CP subunits is important for function (Lecours et al., 2006, Protein Expression and Purification, 47:273-280*).*

VLPs derived from Potato Virus X (PVX) carrying an antigenic determinant from HIV (gp41 or gp120), HCV (NS3, EI or E2), EBV (EBNA-3A, 3B or 3C) or the influenza virus (matrix protein or haemagglutinin) have been described (European Patent Application No. 1 167 530). The ability of the PVX VLP carrying an HIV epitope to induce antibody production in mice via humoral and cell-mediated pathways. Additional adjuvants were used in conjunction with the PVX VLP to potentiate this effect.

International Patent Application Publication No. WO2005/055957 describes a chimeric, C-terminal truncated hepatitis B virus nucleocapsid (HBc) protein that contains an immunogen for inducing antibodies to the influenza M2 protein. De Fillette et al. (2006, Vaccine, 24:6597-6601) describe genetic fusion of the M2e peptide and hepatitis B virus core (HBc) coding sequences, which provide highly immunogenic virus-like particles.

Hepatitis B core protein or parvovirus VLPs have been reported to induce a CTL response even when they do not carry genetic information (RuedI et al., 2002, Eur. J. Immunol. 32; 818-825; Martinez et al., 2003, Virology, 305; 428-435) and can not actively replicate in the cells where they are invaginated. The cross-presentation of such VLPs carrying an epitope from lymophocytic choriomeningitis virus (LCMV) or chicken egg albumin by dendritic cells *in vivo* has also been described (RuedI et al., 2002, *ibid;* Moron, et al., 2003, J Immunol. 171:2242-2250). The ability of a hepatitis B core protein VLP carrying an epitope from LCMV to prime a CTL response has also been described, however, this VLP was unable to induce the CTL response when administered alone and failed to mediate effective protection from viral challenge. An effective CTL response was induced only when the VLP was used in conjunction with anti-CD40 antibodies or CpG oligonucleotides (Storni, et al., 2002, J. Immunol. 168:2880-2886). An earlier report indicated that porcine parvovirus-like particles (PPMV) carrying a peptide from LCMV were able to protect mice against a lethal LCMV challenge (Sedlik, et al., 2000, J. Virol. 74:5769-5775).

Papaya mosaic virus VLPs fused to affinity peptides have been proposed as an alternative to monoclonal antibodies in the detection of fungal diseases (Morin et al., 2007, J. Biotechnology, 128: 423.434 [epub ahead of print October 26, 2006]). VLPs were developed that were capable of binding *Plasmodiophora brassicae* spores with high avidity and binding of one construct to the spores was demonstrated to be at a level comparable to that of polyclonal antibodies.

This background information is provided for the purpose of making known information believed by the applicant to be of possible relevance to the present invention. No admission is necessarily intended, nor should be construed, that any of the preceding information constitutes prior art against the present invention.

### SUMMARY OF THE INVENTION

An object of the present invention is to provide papaya mosaic virus-based vaccines for influenza. In accordance with one aspect of the invention, there is provided an antigen-presenting system comprising one or more influenza virus antigens in combination with a papaya mosaic virus (PapMV) or a VLP comprising PapMV coat protein, wherein said one or more influenza virus antigens are not conjugated to said PapMV or VLP.

In accordance with another aspect, there is provided an influenza vaccine composition comprising one or more antigen-presenting systems of the invention.

In another aspect the invention provides a composition comprising papaya mosaic virus (PapMV) or a VLP comprising PapMV coat protein for use to improve the efficacy of a commercially available influenza vaccine in inducing an immune response in an animal against an influenza virus, wherein said composition and said influenza vaccine are administered concomitantly to said animal.

In another aspect the invention provides a composition comprising:
one or more influenza virus antigens in combination with a papaya mosaic virus (PapMV) or a VLP comprising PapMV coat protein, and
an adjuvant,
wherein said one or more influenza virus antigens are peptide antigens that are conjugated to the coat protein of said PapMV or VLP, and
wherein said adjuvant comprises additional PapMV or PapMV VLPs.

In accordance with another aspect of the invention, there is provided a polypeptide comprising a papaya mosaic virus coat protein fused to one or more influenza virus peptide antigens for use in the preparation of the composition wherein said polypeptide is capable of self-assembly to form a VLP.

In accordance with another aspect, there is provided a polynucleotide encoding a polypeptide of the invention.

In accordance with another aspect, there is provided a use of an antigen-presenting system of the invention for the manufacture of a medicament for inducing an immune response against an influenza virus in an animal.

### BRIEF DESCRIPTION OF THE DRAWINGS

These and other features of the invention will become more apparent in the following detailed description in which reference is made to the appended drawings.

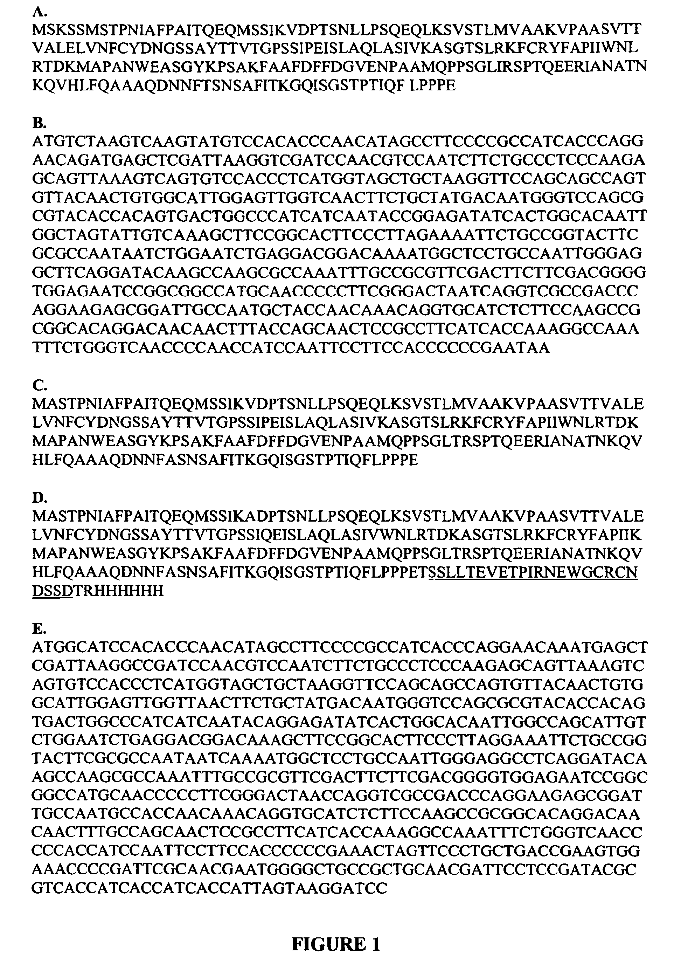

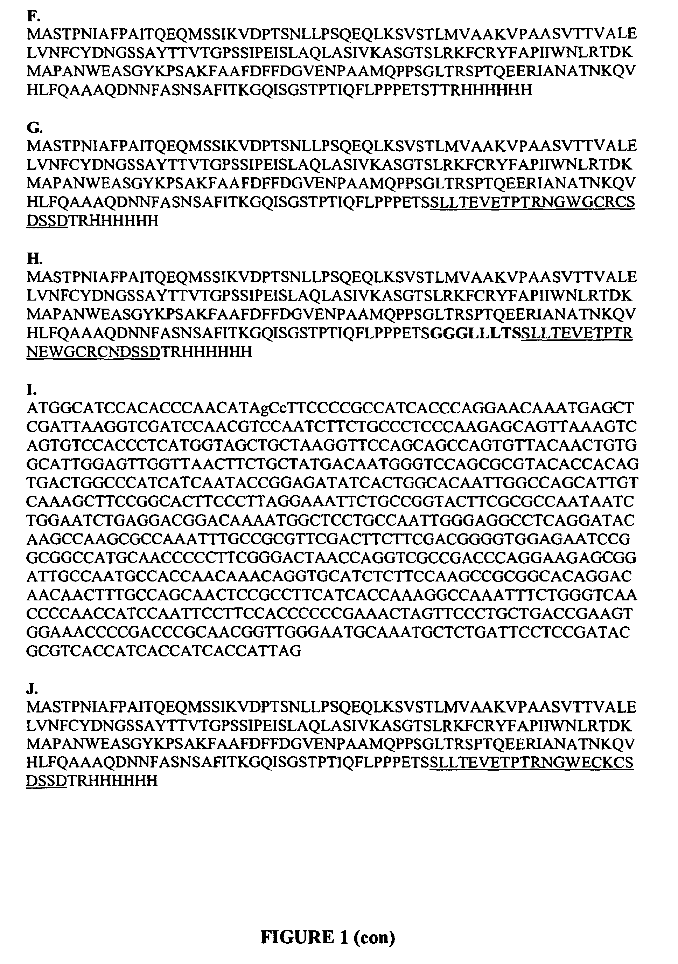

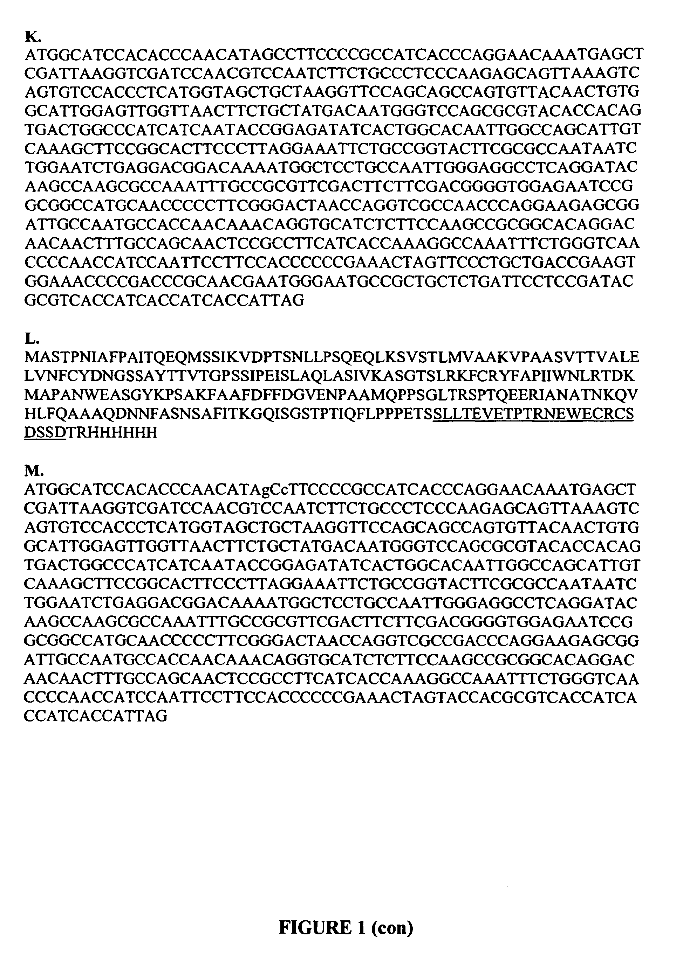

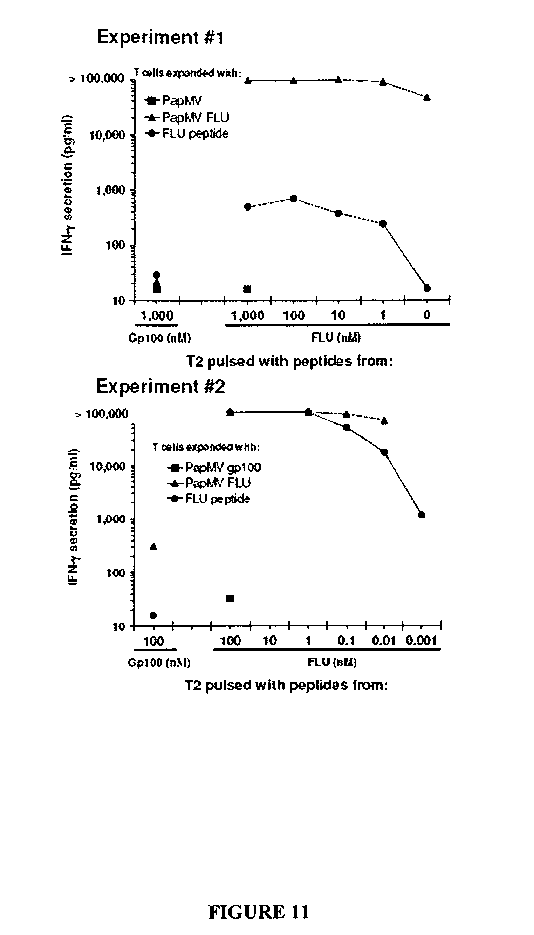

**Figure 1** presents (A) the amino acid sequence for the papaya mosaic virus (PapMV) coat protein (GenBank Accession No. NP_044334.1; SEQ ID NO:1), (B) the nucleotide sequence encoding the PapMV coat protein (GenBank Accession No. NC_001748 (nucleotides 5889-6536); SEQ ID NO:2), (C) the amino acid sequence of the mutant PapMV coat protein CPΔN5 (SEQ ID NO:3), (D) the amino acid sequence of a mutant PapMV coat protein CPΔN5-SM fused at its C-terminus to the M2e peptide from influenza virus strain H1N1 (A/New Caledonia/20/99) (underlined) and containing a 6xHis tag (SEQ ID NO:4), (E) the nucleotide sequence encoding the protein shown in (D) (SEQ ID NO:5), (F) the amino acid sequence of the mutant PapMV coat protein CPΔN5 containing the restriction sites SpeI-MluI at the C-terminus (CPΔN5-SM) (SEQ ID NO:46), (G) the amino acid sequence of the PapMV coat protein CPΔN5-SM fused at its C-terminus to the M2e peptide from influenza virus strain H9N2 (underlined) and containing a 6xHis tag (SEQ ID NO:47), (H) the amino acid sequence of the PapMV coat protein CPΔN5-SM fused at its C-terminus to the M2e peptide from influenza virus strain H9N2 (underlined) with a glycine spacer (in bold) inserted between the coat protein sequence and the M2e peptide sequence and also containing a 6xHis tag (SEQ ID NO:48), (I) the nucleotide sequence encoding a PapMV coat protein fused at its C-terminus to the M2e peptide from influenza virus strain H3N8 and containing a 6xHis tag (SEQ ID NO:56), (J) the amino acid sequence encoded by the sequence shown in Fig. 11 (SEQ ID NO:57), the H3N8 M2e peptide sequence is underlined, (K) the nucleotide sequence encoding the PapMV coat protein CPΔN5-SM fused at its C-terminus to the M2e peptide from influenza virus strain H5N1 and containing a 6xHis tag (SEQ ID NO:58), (L) the amino acid sequence encoded by the sequence shown in Fig. 1K (SEQ ID NO:59), the H5N1 M2e peptide sequence is underlined, and (M) the nucleotide sequence encoding the amino acid sequence of CPΔN5-SM shown in (F) (SEQ ID NO:49).

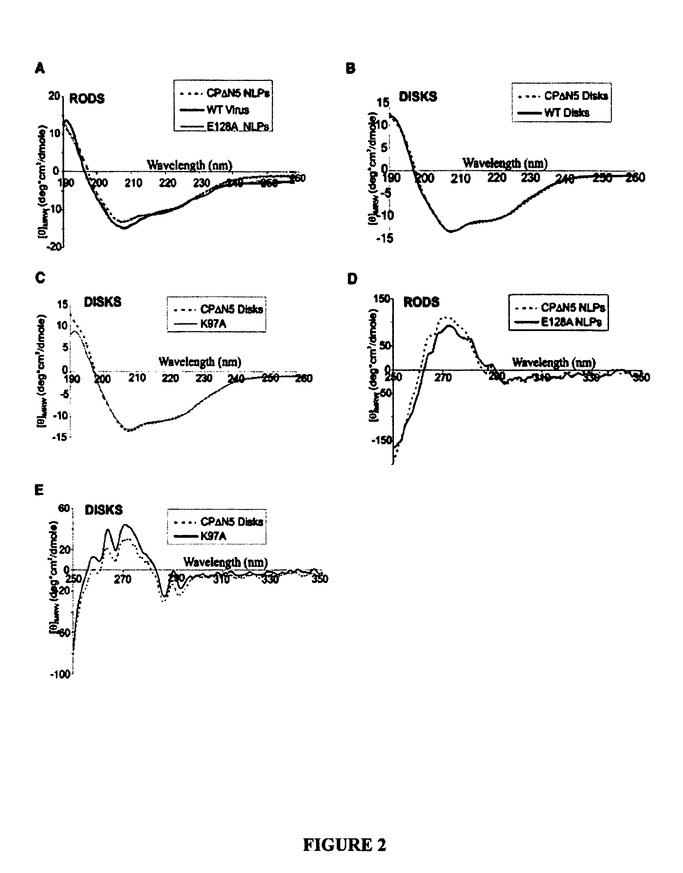

**Figure 2** presents circular dichroism (CD) spectra of wild-type (WT) PapMV and PapMV virus-like particles (VLPs) comprising the recombinant mutant PapMV coat proteins CPΔN5, E128A or K97A; (A) shows far-UV spectra of WT virus (black line), CPΔN5 (dotted line) and E128A VLPs (grey line), (B) shows far-UV spectra of isolated discs from the WT virus by the acetic acid method (black line) and high speed supernatant of the CPΔN5 protein (discs) (dotted line), (C) shows far-UV spectra of the CPΔN5 protein (discs) (dotted line) and the K97A protein (grey line), (D) shows far-UV spectra of the CPΔN5 and E128A VLPs between 250 and 350 nm, and (E) shows far-UV spectra of the CPΔN5 and K97A discs between 250 and 350 nm.

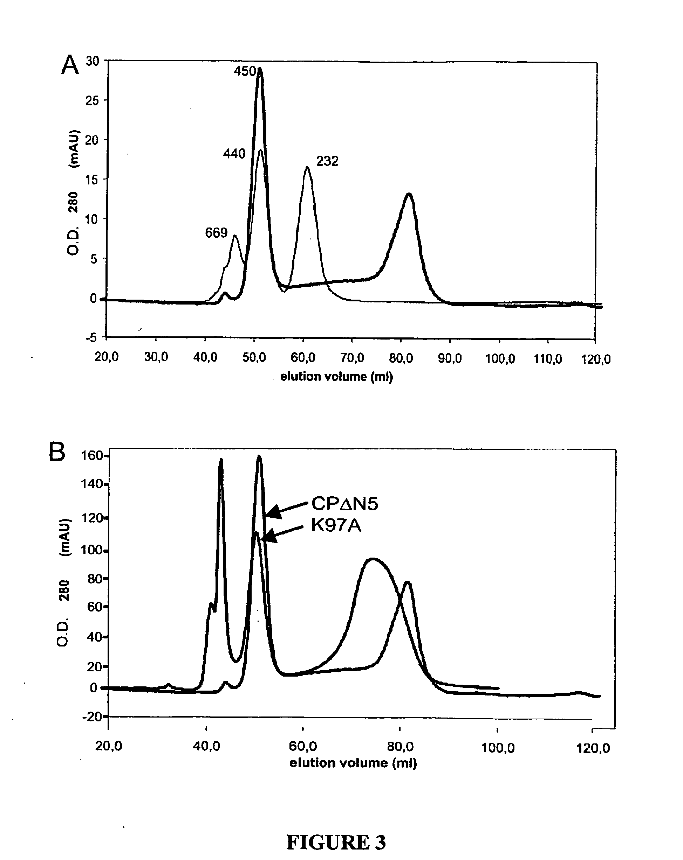

**Figure 3** presents the results of a gel filtration analysis of the recombinant mutant PapMV coat proteins, CPΔN5 and K97A; (A) depicts the protein elution profile for the CPΔN5 purified protein (black line; grey line: molecular weight markers), and (B) depicts the protein elution profile for the CPΔN5 purified protein (black line) and the K97A purified protein (grey line).

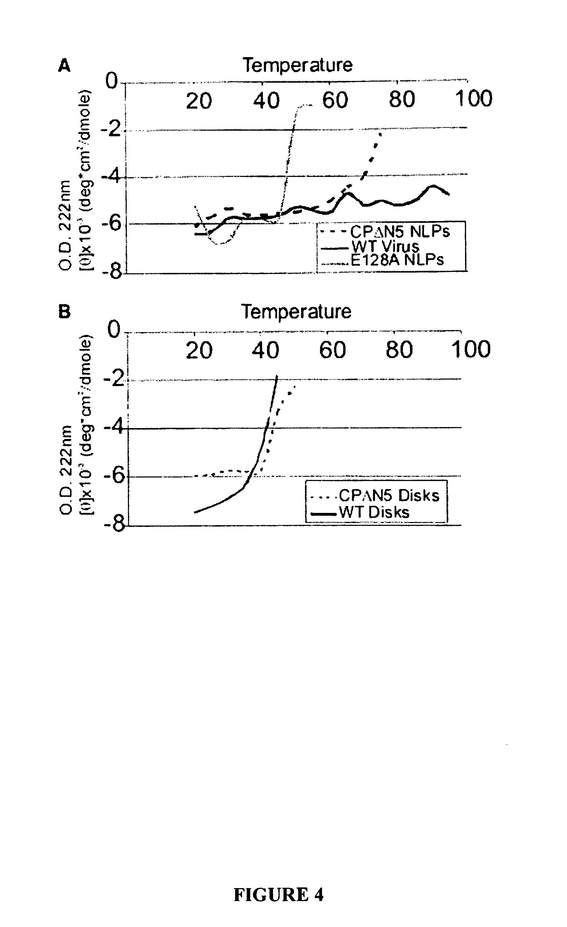

**Figure 4** presents circular dichroism (CD) spectra temperature-induced denaturation curves for the recombinant mutant and wild-type (WT) PapMV coat proteins; (A) shows the profiles for CPΔN5 and E128A mutant VLPs and WT virus, and (B) shows the profiles for CPΔN5 and WT discs. Spectra are presented in units of mean residue ellipticity. Unfolding was monitored by recording [Q] at 222 nm as a function of temperature. All CD spectra shown were generated with proteins at a concentration of 1 mg/ml in 10mM NaP buffer pH 7.2.

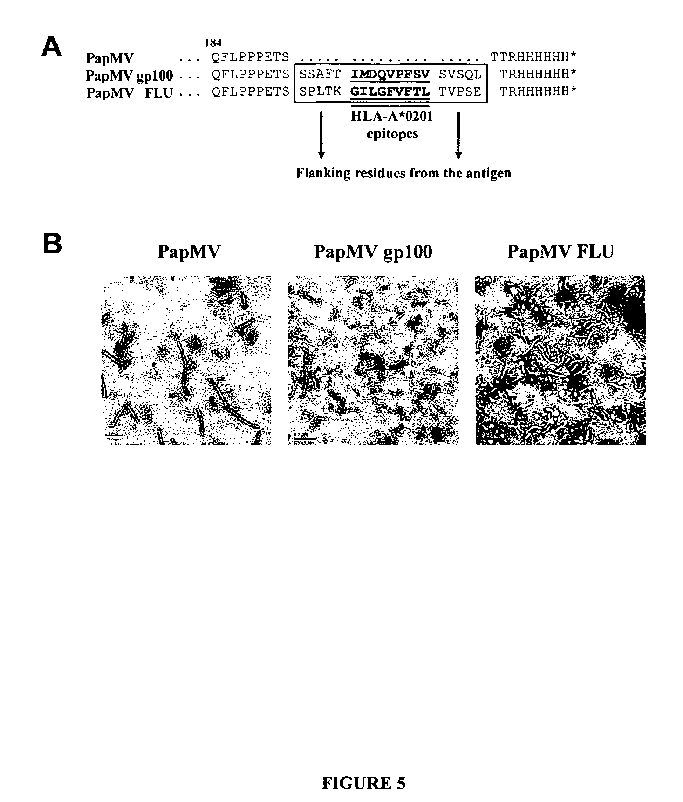

**Figure 5** presents (A) an alignment of the C-terminal sequences of PapMV coat protein (PapMV), and modified PapMV constructs containing HLA-A*0201 restricted epitopes from Influenza M1 protein (position 57 to 65; designated PapMV Flu) or from gp100 (position 209-217 with an M in position 210; designated PapMV gp100), and (B) electron microscope images of each of the recombinant self-assembled PapMV preparations.

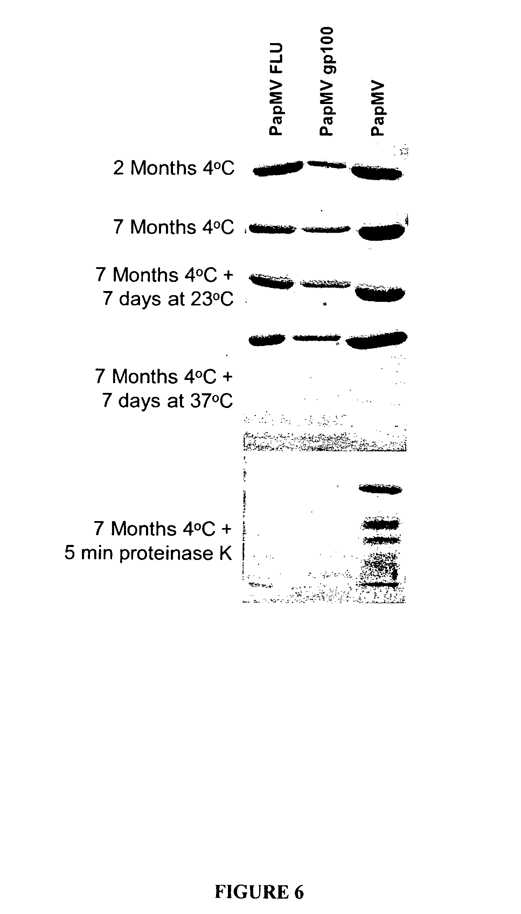

**Figure 6** presents the results of SDS-PAGE analysis of the stability of recombinant PapMV coat protein (PapMV) and coat protein fusions (PapMV-gp100 and PapMV-Flu) 2 and 7 months after their synthesis. Also illustrated are the results of incubating 7 month old protein preparations for 7 additional days at 23 or 37°C, and treatment with proteinase K for 5 minutes prior to addition of loading buffer and incubation in boiling water.

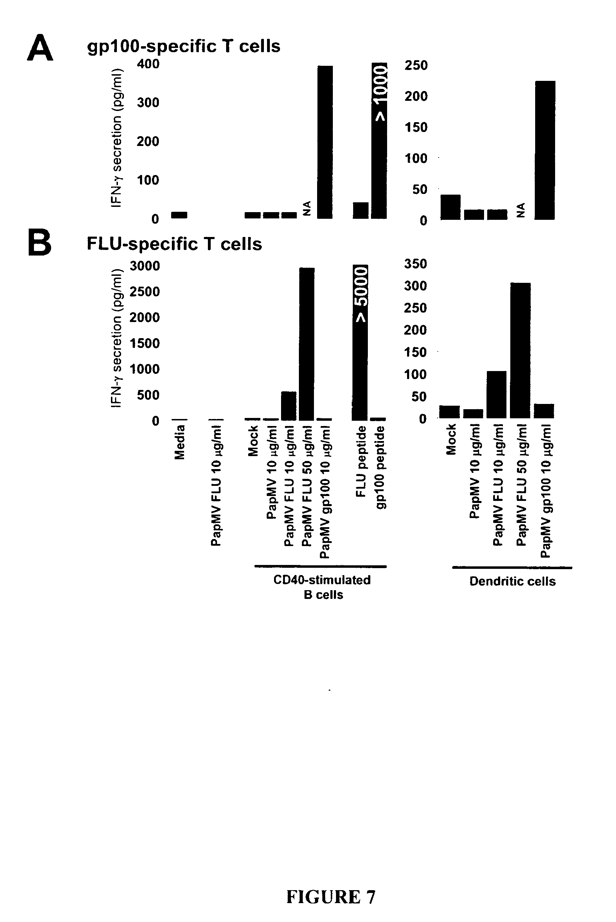

**Figure 7** illustrates that MHC class I epitopes from both antigens can be cross-presented when pulsed on 2 different sources of APC. CD40-activated B lymphocytes and DC were prepared from an HLA-A*0201 donor and pulsed for 18 h with indicated preparations at various concentrations. Pulsed cells were washed and gp100- (A) or FLU- (B) specific T cells were added for additional 18 h. Evidence of T cells activation was revealed by IFN-γ secretion determined by ELISA assay.

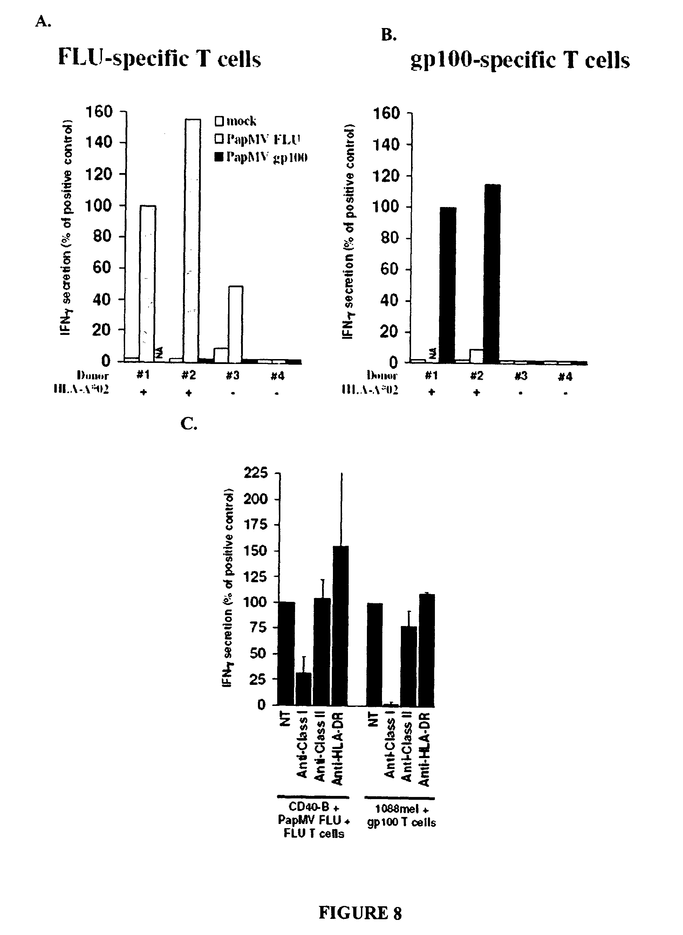

**Figure 8** illustrates the results from (A) co-culturing PapMV Flu or PapMV gp100 pulsed CD40-activated B lymphocytes prepared from HLA-A*02 positive or negative donors with FLU- (left panel) or gp100 (right panel)-specific T cells, and (B) incubating an HLA-A*0201⁺ and gp100⁺ melanoma line, or PapMV Flu pulsed HLA-A*02⁺ CD40-activated B cells with antibodies blocking MHC class I, class II or HLA-DR presentation. FLU- or gp100-specific T cells were then added. Results are represented as percentage of recognition based on IFN-γ secretion assay, 100% corresponding to the amount secreted by positive controls.

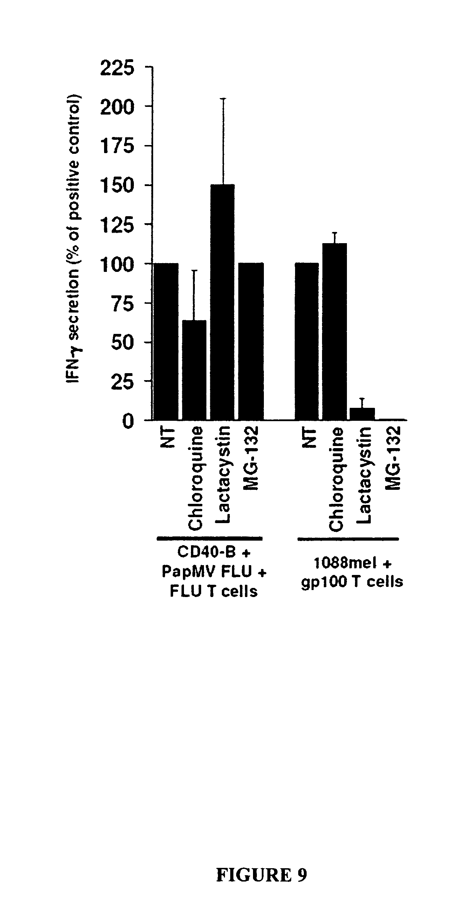

**Figure 9** il lustrates that MHC class I cross-presentation mediated by PapMV is proteasome independent. An HLA-A*0201⁺ and gp100⁺ melanoma line, or PapMV Flu-pulsed HLA-A*02⁺ CD40-activated B cells were incubated with antibodies blocking MHC class I, class II or HLA-DR presentation. FLU- or gp100-specific T cells were then added. Results are represented as percentage of recognition based on IFN-γ secretion assay, 100% corresponds to the amount secreted by positive controls.

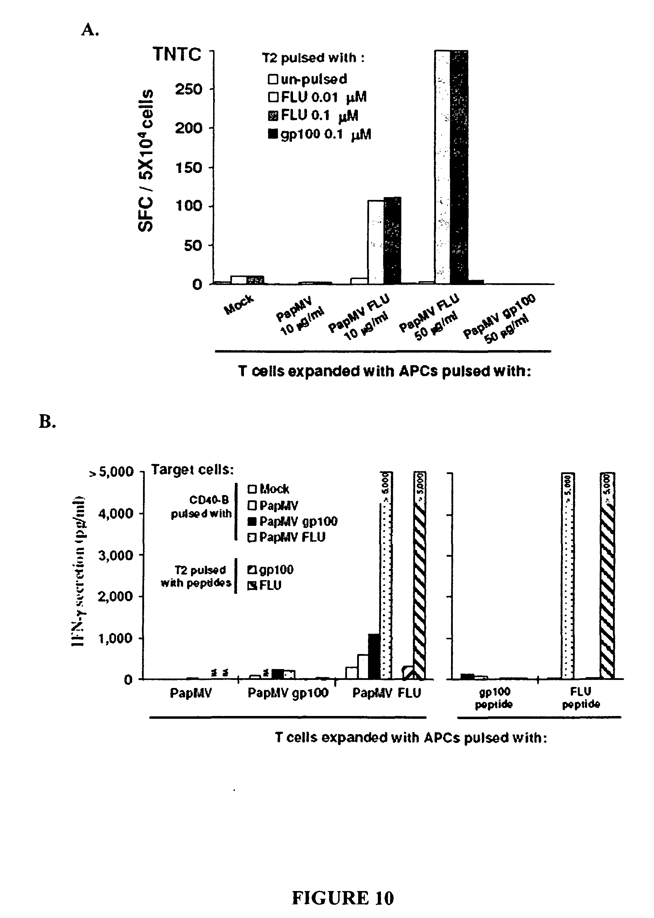

**Figure 10** presents an analysis of expansion of T lymphocytes specific to the HLA-A*0201 epitope from Influenza M1 protein with PapMV Flu-pulsed APC. (A) PBMC from an HLA-A2⁺ normal donor were co-cultured with autologous CD40-activated B cells (mock) or CD40-activated B cells pulsed with either PapMV, PapMV Flu or PapMV gp100. Cultured cells were restimulated on day 7 and IL-2 was added at different times. Specificity of expanded cells was assessed on day 15 by co-culture with T2 cells pulsed with HLA-A*0201 peptides from Flu or gp100. The frequency of IFN-γ secreting cells was determined by ELISPOT. (B) Expanded cultured T cells were co-cultured with pulsed CD40-activated B lymphocytes.
**Figure 11** presents the results of an analysis of IFN-γ secretion of expanded T cells treated as for Figure 10.

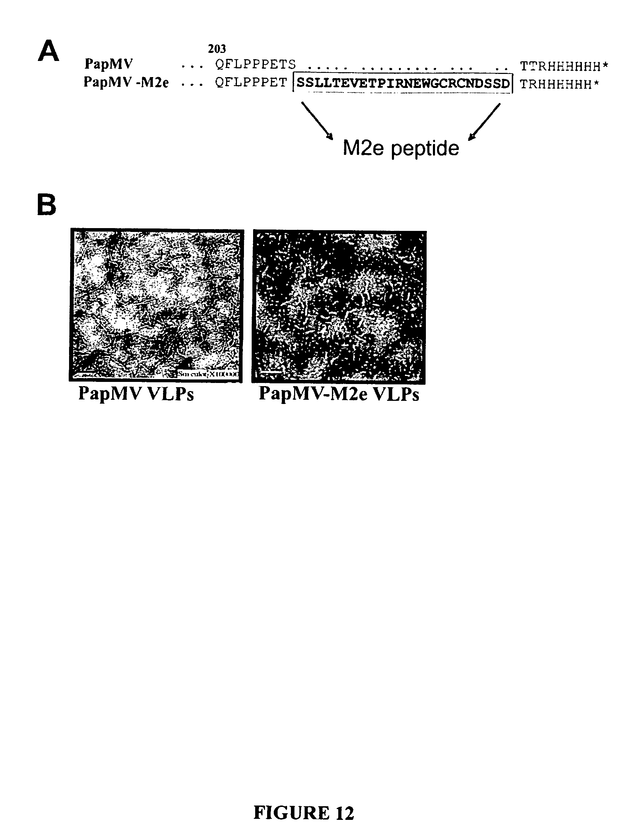

**Figure 12** presents (A) C-terminal sequences of PapMV coat protein (PapMV), and modified PapMV coat protein fused with the M2e epitope (position 2 to 24 of the M2e peptide from influenza virus strain H1N1 A/New Caledonia/20/99; designated PapMV-M2e), and (B) electron microscopy images of PapMV and PapMV-M2e VLPs.

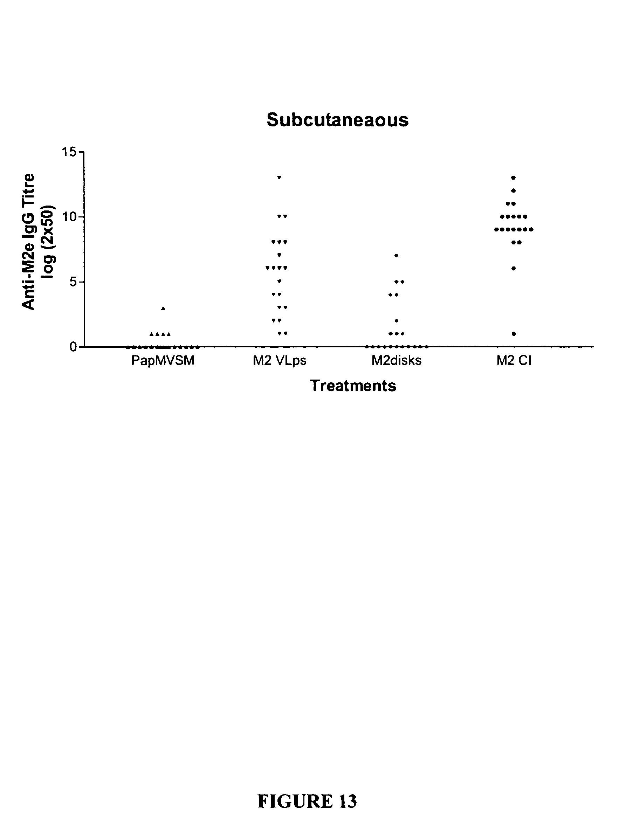

**Figure 13** illustrates the antibody response in Balb/C mice injected subcutaneously twice (day 0 and day 15) with 100µg of PapMV VLPs; PapMV-M2e VLPs, PapMV-M2e discs or PapMV-M2e VLPs + IgG (0.5µl of serum isolated from a pool of mice that were immunised twice with the PapMV-M2e VLPs was added to the VLPs to opsonise the protein with antibodies). The titer in IgG is shown for each mouse. 20 mice were immunised per treatment.

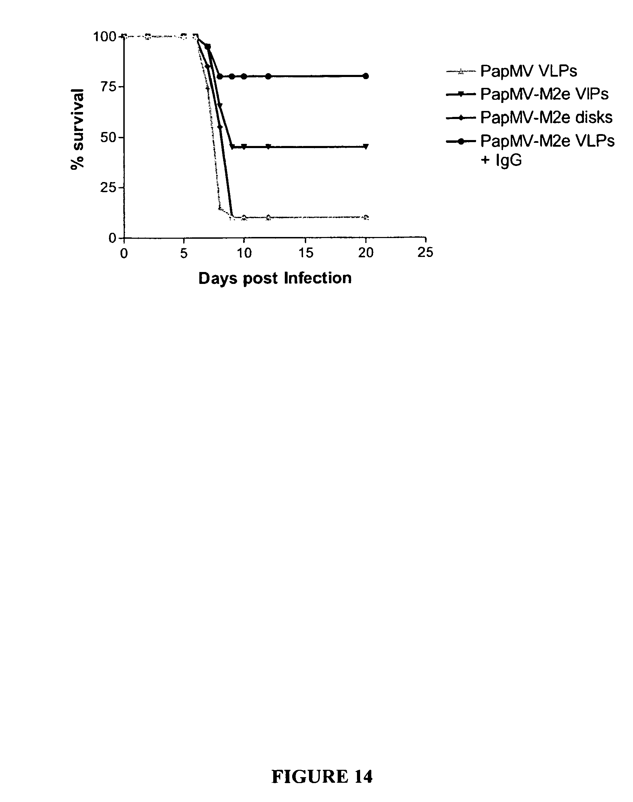

**Figure 14** presents the results of a protection assay against influenza virus strain H1N1 A/WSN/33 (3 x LD₅₀). Mice were vaccinated twice by subcutaneous injections, at two-week intervals (day -30 and -15) with 100µg of PapMV VLPs, PapMV-M2e VLPs, PapMV-M2e discs or PapMV-M2e VLPs + IgG (0.5µl of serum isolated from a pool of mice that were immunised twice with the PapMV-M2e VLPs was added to the VLPs to opsonise the protein with antibodies).

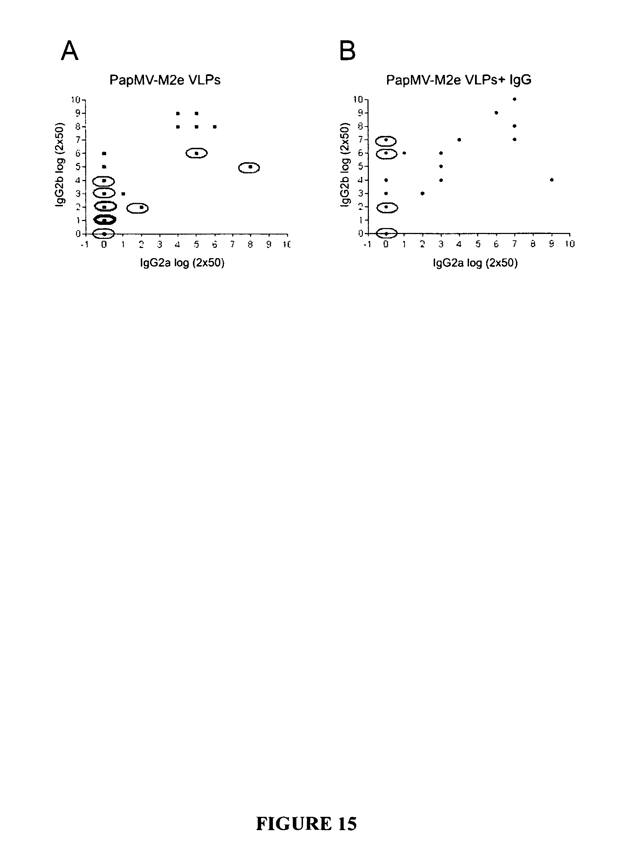

**Figure 15** illustrates the correlation between the levels of IgG2a and IgG2b measured in mice vaccinated with PapMV-M2e VLPs (A) or PapMV-M2e VLPs + IgG (B) and their capacity to survive to a challenge of 3 x the LD50 of the influenza virus strain H1N1 (A/WSN/33). The animals that survive the infection are shown as free dots.

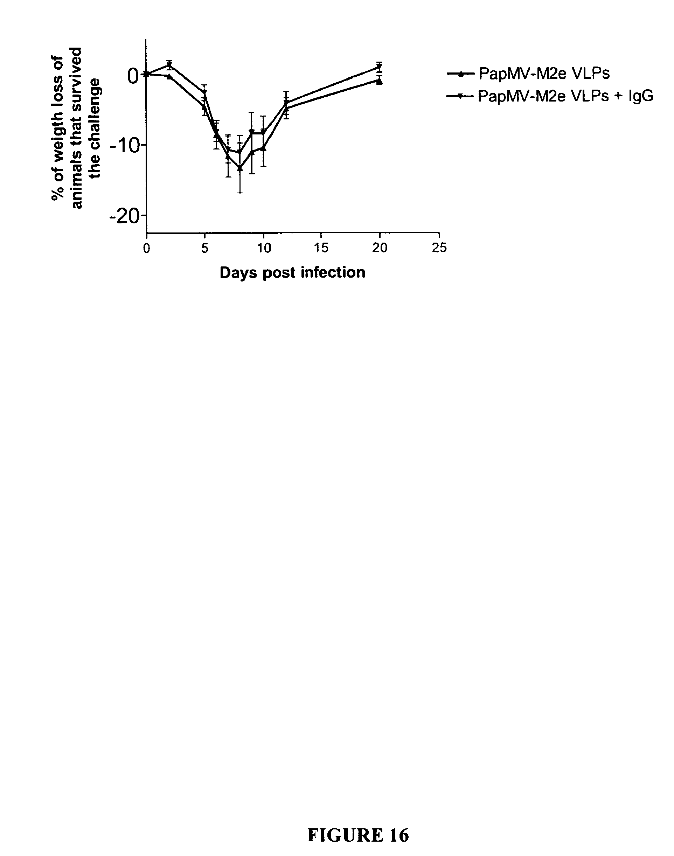

**Figure 16** presents weight loss data for mice vaccinated that survive the challenge with 3 x the LD₅₀ of the influenza virus strain H1N1 (A/WSN/33). The weight of 9 animals vaccinated with PapMV-M2e VLP and 16 animals vaccinated with PapMV-M2e VLPs + IgG that survived the challenge were pooled at days 2, 5, 6, 7, 8, 9, 10, 12 and 20.

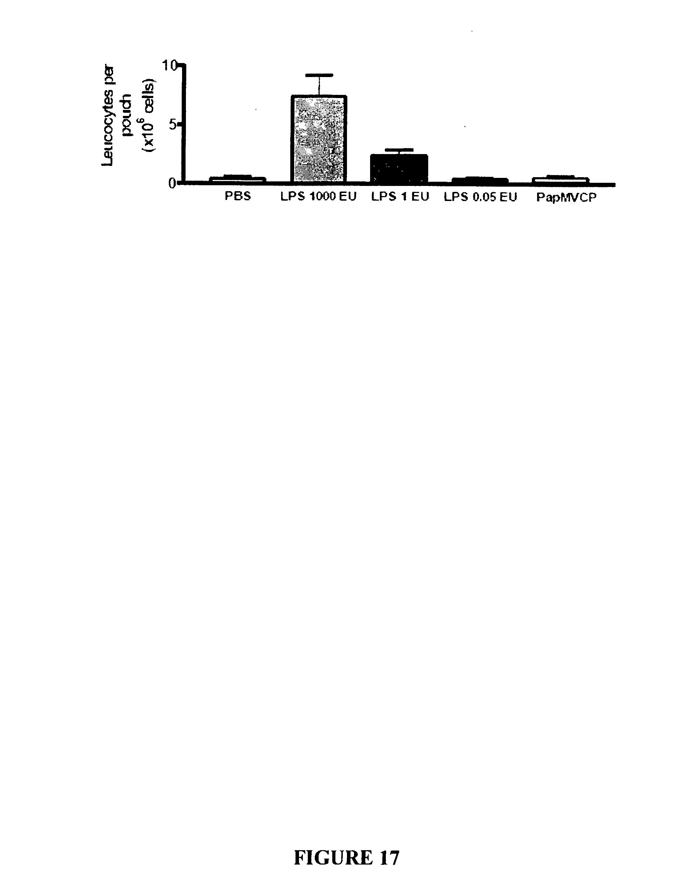

**Figure 17** presents a comparison of the pro-inflammatory response of PapMVCP and LPS in dorsal air pouches raised in CD-1 mice. Data represent the mean ± SEM of 5 mice. The results are representative of two identical and independent experiments.

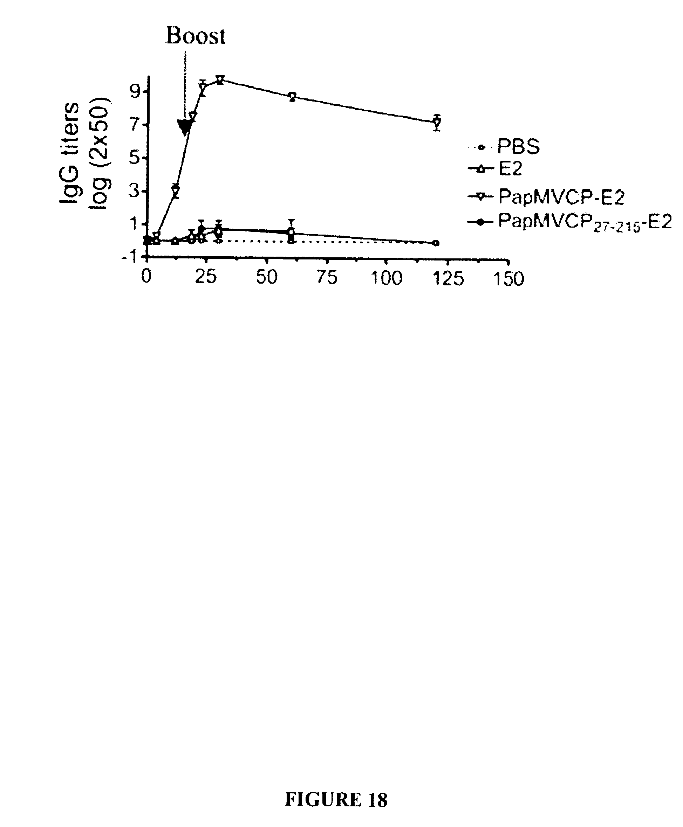

**Figure 18** illustrates the IgG antibody response specific for the PapMV capsid protein in C3H/HeJ mice injected subcutaneously with the multimeric PapMVCP-E2 (VLPs), the monomeric counterpart (PapMVCP27-215-E2), HCV E2 peptide alone, or PBS (ELISA plates were coated with PapMV-CP or PapMV27-215). Data represent the average of antibody titers from 4 mice. These results are representative of two identical and independent experiments. Black arrows on the graphs indicate the booster injections on day 15.

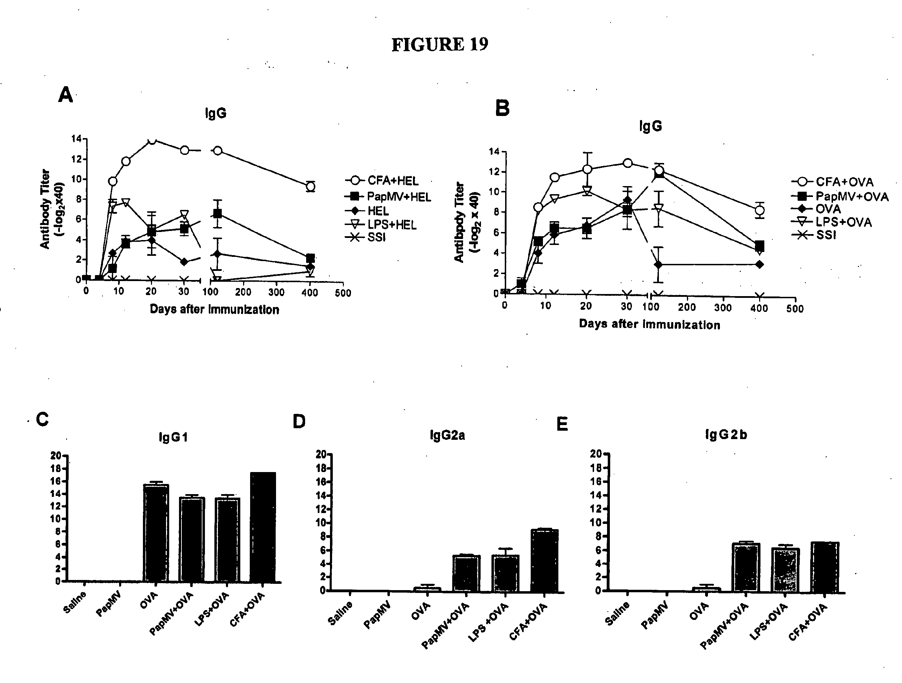

**Figure 19** illustrates the ability of purified PapMV isolated from plants to strengthen antibody responses to the model antigens (A) hen egg lysozyme (HEL) and (B) ovalbumin (OVA) in BALB/c mice (three per group) immunized on day 0 with antigen alone, antigen plus PapMV, Freund's complete adjuvant (FCA) or LPS from *E. coli* O111:B4. A representative result from 2 experiments is shown. The antibodies of the serum collected from the immunized animals were isotyped by ELISA on the model antigens (HEL or OVA) for (C) IgG1, (D) IgG 2a and (E) IgG2b.

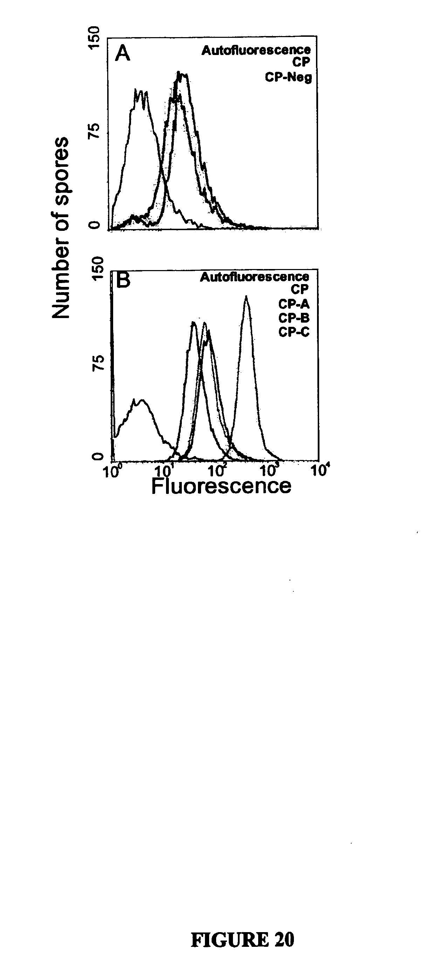

**Figure 20** presents fluorescence-assisted cell sorting (FACS) analysis of the binding of labelled VLPs comprising PapMV coat protein (CP)-affinity peptides fusions to *P. brassicae* spores (PapMV VLPs without any fusion (CP); PapMV VLPs fused to negative control peptide (CP-Neg); PapMV VLPs fused to peptide A (CP-A); PapMV VLPs fused to peptide B (CP-B); PapMV VLPs fused to peptide C (CP-C)).

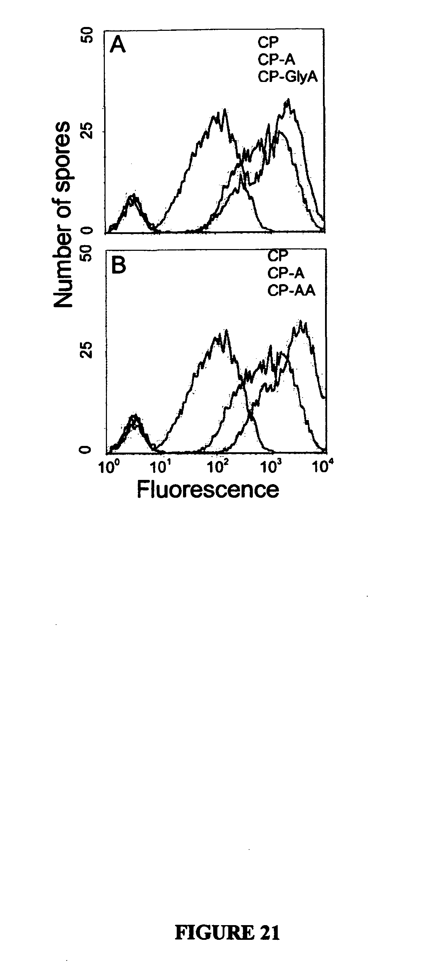

**Figure 21** demonstrates the improvement of avidity of binding of labelled VLPs comprising PapMV coat protein (CP)-affinity peptides fusions to *P. brassicae* spores using FACS analysis (A) Adding a stretch of 3 glycines between the C-terminus of CP and the peptide A (CP-GlyA) resulted in improved avidity for resting spores of *P. brassicae* as compared to CP-A and (B) duplication of peptide A to generate CP-AA improved the avidity as compared to CP-A.

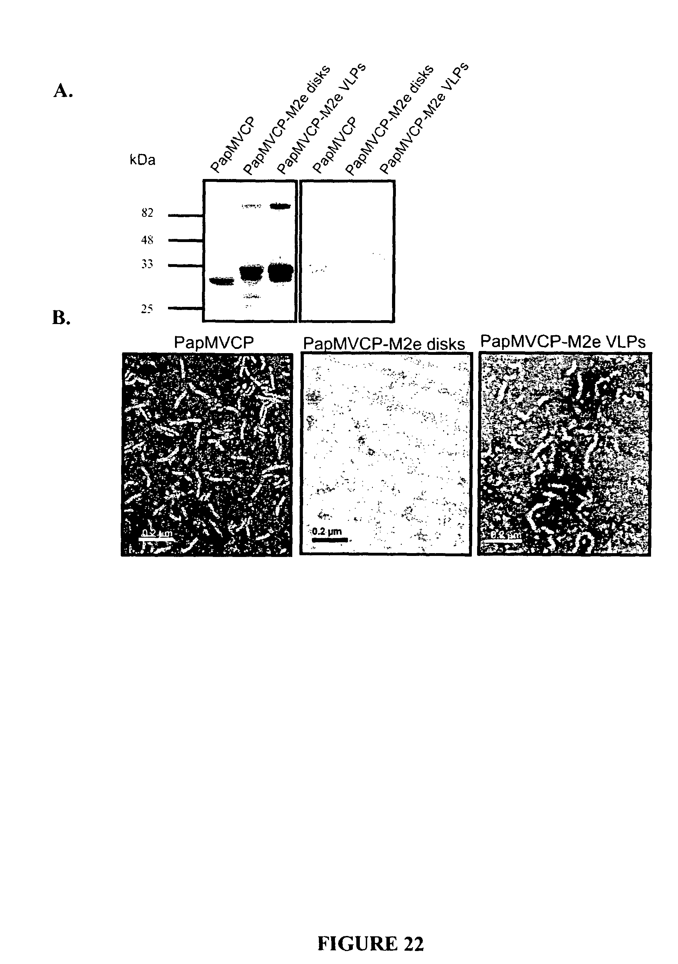

**Figure 22** presents (A) SDS-PAGE (left hand panel) and Western blot using an anti-PapMVCP antibody (right hand panel) of recombinant PapMV coat protein (PapMVCP) and recombinant PapMV coat protein fused to the M2e peptide (PapMVCP-M2e), and (B) electron micrographs of PapMVCP virus-like particles (VLPs) (left hand panel), PapMVCP-M2e discs (centre panel) and PapMVCP-M2e VLPs (right hand panel). Bars are 200 nm.

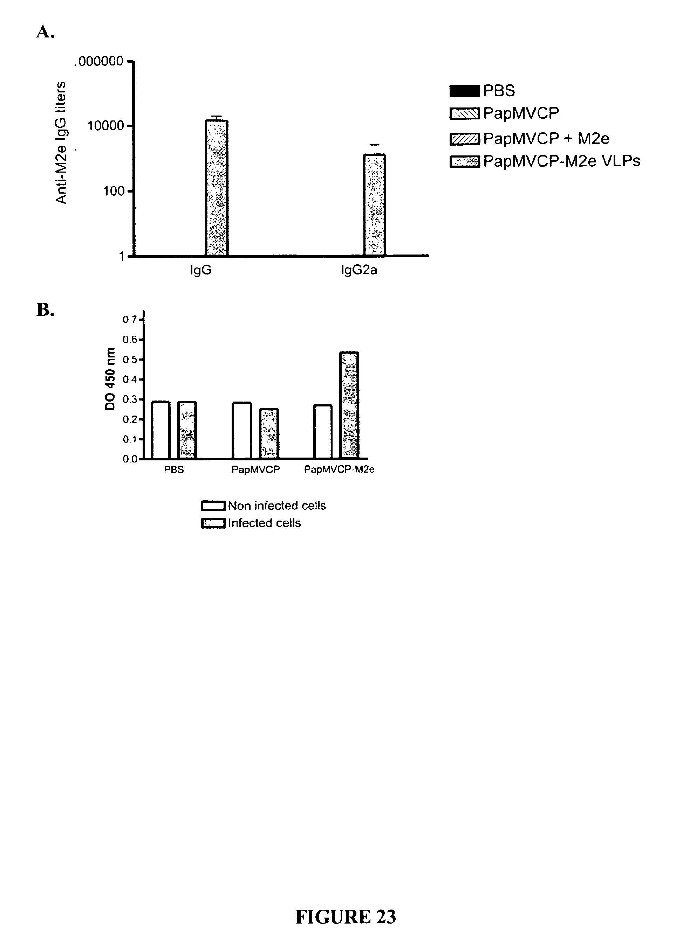

**Figure 23** presents (A) IgG and IgG2a antibody titers against influenza M2 epitope in BALB/c mice that were immunized twice with: PBS, 100µg of PapMVCP VLPs, PapMVCP VLPs with 10µg of the M2 peptide, or PapMVCP-M2e VLPs alone. Results are expressed as an antibody endpoint titer, where the O.D. value is 3-fold higher than the background value obtained with a 1:50 dilution of pre-immune serum from BALB/c mice. Data represent the mean ± SEM of 5 mice. (B) Binding of serum from the vaccinated mice by cells infected with the A/WSN/33 influenza strain (H1N1) measured by ELISA. Non-infected cells are used as a negative control. The results are representative of two identical and independent experiments.

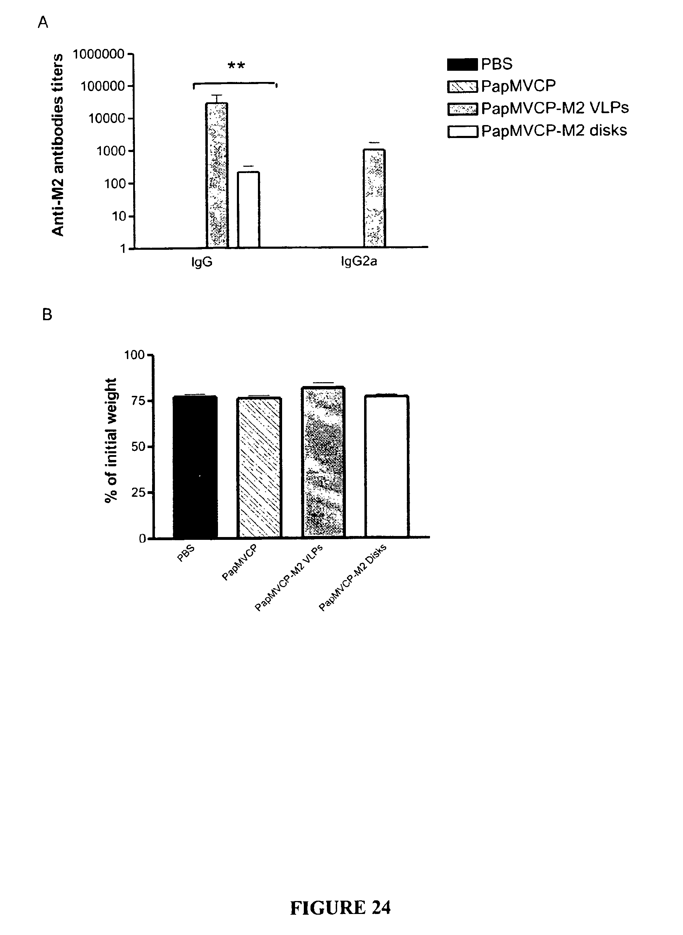

**Figure 24** presents (A) IgG and IgG2a antibody titers against influenza M2 epitope and (B) body weight in mice immunized twice (at days 0 and 14) with: PBS, PapMVCP VLPs, PapMVCP-M2e VLPs or PapMVCP-M2e discs before infection (on day 28) with 1LD₅₀ of A/WSN/33 influenza virus (H1N1). Antibody titers are expressed as an antibody endpoint titer, defined as the O.D. value that is 3-fold greater than the background value obtained from a 1:50 dilution of pre-immune serum from BALB/c mice. Body weight was measured 8 days post-infection and is expressed as the percentage of initial weight. Data represent the mean ± SEM of 20 mice.

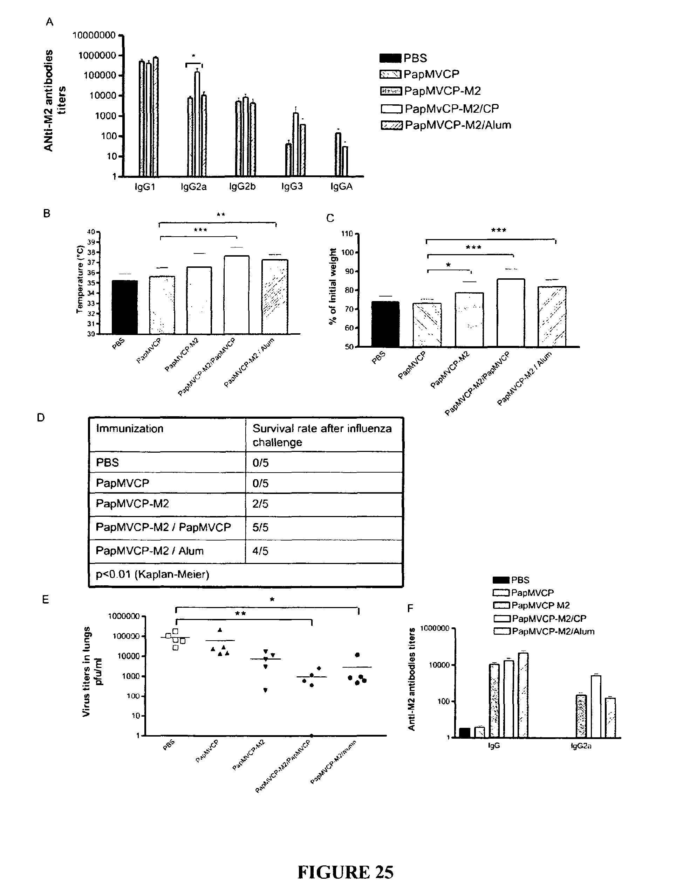

**Figure 25** presents the results of (A) IgG isotyping for influenza M2e antibody response after 3 immunisations, (B) rectal temperature measurement, (C) body weight, (D) survival rate and results of Kaplan-Meier analysis, (E) lung viral titers and (F) anti-M2 antibody titers in the bronchoalveolar lavage from BALB/c mice that were immunized 3 times (at days 0, 14 and 28) with: PBS, PapMVCP VLPs (PapMVCP), PapMVCP-M2e VLPs (PapMVCP-M2e), or PapMVCP-M2e VLPs adjuvanted with either PapMVCP (PapMVCP-M2e/CP) or with alum (PapMVCP-M2e/Alum) and subsequently challenged with influenza virus. Body weight and anti-M2 antibody titers in the lung were determined 7 days post-infection. On day 38, mice were bled and serum antibody levels (titration) determined. On day 42, they were infected with 4LD₅₀ of A/WSN/33 Influenza virus (H1N1). On day 49, half of the mice in each group (5/10) were sacrificed for bronchoalveolar lavages (BAL) and lung viral titration. Remaining mice (5/10) were analysed for survival rate. Antibody titer results are expressed as an antibody endpoint titer, defined as the O.D. value that is 3-fold greater than the background value obtained from either a 1:50 dilution of pre-immune serum from BALB/c mice (A), or from a 1:5 dilution of pre-immune BAL from BALB/c mice (F). Data represent the mean ± SEM of 10 (A-C) or 5 mice (D-F).

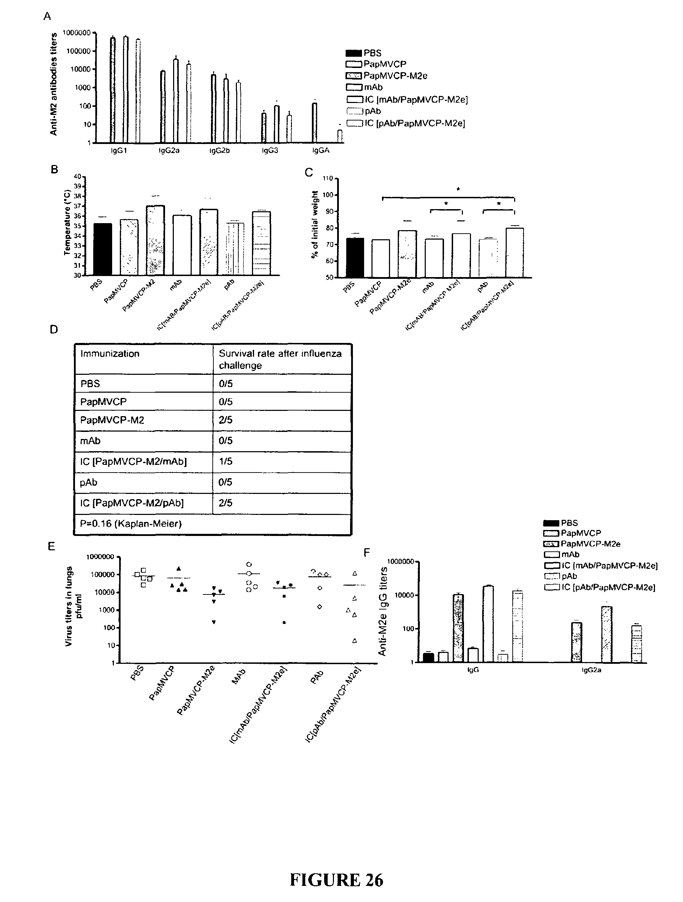

**Figure 26** presents the results of (A) IgG isotyping for the influenza M2 epitope antibody response after 3 immunisations, (B) rectal temperature measurement, (C) body weight, (D) survival rate and results of Kaplan-Meier analysis, (E) lung viral titers and (F) lung anti-M2 antibody titers in BALB/c mice that were immunized 3 times (at days 0, 14 and 28) with: PBS, PapMVCP VLPs (PapMVCP), PapMVCP-M2e VLPs (PapMVCP-M2e), anti-PapMVCP monoclonal antibodies (mAb), PapMVCP-M2e VLPs opsonised with anti-PapMVCP monoclonal antibodies (mAb) (IC [mAb/PapMVCP-M2e]), polyclonal anti-PapMVCP-M2e antibodies (pAb), or PapMVCP-M2e VLPs opsonised with anti-PapMVCP polyclonal antibodies (pAb) (IC [pAb/PapMVCP-M2e]). Body weight and lung anti-M2 antibody titers were determined 7 days post-infection. On day 38, mice were bled for serum antibody titration determination. On day 42, mice were infected with 4 LD₅₀ of A/WSN/33 Influenza virus (H1N1). On day 49, half of the mice in each group (5/10) were sacrificed for bronchoalveolar lavage (BAL) and lung viral titration, while the remaining mice (5/10) were analysed for survival rate. Data represent the mean ± SEM of 10 (A-C) or 5 mice (D-F). Antibody titer results are expressed as an antibody endpoint titer, defined as the O.D. value that is 3-fold higher than the background value obtained with either a 1:50 dilution of pre-immune serum from BALB/c mice (A), or with a 1:5 dilution of pre-immune BAL from BALB/c mice (F).

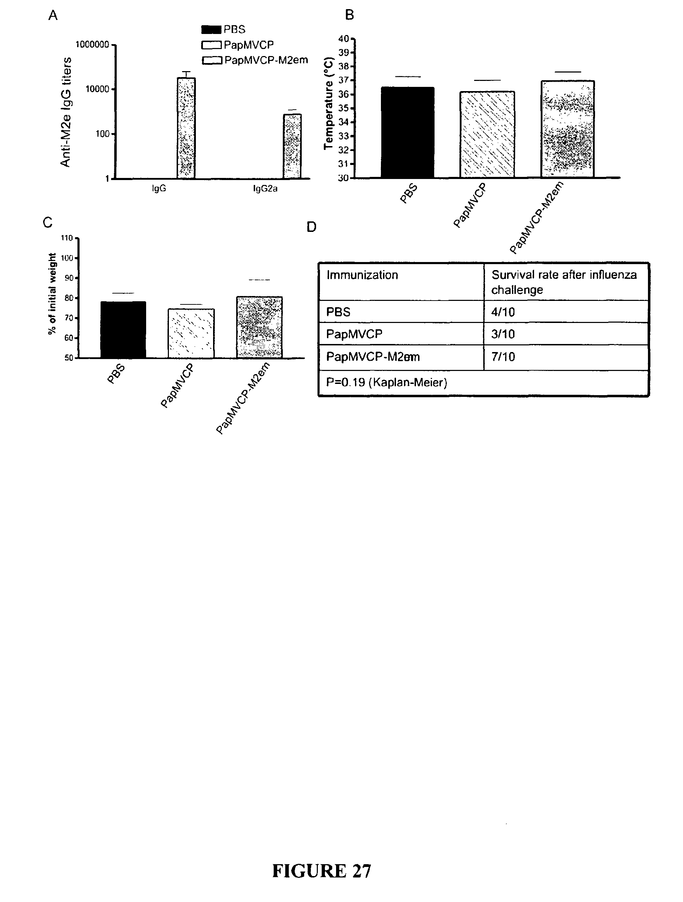

**Figure 27** presents (A) IgG isotyping for the influenza M2 epitope antibody response, (B) rectal temperature measurement, (C) body weight, and (D) survival rate and results of Kaplan-Meier analysis for BALB/c mice that were immunized 3 times (at days 0, 14 and 28) with: PBS, PapMVCP VLPs (PapMVCP), or a mixture of non-ultracentrifuged PapMVCP-M2em VLPs and lower molecular weight proteins (discs, dimmers and monomers)(PapMVCP-M2em). Body weight was measured 8 days after challenge. On day 38, mice were bled for serum antibody titration determinations. On day 42, they were infected with 1 LD₅₀ of A/WSN/33 influenza virus (H1N1). Results are expressed as an antibody endpoint titer, defined as being the O.D. value that is 3-fold higher than the background value obtained with a 1:50 dilution of pre-immune serum from BALB/c mice.

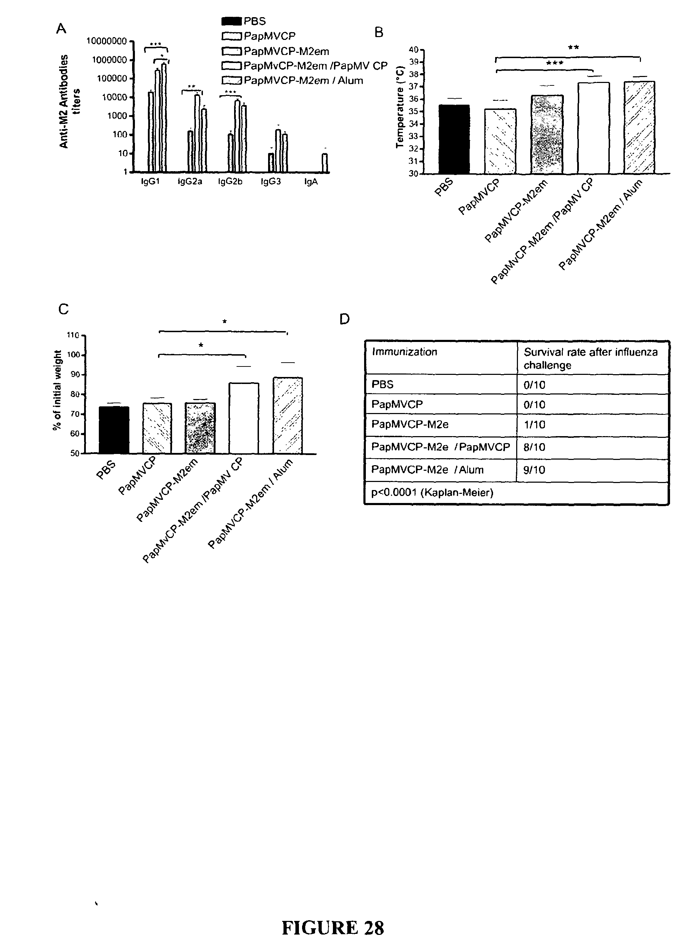

**Figure 28** presents (A) IgG isotyping for the influenza M2 epitope antibody response, (B) rectal temperature measurement, (C) body weight, and (D) survival rate and results of Kaplan-Meier analysis for BALB/c mice that were immunized 3 times (at days 0, 14 and 28) with: PBS, PapMVCP VLPs (PapMVCP), PapMVCP-M2e mixture (PapMVCP-M2em) comprised of VLPs and lower molecular weight proteins (discs, dimmers and monomers), PapMVCP-M2em adjuvanted with PapMVCP (PapMVCP-M2em/PapMVCP) or with alum (PapMVCP-M2em/Alum). Body weight was measured 7 d after challenge. On day 38, mice were bled for serum antibody titration determinations. On day 42, they were infected with 4 LD₅₀ of A/WSN/33 Influenza virus (H1N1). Results are expressed as an antibody endpoint titer, defined as the O.D. value that is 3-fold higher than the background value obtained with a 1:50 dilution of pre-immune serum from BALB/c mice. Data represent the mean ± SEM of 10 mice (D- F).

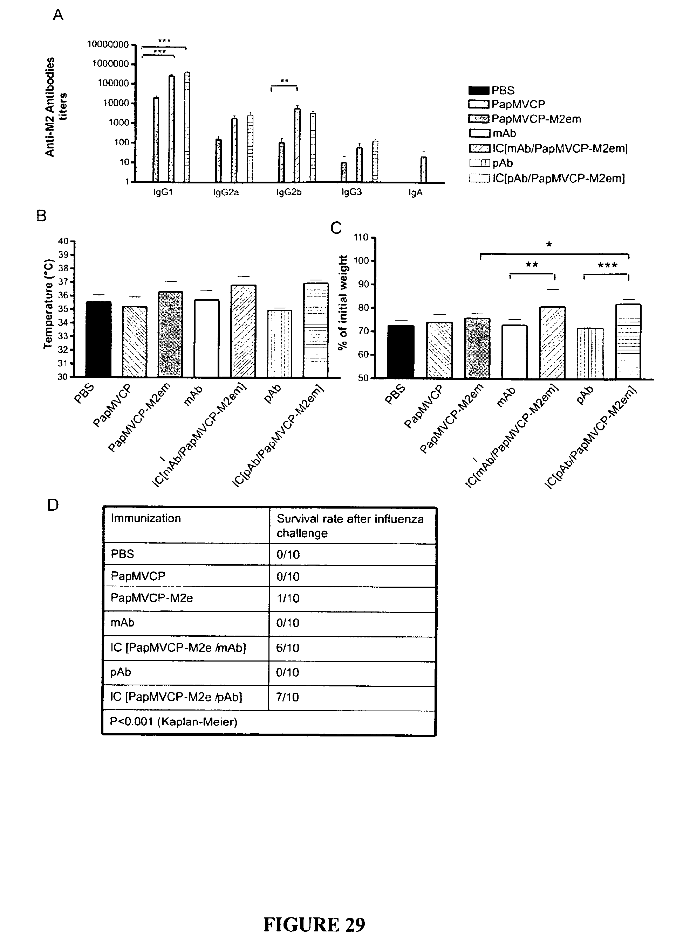

**Figure 29** presents the results of (A) IgG isotyping for the influenza M2 epitope antibody response after 3 immunisations, (B) rectal temperature measurement, (C) body weight, (D) survival rate and results of Kaplan-Meier analysis for BALB/c mice that were immunized 3 times (at days 0, 14 and 28) with: PBS, PapMVCP VLPs (PapMVCP), PapMVCP-M2e mixture (PapMVCP-M2em) comprised of VLPs and lower molecular weight proteins (discs, dimers and monomers), anti-PapMVCP monoclonal antibodies (mAb), PapMVCP-M2em opsonised with anti-PapMVCP monoclonal antibodies (mAb) (IC [mAb/PapMVCP-M2em]), polyclonal anti-PapMVCP-M2e antibodies (pAb), or PapMVCP-M2em VLPs opsonised with anti-PapMVCP polyclonal antibodies (pAb) (IC [pAb/PapMVCP-M2e]). Body weight was determined 7 days post-infection. On day 38, mice were bled for serum antibody titration determination. On day 42, mice were infected with a 4 LD₅₀ dose of A/WSN/33 Influenza virus (H1N1). On day 49 mice were analysed for survival rate. Data represent the mean ± SEM of 10. Antibody titer results are expressed as an antibody endpoint titer, defined as the O.D. value that is 3-fold higher than the background value obtained with either a 1:50 dilution of pre-immune serum from BALB/c mice (A).

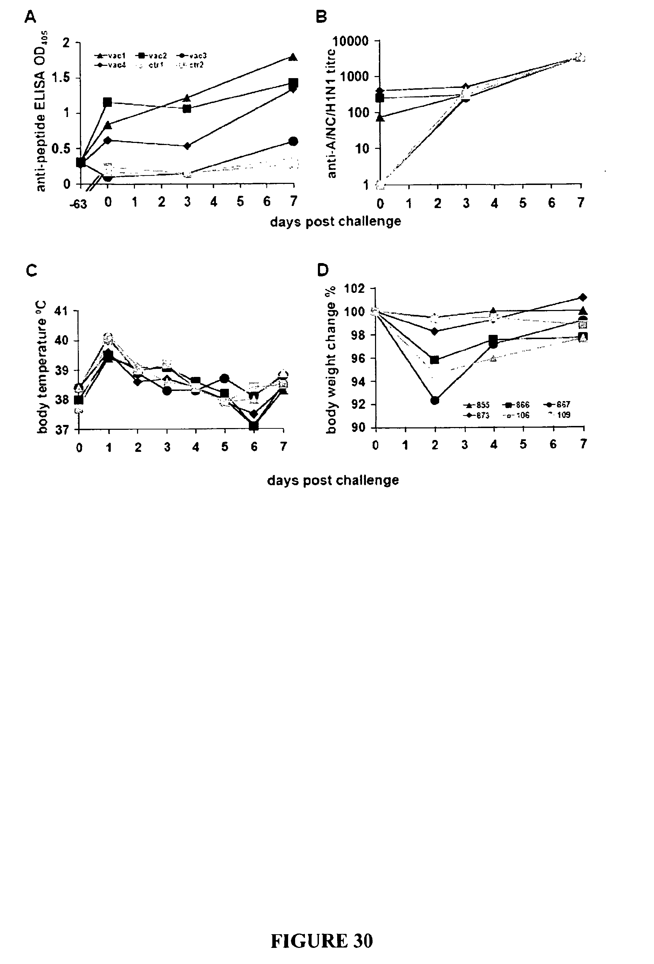

**Figure 30** presents serum IgG antibody response to **(A)** the M2e peptide or **(B)** virus infected cells in ferrets vaccinated three times with 25 µg of PapMV-M2e VLPs, and clinical signs observed after intranasal challenge with 10⁵ infectious doses of the PR8/New Caledonia vaccine reassortant **(C)** body temperature and **(D)** body weight in the same animals. Vaccinated animals are shown in black, control animals are shown in grey.

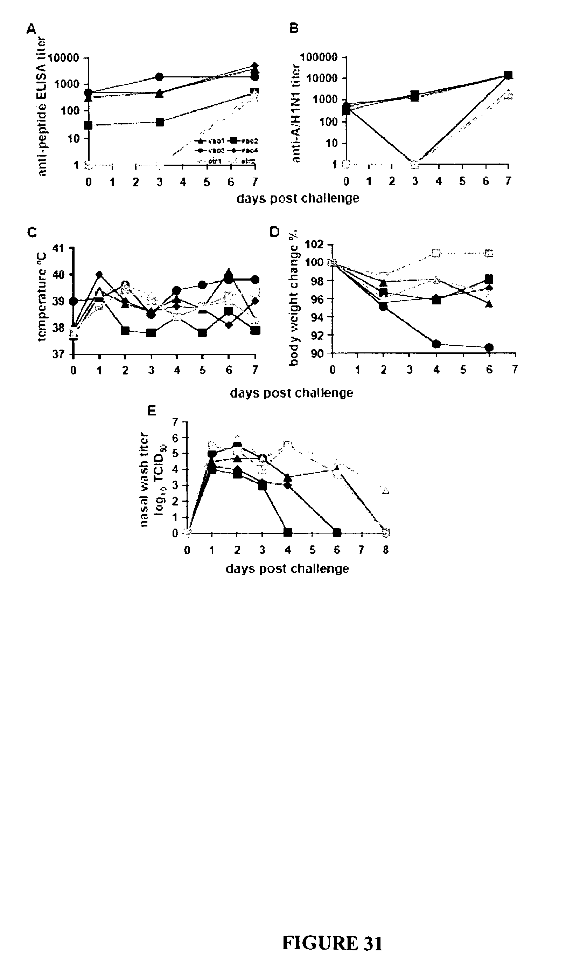

**Figure 31** presents **(A)** serum IgG antibody titers against the M2 peptide, **(B)** serum IgG antibody titers against virus-infected cells, **(C)** body temperature, **D)** body weight, and **(E)** viral titers in nasal lavage fluids (measured by limited dilution daily for the first four days, and every second day thereafter) in ferrets vaccinated twice with 25µg and a third time with 250µg of PapMV-M2e VLPs, followed by intranasal challenge with 10⁵ TCID₅₀ influenza virus strain A/USSR/77. Vaccinated animals are represented in black, control animals are shown in grey.

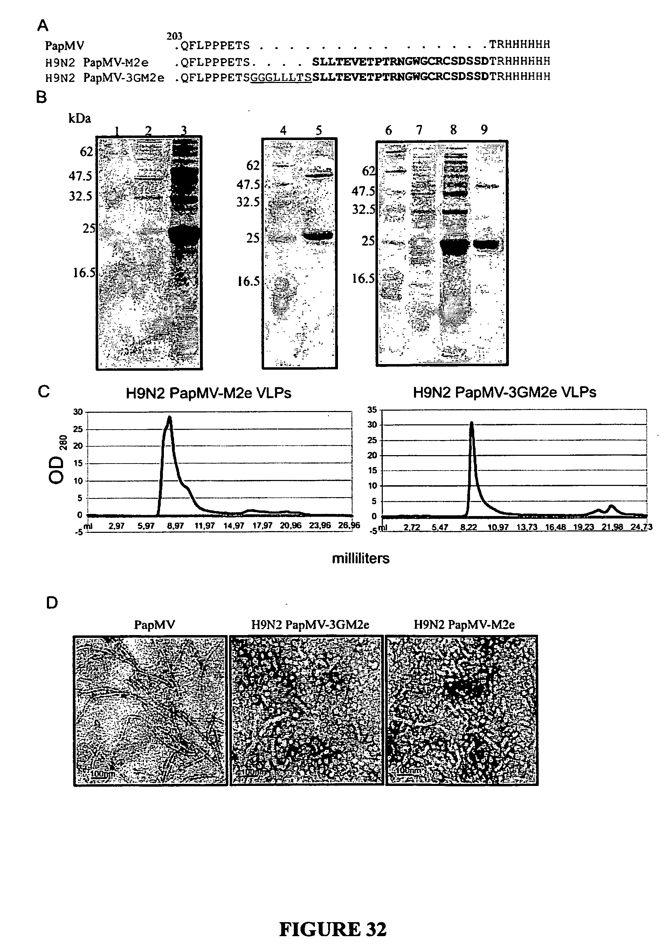

**Figure 32** presents (A) the C-terminal sequences of PapMV coat proteins comprising the H9N2 M2e peptide **(bold)** fused between the PapMV CP and a 6x His tag (6His) located at the C-terminus of the protein, with (H9N2 PapMV-3GLM2e) or without (H9N2 PapMV-M2e) a glycine spacer (underlined) inserted between the PapMV CP and the H9N2 M2e, (B) SDS-PAGE of the purified H9N2 PapMV-M2e and H9N2 PapMV-3GLM2e proteins (Lanes: 1. Molecular weight markers, 2. total bacterial lysates prior to induction of expression of the protein H9N2 PapMV-3M2e with IPTG, 3. total bacterial lysates after induction of expression of the protein H9N2 PapMV-3M2e with IPTG, 4. on a second gel, molecular weight markers, 5. recombinant purified by H9N2 PapMV-M2e protein by affinity on a nickel column, 6. on a third gel, molecular weight markers, 7. total bacterial lysates prior to induction of expression of the protein H9N2 PapMV-3GLM2e with IPTG, 8. total bacterial lysates after induction of expression of the protein H9N2 PapMV-3GLM2e with IPTG, 9. purified H9N2 PapMV-3GLM2e protein), (C) FPLC profile of the purified H9N2 PapMV-M2e and H9N2 PapMV-3GLM2e VLPs, and (D) electron micrographs of PapMV VLPs, H9N2 PapMV-3GLM2e VLPs and H9N2 PapMV-M2e VLPs (Bars are 100 nm).

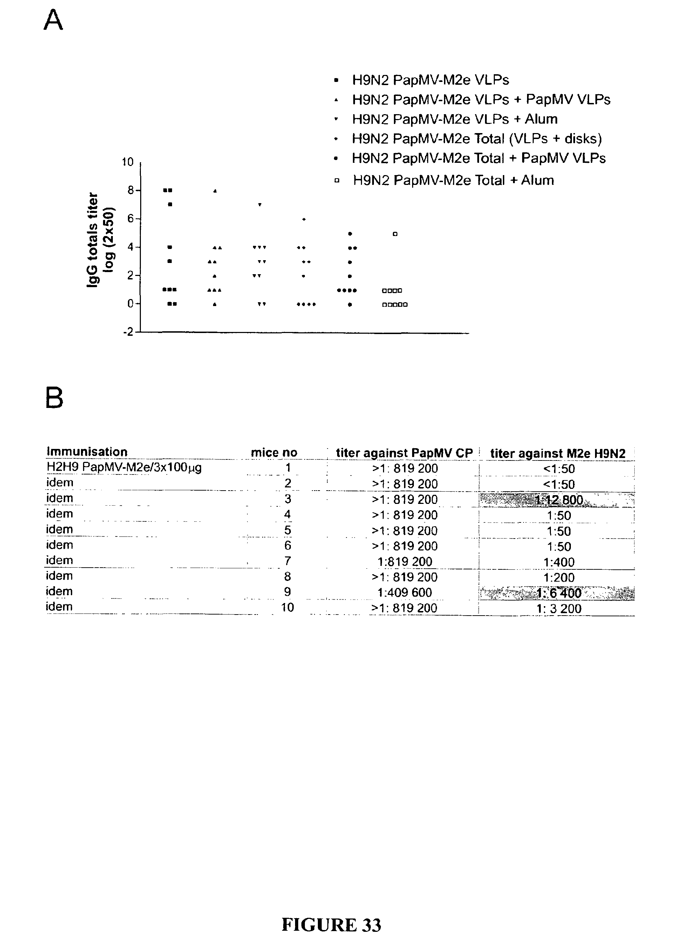

**Figure 33** presents A) the results of ELISA analysis of serum from mice injected 3 times at 2-week intervals with H9N2 PapMV-M2e preparations as indicated (Each mouse is represented by a dot. The baseline represents the background level obtained with the pre-serum.), and B) the IgG titers against the PapMV CP and H9N2 M2e peptide in the treated mice.

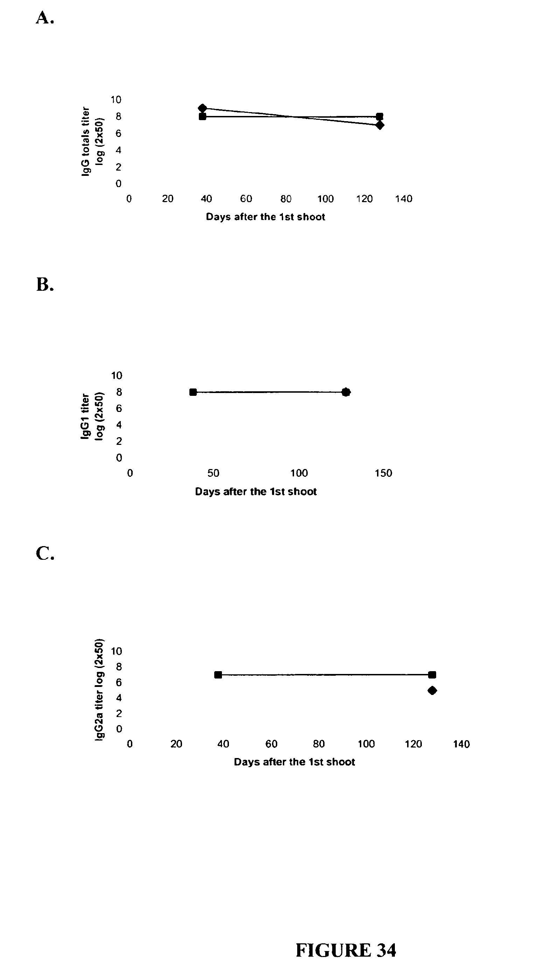

**Figure 34** presents A) the total IgG levels for two mice that were injected 3 times at 2-week intervals with 100µg H9N2 PapMV-M2e and that reacted well to the M2e peptide over a period of 140 days after the first injection, B) the level of IgG1 IgG isotype in the same sera as (A) over the same period, and C) the amount of IgG2a IgG isotype in the same sera as (A) over the same period. For the second mouse (diamond), only sera collected at day 140 was used.

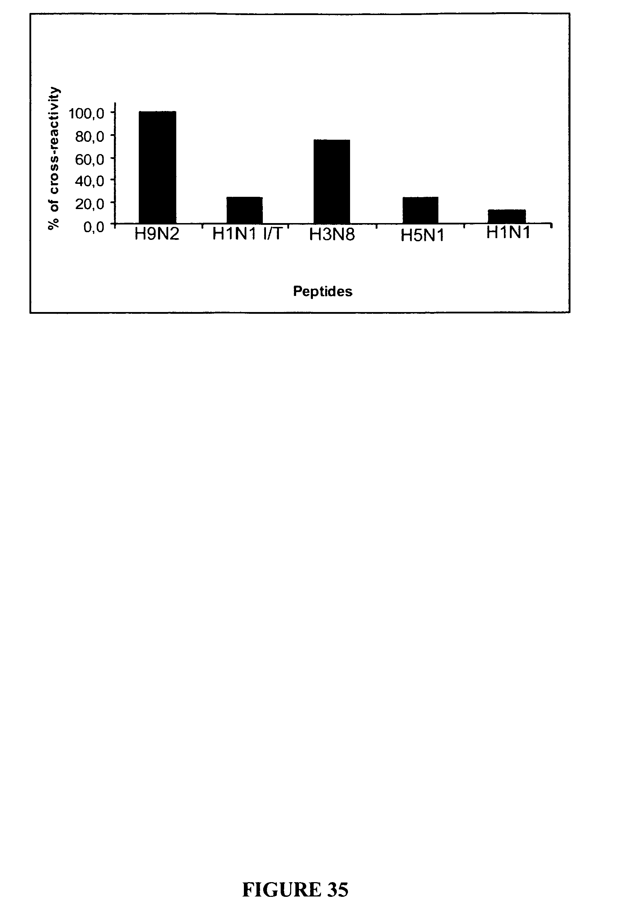

**Figure 35** presents the results of ELISA of the H9N2 PapMV-M2e antiserum obtained from mice that were injected 3 times at 2-week intervals with 100µg H9N2 PapMV-M2e and that reacted well to the M2e peptide toward M2e peptides from different strains of the influenza virus. The results are shown as a percentage of the reactivity on the reference H9N2 peptide [SEQ ID NO:40].

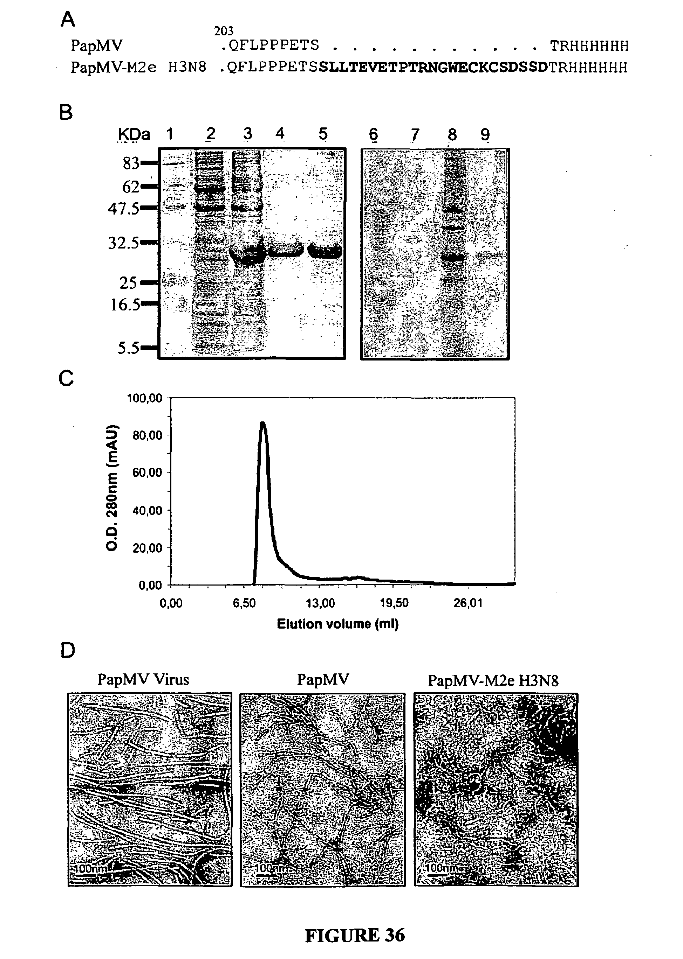

**Figure 36** presents (A) the C-terminal sequences of wild-type PapMV coat protein and PapMV coat protein comprising the H3N8 M2e peptide **(bold)** fused between the coat protein sequence and a 6x His tag (6His), (B) an SDS-Page gel showing the purification of the recombinant proteins (Lane: 1. Molecular weight markers 2. Total bacterial lysates of bacteria prior to induction of the expression of the PapMV coat protein with IPTG 3. Total bacterial lysates after induction of expression of the coat PapMV protein with IPTG 4. Purified total PapMV-M2e H3N8 protein, 5. High speed pellet containing VLPs of the purified PapMV-M2e H3N8 protein. 6. On a second gel, molecular weight markers, 7. Total bacterial lysates prior to induction of expression of the protein PapMV-M2e H3N8 8. Total bacterial lysate of bacteria after induction of expression of the protein PapMV-M2e H3N8 with IPTG. 9. High speed pellet containing VLPs of the purified PapMV-M2e H3N8 protein), (C) FPLC profile of the purified PapMV-M2e H3N8 VLPs, and (D) electron micrographs of PapMV virus (left-hand panel), PapMV recombinant virus like particles (VLPs) (centre panel) and H3N8 PapMV-M2e VLPs (right-hand panel).

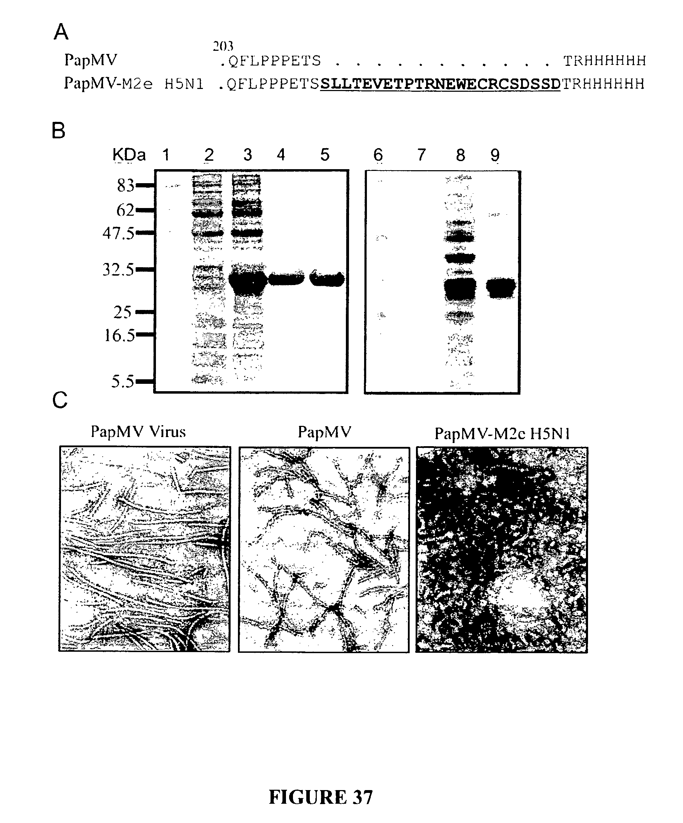

**Figure 37** presents (A) the C-terminal sequences of wild-type PapMV coat protein and PapMV coat protein comprising the H5N1 M2e peptide (bold) fused between the coat protein sequence and a 6x His tag (6His), (B) an SDS-Page gel showing the purification of the recombinant proteins (Lanes: 1. Molecular weight markers 2. Total bacterial lysates of bacteria prior induction of the expression of the protein PapMV VLPs with IPTG 3. Total bacterial lysates of bacteria after induction of expression of the protein PapMV VLPs with IPTG 4. Purified total PapMV-M2e H5N1 protein, 5. High speed pellet containing VLPs of the purified PapMV-M2e H5N1 protein. 6. On another gel, molecular weight markers, 7. Total bacterial lysates of bacteria prior induction of the expression of the protein PapMV-M2e H5N1 8. Total bacterial lysate of bacteria after induction of expression of the protein PapMV-M2e H5N1 with IPTG. 9. High speed pellet containing VLPs of the purified PapMV-M2e H5N1 protein), and (C) electron micrographs of PapMV virus (left-hand panel), PapMV recombinant virus like particles (VLPs) (centre panel) and H5N1 PapMV-M2e VLPs (right-hand panel).

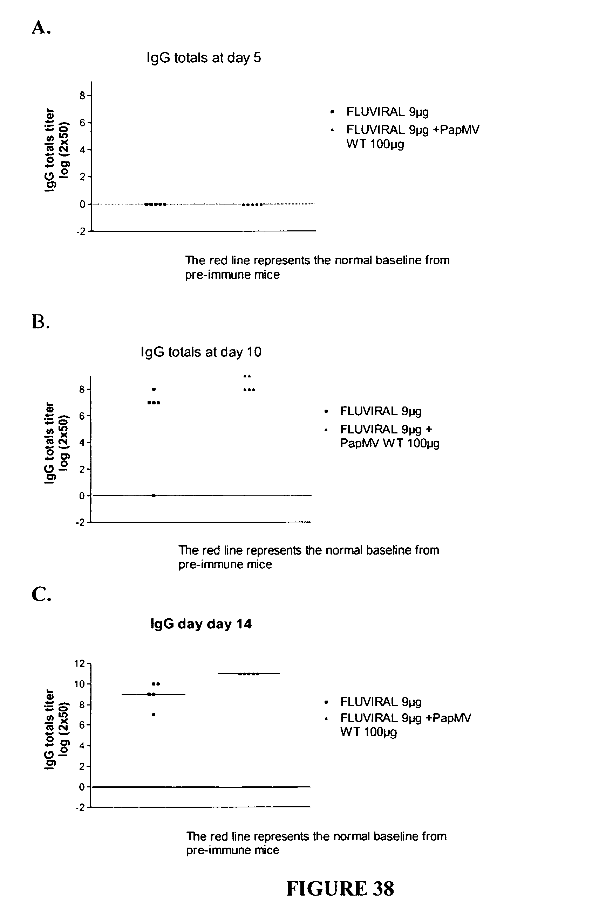

**Figure 38** presents charts showing the results of adjuvanting the Fluviral® influenza vaccine with isolated PapMV in mice. Mice were immunized.with the Fluviral® vaccine or with Fluviral® vaccine adjuvanted with PapMV WT virus. Mice were bled on day 5 (A), day 10 (B) or day 14 (C) after the immunisation.

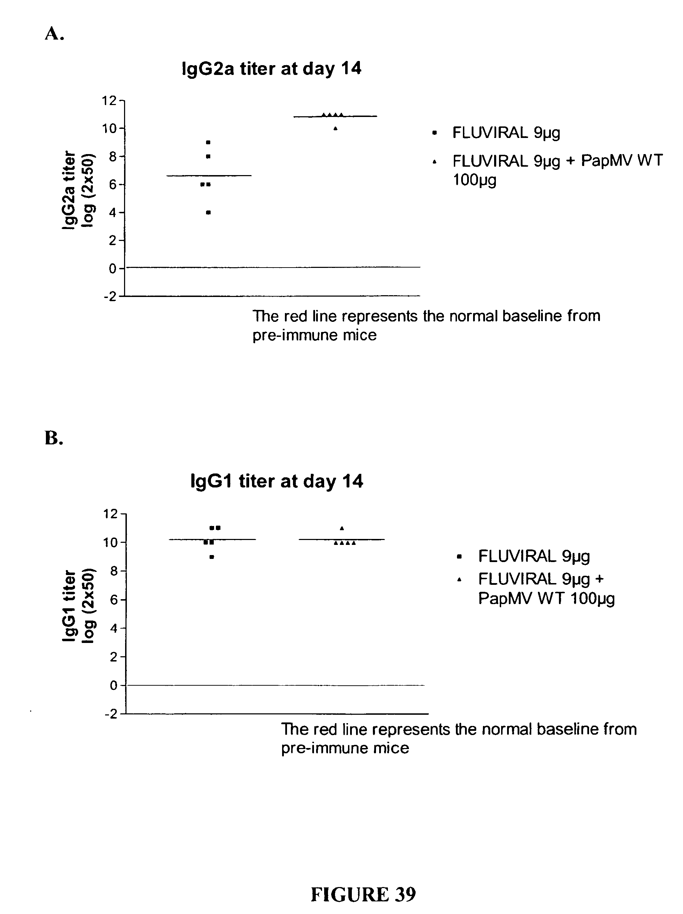

**Figure 39** presents the amounts of IgG2a (A) and the IgG1 (B) antibody isotypes in mice immunized with the Fluviral® vaccine or with the Fluviral® vaccine adjuvanted with PapMV WT virus 14 days after immunization.

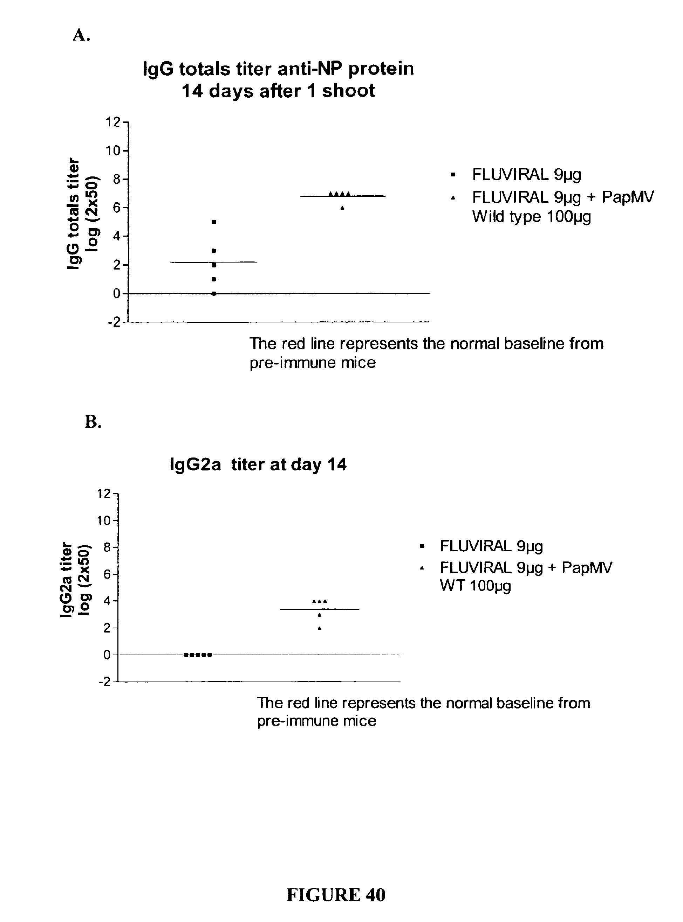

**Figure 40** presents the results of ELISA to show the immune response of mice immunized with the Fluviral® vaccine or with the Fluviral® vaccine adjuvanted with PapMV WT toward the recombinant NP protein: (A) shows the total IgG titers and (B) shows the IgG2a titers in the mice sera 14 days after immunisation.

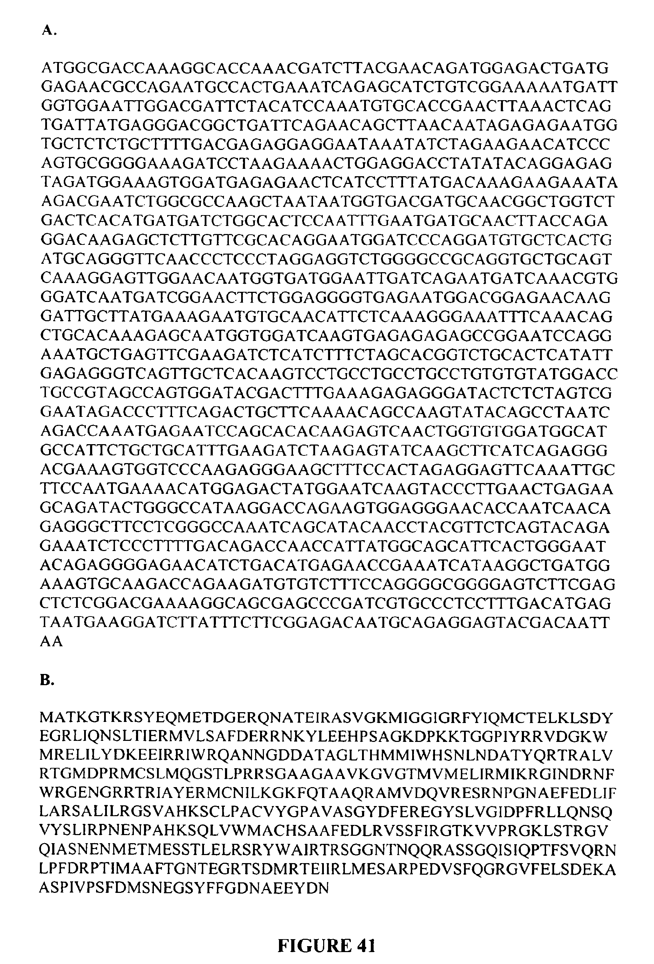

**Figure 41** presents (A) the nucleotide sequence encoding the NP protein from influenza virus strain A/WSN/33 [SEQ ID NO:63], and (B) the amino acid sequence of the NP protein [SEQ ID NO:64] encoded by the sequence provided in (A).

### DETAILED DESCRIPTION OF THE INVENTION

An antigen-presenting system (APS) comprising one or more antigens in combination with a papaya mosaic virus (PapMV) or a VLP derived from papaya mosaic virus is provided. In accordance with one embodiment of the present invention, the APS is capable of inducing a humoral immune response, a cellular immune response, or both, in an animal. The APS is thus suitable for use as a vaccine, which may require an active participation of one or both of these two branches of the immune system.

In one embodiment of the present invention, the APS comprises one or more influenza virus antigens and is suitable for use as a vaccine for influenza.

Unless defined otherwise, all technical and scientific terms used herein have the same meaning as commonly understood by one of ordinary skill in the art to which this invention belongs.

As used herein, the term "about" refers to approximately a +/-10% variation from a given value. It is to be understood that such a variation is always included in any given value provided herein, whether or not it is specifically referred to.

The term "adjuvant," as used herein, refers to an agent that augments, stimulates, actuates, potentiates and/or modulates an immune response in an animal. An adjuvant may or may not have an effect on the immune response in itself.

As used herein, a "chimeric protein" is a protein that is created when two or more genes that normally code for two separate proteins or protein fragments recombine, either naturally or as the result of human intervention, to provide a polynucleotide encoding a protein (the "chimeric protein") that is a combination of all or part of each of those two proteins. In the context of the present invention, a "fusion protein" is considered to be a "chimeric protein."

The expression "fusion coat protein" is used herein to refer to a fusion protein in which one of the proteins in the fusion is a PapMV coat protein.

The term "immune response," as used herein, refers to an alteration in the reactivity of the immune system of an animal in response to an antigen or antigenic material and may involve antibody production, induction of cell-mediated immunity, complement activation, development of immunological tolerance, or a combination thereof.

The terms "effective immunoprotective response" and "immunoprotection," as used herein, mean an immune response that is directed against one or more antigen so as to protect against disease and/or infection by a pathogen in a vaccinated animal. For purposes of the present invention, protection against disease and/or infection by a pathogen includes not only the absolute prevention of the disease or infection, but also any detectable reduction in the degree or rate of disease or infection, or any detectable reduction in the severity of the disease or any symptom or condition resulting from infection by the pathogen in the vaccinated animal as compared to an unvaccinated infected or diseased animal. An effective immunoprotective response can be induced in animals that were not previously suffering from the disease, have not previously been infected with the pathogen and/or do not have the disease or infection at the time of vaccination. An effective immunoprotective response can also be induced in an animal already suffering from the disease or infected with the pathogen at the time of vaccination. Immunoprotection can be the result of one or more mechanisms, including humoral and/or cellular immunity.

The terms "immune stimulation" and "immunostimulation" as used interchangeably herein, refer to the ability of a molecule, such as a PapMV or PapMV VLP, that is unrelated to an animal pathogen or disease to provide protection to against infection by the pathogen or against the disease by stimulating the immune system and/or improving the capacity of the immune system to respond to the infection or disease. Immunostimulation may have a prophylactic effect, a therapeutic effect, or a combination thereof.

A "recombinant virus" is one in which the genetic material of a naturally-occurring virus has combined with other genetic material.

"Naturally-occurring," as used herein, as applied to an object, refers to the fact that an object can be found in nature. For example, an organism (including a virus), or a polypeptide or polynucleotide sequence that is present in an organism that can be isolated from a source in nature and which has not been intentionally modified by man in the laboratory is naturally-occurring.

The terms "polypeptide" or "peptide" as used herein is intended to mean a molecule in which there is at least four amino acids linked by peptide bonds.

The expression "viral nucleic acid," as used herein, may be the genome (or a majority thereof) of a virus, or a nucleic acid molecule complementary in base sequence to that genome. A DNA molecule that is complementary to viral RNA is also considered viral nucleic acid, as is a RNA molecule that is complementary in base sequence to viral DNA.

The term "virus-like particle" (VLP), as used herein, refers to a self-assembling particle which has a similar physical appearance to a virus particle. The VLP may or may not comprise viral nucleic acids. VLPs are generally incapable of replication.

The term "pseudovirus," as used herein, refers to a VLP that comprises nucleic acid sequences, such as DNA or RNA, including nucleic acids in plasmid form. Pseudoviruses are generally incapable of replication.

The term "vaccine," as used herein, refers to a material capable of producing an immune response.

The terms "immunogen" and "antigen" as used herein refer to a molecule, molecules, a portion or portions of a molecule, or a combination of molecules, up to and including whole cells and tissues, which are capable of inducing an immune response in a subject alone or in combination with an adjuvant. The immunogen/antigen may comprise a single epitope or may comprise a plurality of epitopes. The term thus encompasses peptides, carbohydrates, proteins, nucleic acids, and various microorganisms, in whole or in part, including viruses, bacteria and parasites. Haptens are also considered to be encompassed by the terms "immunogen" and "antigen" as used herein.

The terms "immunization" and "vaccination" are used interchangeably herein to refer to the administration of a vaccine to a subject for the purposes of raising an immune response and can have a prophylactic effect, a therapeutic effect, or a combination thereof. Immunization can be accomplished using various methods depending on the subject to be treated including, but not limited to, intraperitoneal injection (i.p.), intravenous injection (i.v.), intramuscular injection (i.m.), oral administration, intranasal administration, spray administration and immersion.

As used herein, the terms "treat," "treated," or "treating" when used with respect to a disease or pathogen refers to a treatment which increases the resistance of a subject to the disease or to infection with a pathogen (*i.e.* decreases the likelihood that the subject will contract the disease or become infected with the pathogen) as well as a treatment after the subject has contracted the disease or become infected in order to fight a disease or infection (for example, reduce, eliminate, ameliorate or stabilise a disease or infection).

The term "prime" and grammatical variations thereof, as used herein, means to stimulate and/or actuate an immune response against an antigen in an animal prior to administering a booster vaccination with the antigen.

The term "subject" or "patient" as used herein refers to an animal in need of treatment.

The term "animal," as used herein, refers to both human and non-human animals, including, but not limited to, mammals, birds and fish, and encompasses domestic, farm, zoo, laboratory and wild animals, such as, for example, cows, pigs, horses, goats, sheep or other hoofed animals, dogs, cats, chickens, ducks, non-human primates, guinea pigs, rabbits, ferrets, rats, hamsters and mice.

The term "substantially identical," as used herein in relation to a nucleic acid or amino acid sequence indicates that, when optimally aligned, for example using the methods described below, the nucleic acid or amino acid sequence shares at least 70%, at least 75%, at least 80%, at least 85%, at least 90%, at least 95%, at least 96%, at least 97%, at least 98% or at least 99% sequence identity with a defined second nucleic acid or amino acid sequence (or "reference sequence"). "Substantial identity" may be used to refer to various types and lengths of sequence, such as full-length sequence, functional domains, coding and/or regulatory sequences, promoters, and genomic sequences. Percent identity between two amino acid or nucleic acid sequences can be determined in various ways that are within the skill of a worker in the art, for example, using publicly available computer software such as Smith Waterman Alignment (Smith, T. F. and M. S. Waterman (1981) J Mol Biol 147:195-7); "BestFit" (Smith and Waterman, Advances in Applied Mathematics, 482-489 (1981)) as incorporated into GeneMatcher Plus™, Schwarz and Dayhof (1979) Atlas of Protein Sequence and Structure, Dayhof, M. O., Ed pp 353-358; BLAST program (Basic Local Alignment Search Tool (Altschul, S. F., W. Gish, et al. (1990) J Mol Biol 215: 403-10), and variations thereof including BLAST-2, BLAST-P, BLAST-N, BLAST-X, WU-BLAST-2, ALIGN, ALIGN-2, CLUSTAL, and Megalign (DNASTAR) software. In addition, those skilled in the art can determine appropriate parameters for measuring alignment, including algorithms needed to achieve maximal alignment over the length of the sequences being compared. In general, for amino acid sequences, the length of comparison sequences will be at least 10 amino acids. One skilled in the art will understand that the actual length will depend on the overall length of the sequences being compared and may be at least 20, at least 30, at least 40, at least 50, at least 60, at least 70, at least 80, at least 90, at least 100, at least 110, at least 120, at least 130, at least 140, at least 150, or at least 200 amino acids, or it may be the full-length of the amino acid sequence. For nucleic acids, the length of comparison sequences will generally be at least 25 nucleotides, but may be at least 50, at least 100, at least 125, at least 150, at least 200, at least 250, at least 300, at least 350, at least 400, at least 450, at least 500, at least 550, or at least 600 nucleotides, or it may be the full-length of the nucleic acid sequence.

The terms "corresponding to" or "corresponds to" indicate that a nucleic acid sequence is identical to all or a portion of a reference nucleic acid sequence. In contradistinction, the term "complementary to" is used herein to indicate that the nucleic acid sequence is identical to all or a portion of the complementary strand of a reference nucleic acid sequence. For illustration, the nucleic acid sequence "TATAC" corresponds to a reference sequence "TATAC" and is complementary to a reference sequence "GTATA."

### ANTIGEN-PRESENTING SYSTEM (APS)

An antigen-presenting system (APS) of the present invention comprises one or more antigens in combination with a papaya mosaic virus (PapMV) or a VLP derived from a PapMV coat protein. By "derived from" it is meant that the VLP comprises coat proteins that have an amino acid sequence substantially identical to the sequence of the wild-type coat protein and may optionally include one or more antigens attached to the coat protein, as described in more detail below. The PapMV coat protein can comprise the sequence of the wild-type coat protein or a modified version thereof which is capable of multimerization and self-assembly to form a VLP. In one embodiment of the present invention, as described in detail below, the APS comprises one or more antigens from an influenza virus.

The one or more antigens comprised by the APS can be conjugated to a coat protein of the PapMV or PapMV VLP, or they may be non-conjugated (*i.e.* separate from the PapMV or PapMV VLP), or the APS may include both conjugated and non-conjugated antigens. In this latter context, the non-conjugated antigens are referred to as additional isolated antigens (AIAs). The AIAs may be the same as or different than the conjugated antigen(s). Conjugation can be, for example, by genetic fusion with the coat protein, or binding via covalent, non-covalent or affinity means.

The PapMV or PapMV VLP included in the APS thus acts as an immunopotentiator capable of potentiating an immune response in an animal, and optionally as a carrier for the antigen(s) comprised by the APS. In accordance with one embodiment of the present invention, the APS is capable of inducing a humoral and/or cellular immune response in an animal and thus is suitable for use as vaccines, for example an influenza vaccine.

### Papaya mosaic virus (PapMV) and PapMV VLPs

The APS of the present invention comprises either PapMV or PapMV VLPs. PapMV VLPs are formed from recombinant PapMV coat proteins that have multimerised and self-assembled to form a VLP. When assembled, each VLP comprises a long helical array of coat protein subunits. The wild-type virus comprises over 1200 coat protein subunits and is about 500nm in length. PapMV VLPs that are either shorter or longer than the wild-type virus can still, however, be effective. In one embodiment of the present invention, the VLP comprises at least 40 coat protein subunits. In another embodiment, the VLP comprises between about 40 and about 1600 coat protein subunits. In an alternative embodiment, the VLP is at least 40nm in length. In another embodiment, the VLP is between about 40nm and about 600nm in length.

The VLPs of the present invention can be prepared from a plurality of recombinant coat proteins having identical amino acid sequences, such that the final VLP when assembled comprises identical coat protein subunits, or the VLP can be prepared from a plurality of recombinant coat proteins having different amino acid sequences, such that the final VLP when assembled comprises variations in its coat protein subunits.

The coat protein used to form the VLP can be the entire PapMV coat protein, or part thereof, or it can be a genetically modified version of the PapMV coat protein, for example, comprising one or more amino acid deletions, insertions, replacements and the like, provided that the coat protein retains the ability to multimerise and assemble into a VLP. The amino acid sequence of the wild-type PapMV coat (or capsid) protein is known in the art (see, Sit, et al., 1989, J. Gen. Virol., 70:2325-2331, and GenBank Accession No. NP_044334.1) and is provided herein as SEQ ID NO:1 (see Figure 1A). The nucleotide sequence of the PapMV coat protein is also known in the art (see, Sit, et al., *ibid.,* and GenBank Accession No. NC_001748 (nucleotides 5889-6536)) and is provided herein as SEQ ID NO:2 (see Figure 1B).

As noted above, the amino acid sequence of the recombinant PapMV coat protein comprised by the VLP need not correspond precisely to the parental (wild-type) sequence, *i.e.* it may be a "variant sequence." For example, the recombinant protein may be mutagenized by substitution, insertion or deletion of one or more amino acid residues so that the residue at that site does not correspond to either the parental (reference) sequence. One skilled in the art will appreciate, however, that such mutations will not be extensive and will not dramatically affect the ability of the recombinant coat protein to multimerise and assemble into a VLP. The ability of a variant version of the PapMV coat protein to assemble into multimers and VLPs can be assessed, for example, by electron microscopy following standard techniques, such as the exemplary methods set out in the Examples provided herein.

Recombinant coat proteins that are fragments of the wild-type protein that retain the ability to multimerise and assemble into a VLP *(i.e.* are "functional" fragments) are, therefore, also contemplated by the present invention. For example, a fragment may comprise a deletion of one or more amino acids from the N-terminus, the C-terminus, or the interior of the protein, or a combination thereof. In general, functional fragments are at least 100 amino acids in length. In one embodiment of the present invention, functional fragments are at least 150 amino acids, at least 160 amino acids, at least 170 amino acids, at least 180 amino acids, and at least 190 amino acids in length. Deletions made at the N-terminus of the protein should generally delete fewer than 25 amino acids in order to retain the ability of the protein to multimerise.

In accordance with the present invention, when a recombinant coat protein comprises a variant sequence, the variant sequence is at least about 70% identical to the reference sequence. In one embodiment, the variant sequence is at least about 75% identical to the reference sequence. In other embodiments, the variant sequence is at least about 80%, at least about 85%, at least about 90%, at least about 95%, and at least about 97% identical to the reference sequence. In a specific embodiment, the reference amino acid sequence is SEQ ID NO:1.

In one embodiment of the present invention, the VLP comprises a genetically modified *(i.e.* variant) version of the PapMV coat protein. In another embodiment, the PapMV coat protein has been genetically modified to delete amino acids from the Nor C-terminus of the protein and/or to include one or more amino acid substitutions. In a further embodiment, the PapMV coat protein has been genetically modified to delete between about 1 and about 10 amino acids from the N- or C-terminus of the protein.

In a specific embodiment, the PapMV coat protein has been genetically modified to remove one of the two methionine codons that occur proximal to the N-terminus of the protein (*i.e.* at positions 1 and 6 of SEQ ID NO:1) and can initiate translation. Removal of one of the translation initiation codons allows a homogeneous population of proteins to be produced. The selected methionine codon can be removed, for example, by substituting one or more of the nucleotides that make up the codon such that the codon codes for an amino acid other than methionine, or becomes a nonsense codon. Alternatively all or part of the codon, or the 5' region of the nucleic acid encoding the protein that includes the selected codon, can be deleted. In a specific embodiment of the present invention, the PapMV coat protein has been genetically modified to delete between 1 and 5 amino acids from the N-terminus of the protein. In a further embodiment, the genetically modified PapMV coat protein has an amino acid sequence substantially identical to SEQ ID NO:3. In a further embodiment, the PapMV coat protein that has been genetically modified to include additional amino acids (for example between about 1 and about 8 amino acids) at the C-terminus that result from the inclusion of one or more specific restriction enzyme sites into the encoding nucleotide sequence. In a specific embodiment, the PapMV coat protein has an amino acid sequence substantially identical to SEQ ID NO:46.

When the recombinant coat protein comprises a variant sequence that contains one or more amino acid substitutions, these can be "conservative" substitutions or "non-conservative" substitutions. A conservative substitution involves the replacement of one amino acid residue by another residue having similar side chain properties. As is known in the art, the twenty naturally occurring amino acids can be grouped according to the physicochemical properties of their side chains. Suitable groupings include alanine, valine, leucine, isoleucine, proline, methionine, phenylalanine and tryptophan (hydrophobic side chains); glycine, serine, threonine, cysteine, tyrosine, asparagine, and glutamine (polar, uncharged side chains); aspartic acid and glutamic acid (acidic side chains) and lysine, arginine and histidine (basic side chains). Another grouping of amino acids is phenylalanine, tryptophan, and tyrosine (aromatic side chains). A conservative substitution involves the substitution of an amino acid with another amino acid from the same group. A non-conservative substitution involves the replacement of one amino acid residue by another residue having different side chain properties, for example, replacement of an acidic residue with a neutral or basic residue, replacement of a neutral residue with an acidic or basic residue, replacement of a hydrophobic residue with a hydrophilic residue, and the like.

In one embodiment of the present invention, the variant sequence comprises one or more non-conservative substitutions. Replacement of one amino acid with another having different properties may improve the properties of the coat protein. For example, as described herein, mutation of residue 128 of the coat protein improves assembly of the protein into VLPs. In one embodiment of the present invention, therefore, the coat protein comprises a mutation at residue 128 of the coat protein in which the glutamic residue at this position is substituted with a neutral residue. In a further embodiment, the glutamic residue at position 128 is substituted with an alanine residue.

Likewise, the nucleic acid sequence encoding the recombinant coat protein need not correspond precisely to the parental reference sequence but may vary by virtue of the degeneracy of the genetic code and/or such that it encodes a variant amino acid sequence as described above. In one embodiment of the present invention, therefore, the nucleic acid sequence encoding a the recombinant coat protein is at least about 70% identical to the reference sequence. In another embodiment, the nucleic acid sequence encoding the recombinant coat protein is at least about 75% identical to the reference sequence. In other embodiments, the nucleic acid sequence encoding the recombinant coat protein is at least about 80%, at least about 85% or at least about 90% identical to the reference sequence. In a specific embodiment, the reference nucleic acid sequence is SEQ ID NO:2.

The PapMV VLP coat protein may optionally be genetically fused to one or more antigens, an affinity peptide or other short peptide sequence to facilitate attachment of one or more antigens, as described in more detail below

### Antigens

In accordance with one embodiment of the present invention, the APS comprises one or more influenza antigens. The antigen may be an immunogen, epitope, mimotope or antigenic determinant derived from the influenza virus and may be, for example, a peptide, a protein, a nucleic acid, or combination thereof derived from the influenza virus, or it may be an inactivated or attenuated version of the virus.

In one embodiment of the invention in which the one or more antigens comprised by the APS are non-conjugated, the antigens can be provided in the form of a known influenza vaccine. Examples of commercially available influenza vaccines include, but are not limited to, Fluviral® (GlaxoSmithKline), FluMist® (MedImmune Vaccines), Fluad^{®} (Chiron), Invivac^{®} (Solvay Pharmaceuticals), Fluarix® (GlaxoSmithKline), and the virosome-based Inflexal V® (Berna Biotech). Other influenza vaccines are known in the art.

In one embodiment of the present invention, the one or more antigens are proteins or peptides derived from the influenza virus. A number of antigenic influenza virus proteins and peptides are known in the art, however, as would be understood by the skilled worker, suitable antigen(s) may also be selected for incorporation into an APS based on testing for their ability to induce an immune response in an animal using standard immunological techniques known in the art. Examples of suitable antigenic proteins include the membrane proteins, haemagglutinin (HA) and neuramidase (NA), the surface exposed region *(i.e.* the N-terminal 24 amino acids, or M2e peptide) or the cytoplasmic region *(i.e.* the C-terminal 54 residues) of the M2 protein, or the conserved internal nucleoprotein (NP) or M1 protein.

As is known in the art, the host range of certain influenza strains may be restricted. In general, influenza A strains are capable of infecting a large number of vertebrates including humans, domestic and farm animals, marine mammals, and various birds. Influenza B, in contrast, is largely restricted to humans and pigs, and influenza C has been observed in humans and seals.

Humans are infected by a variety of influenza A strains, the most common strains being H1N1, H1N2 and H3N2. In pigs, strains H1N1, H1N2 and H3N2 are prevalent, whereas in horses, strains H7N7 and H3N8 are prevalent. Poultry are also affected by a wide variety of strains including H1N7, H2N2, H3N8, H4N2, H4N8, H5N1, H5N2, H5N9, H6N5, H7N2, H7N3, H9N2, H10N7, H11N6, H12N5, H13N6 and H14N5, many of which have also been reported in humans. Strains H5N1, H9N2 and H7N7 are considered to be zoonotic, potential pandemic strains and are capable of affecting a variety of vertebrates. H5N1 has been reported to infect domestic cats and H3N8 has been reported in dogs.

The sequences of the influenza virus HA, NA, M2, NP and M1 protein from various influenza strains are known in the art and are readily accessible from GenBank database maintained by the National Center for Biotechnology Information (NCBI). For example, the amino acid sequence of the NP protein from the influenza A strain A/WSN/33 is provided in Fig. 41B [SEQ ID NO:64]. Suitable antigens for inclusion in the APS can, therefore, be readily selected by the skilled worker based on the knowledge in the art of antigenic regions of the influenza proteins and taking into consideration the animal in which an immune response is to be raised with the final APS.

Various antigenic regions of the above-noted proteins have been identified and are suitable for use in the APS of the present invention. The antigens comprised by the APS of the present invention can be full-length proteins or antigenic fragments thereof. For humans, antigenic fragments of HA, NP and the matrix proteins include, but are not limited to, the haemagglutinin epitopes: HA 91-108, HA 307-319 and HA 306-324 (Rothbard, 1988, Cell 52:515-523), HA 458-467 (Alexander et al., J. Immunol. 1997, 159(10): 4753-61), HA 213-227, HA 241-255, HA 529-543 and HA 533-547 (Gao, W. et al., 2006 J. Virol., 80:1959-1964); the nucleoprotein epitopes: NP 206-229 (Brett, 1991, J. Immunol. 147:984-991), NP335-350 and NP380-393 (Dyer and Middleton, 1993, In: Histocompatibility testing, a practical approach (Ed.: Rickwood, D. and Hames, B. D.) IRL Press, Oxford, p. 292; Gulukota and DeLisi, 1996, Genetic Analysis: Biomolecular Engineering, 13:81), NP 305-313 (DiBrino, 1993, PNAS 90:1508-12); NP 384-394 (Kvist, 1991, Nature 348:446-448); NP 89-101 (Cerundolo, 1991, Proc. R. Soc. Lon. 244:169-7); NP 91-99 (Silver et al, 1993, Nature 360: 367-369); NP 380-388 (Suhrbier, 1993, J. Immunol. 79:171-173); NP 44-52 and NP 265-273 (DiBrino, 1993, *ibid*.); and NP 365-380 (Townsend, 1986, Cell 44:959-968); the matrix protein (M1) epitopes: M1 2-22, M1 2-12, M1 3-11, M1 3-12, M1 41-51, M1 50-59, M1 51-59, M1 134-142, M1 145-155, M1 164-172, M1 164-173 (all described by Nijman, 1993, Eur. J. Immunol. 23:1215-1219); M1 17-31, M1 55-73, M1 57-68 (Carreno, 1992, Mol Immunol 29:1131-1140); M1 27-35, M1 232-240 (DiBrino, 1993, *ibid*.), M1 59-68 and M1 60-68 (Connan et al., Eur. J. Immunol. 1994, 24(3): 777-80); and M1 128-135 (Dong et al., Eur. J. Immunol. 1996, 26(2): 335-39).

In one embodiment of the present invention, the APS comprises a full-length antigenic protein, or a fragment that encompasses several of the above-noted epitopes. In another embodiment, the APS comprises the full-length M1 or NP protein, or a fragment of M1 or NP that comprises a plurality of epitopes. In a further embodiment, the APS comprises a fragment of M1 or NP that comprises a plurality of the epitopes listed above.

Other antigenic regions and epitopes are known. For example, fragments of the ion channel protein (M2) can be employed as antigens for inclusion in the APS of the present invention, including the M2e peptide (the extracellular domain of M2). The sequence of this peptide is highly conserved across different strains of influenza. An example of a M2e peptide sequence is shown in Table 1 as SEQ ID NO:6. Variants of this sequence have been identified in the art and some are also shown in Table 1.

**Table 1: M2e Peptide and Variations Thereof**

| **Region of M2** | **Sequence** | **Viral Strain** | **SEQ ID NO** |
|---|---|---|---|
| 2-24 | SLLTEVETPIRNEWGCRCNDSSD | Human H1N1 *e.g.* A/USRR/90/77 and A/WSN/33 | 6 |
| 2-24 | SLLTEVETPIRNEWGCRCNGSSD | N/A* | 7 |
| 2-24 | SLLTEVETPTKNEWDCRCNDSSD | N/A* | 8 |
| 2-24 | SLLTEVETPTRNGWECKCSDSSD | Equine H3N8 A/equine/Massachussetts/ 213/2003 | 9 |
| 2-24 | SLLTEVETPTRNEWECRCSDSSD | H5N1 A/Vietnam/1196/04 | 10 |
| 1-24 | MSLLTEVETPIRNEWGCRCNDSSD | Human H1N1 e.g. A/USRR/90/77 and A/WSN/33 | 40 |
| 1-24 | HSLLTEVETPTRNEWECRCSDSSD | Avian H5N1 A/Vietnam/1196/04 | 41 |
| 1-24 | MSLLTEVETPTRNGWECKCSDSSD | H3N8, Horse-Dog A/equine/Massachussetts/ 213/2003 | 42 |
| 1-24 | MSLLTEVETPTRNGWGCRCSDSSD | H9N2, A/chicken/Osaka/aq69/2001 | 43 |
| 1-24 | MSLLTEVETPTRNEWC3CRCSDSSD | Mutant H1N1 I/T | 44 |

| | | | |
|---|---|---|---|
| ^{*}see U.S. Patent Application No. 2006/0246092 | | | |

The entire M2e sequence or a partial M2e sequence may be used, for example, a partial sequence that is conserved across the variants, such as fragments within the region defined by amino acids 2 to 10, or the conserved epitope EVETPIRN [SEQ ID NO:11] (amino acids 6-13 of the M2e sequence). The 6-13 epitope has been found to be invariable in 84% of human influenza A strains available in GenBank. Variants of this sequence that were also identified include EVETLTRN [SEQ ID NO:12] (9.6%), EVETPIRS [SEQ ID NO:13] (2.3%), EVETPTRN [SEQ ID NO:14] (1.1%), EVETPTKN [SEQ ID NO:15] (1.1%) and EVDTLTRN [SEQ ID NO:16], EVETPIRK [SEQ ID NO:17] and EVETLTKN [SEQ ID NO:18] (0.6% each) (see Zou, P., et al., 2005, Int Immunopharmacology, 5:631-635; Liu et al. 2005, Microbes and Infection, 7:171-177).

In one embodiment of the present invention, at least one antigen selected for incorporation into the APS is from a conserved protein that is substantially unaffected by genetic drift, such as M1, M2 or NP. In accordance with this embodiment, the resulting APS can provide effective protection against a variety of strains of influenza.

In one embodiment, the APS comprises antigen(s) derived from the M2 protein. In another embodiment, at least one antigen is derived from the M2e peptide. In a further embodiment, the APS comprises at least one antigen that comprises an amino acid sequence substantially identical to the sequence as set forth in any one of SEQ ID NO:6, SEQ ID NO:7, SEQ ID NO:8, SEQ ID NO:9, SEQ ID NO:10, SEQ ID NO:40, SEQ ID NO:41, SEQ ID NO:42, SEQ ID NO:43 or SEQ ID NO:44. In another embodiment, the APS comprises at least one antigen that comprises an amino acid sequence as set forth in SEQ ID NO:11. In a further embodiment, the APS comprises a plurality of antigens, each antigen having a sequence selected from: SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:40, SEQ ID NO:41, SEQ ID NO:42, SEQ ID NO:43 and SEQ ID NO:44.

In another embodiment, the antigens selected are from proteins susceptible to genetic drift, such as the HA and/or NA proteins. In accordance with this embodiment, the PapMV platform allows for rapid adjustment of the selected antigen to account for the emergence of new strains in that the surface glycoprotein antigen(s) can be replaced with different subtypes of HA and/or NA thus permitting the updating of formulations with new antigenic variants of these proteins. As antigenic variants of these glycoproteins are identified, the APS can be updated to include these new variants. Accordingly, even dangerous surface glycoproteins such as H1N1 or a HA/NA combination with pandemic potential could be incorporated into the APS of the present invention as the constituent PapMV or PapMV VLP is not infectious in animals and cannot transmit disease.

The antigen(s) selected can vary in size and the size of antigen selected is not critical to the practice of the present invention. The antigen may be specific or recognised by surface structures on T cells, B cells, NK cells and macrophages, or Class I or Class II APC associated cell surface structures. In one embodiment, the invention is especially useful for small weakly immunogenic antigens.

As noted above, the antigens comprised by the APS can be entire proteins or fragments thereof. Thus, the APS of the present invention may comprise one or more antigens each having a single epitope capable of triggering a specific immune response, or the APS may comprise one or more antigen, wherein each antigen comprises a plurality of epitopes. The antigens comprised by the APS can be the same, or they can be different, and when a plurality of antigens are present, they may be derived from a single protein or from a plurality of proteins. Thus, APSs that comprise antigens representing a combination of influenza antigenic proteins are also contemplated, as are APSs that comprise antigens from the same region of a protein but which represent variations of the protein seen in different viral strains. A non-limiting example of the former embodiment is an APS that comprises a plurality of antigens derived from the M2 and M1, M2 and NP, or M2, M1 and NP proteins. An example of the latter embodiment is an APS that comprises a plurality of antigens each derived from the same region of the HA, NA or M2 protein, but which each represent a different influenza strain.

### Antigen-PapMV and Antigen PapMV - VLP Combinations

As noted above, the one or more antigens comprised by the APS can be conjugated to a coat protein of the PapMV of PaPMV VLP, or they may be present in the APS in a non-conjugated form *(i.e.* simply combined with the PapMV of PapMV VLP). Conjugation can be, for example, by genetic fusion with the coat protein, or binding via covalent, non-covalent or affinity means. Combination of the antigen(s) with the PapMV or VLP, however, should not interfere with the recognition of the antigen by the host's immune system or the ability of the PapMV or VLP to potentiate the immune response.

In accordance one embodiment of the present invention, the one or more antigens are conjugated to a coat protein of a PapMV VLP. As the VLP comprises multiple copies of self-assembled coat protein, attaching the antigen to the coat protein allows presentation of the antigens in an organized fashion on the surface of the VLP. In accordance with this embodiment, the VLP comprises coat proteins that include a first portion that is a recombinant PapMV coat protein conjugated to a second portion that comprises one or more antigens. The antigen-conjugated coat protein retains the ability to assemble with other fusion coat proteins or with wild-type coat protein to form an immunogen-carrier VLP.

In order to allow presentation of the antigen on the surface of the VLP and enhance immune recognition of the antigen, the antigen is preferably attached to a region of the coat protein that is disposed on the outer surface of the VLP. Thus the antigen can be inserted near, or attached at, the amino- (N-) or carboxy- (C-) terminus of the coat protein, or it can be inserted into, or attached to, an internal loop of the coat protein which is disposed on the outer surface of the VLP. In one embodiment of the present invention, the antigen is present at, or proximal to, the C-terminus of the PapMV coat protein.

In accordance with one embodiment of the invention, the APS comprises a PapMV coat protein genetically fused to one or more antigens. The antigen can be directly fused to the coat protein sequence such that the sequences are contiguous, or a "spacer" of one or more amino acids can be inserted between the coat protein sequence and the sequence of the antigen. For example, a spacer comprising one or more neutral amino acids, such as those having aliphatic side chains, can be inserted between the two sequences. Non-limiting examples of neutral amino acids include alanine, glycine, valine, leucine and isoleucine. In one embodiment, the spacer can vary in length between one and about 10 amino acids, for example, between 2 and about 10, between 2 and about 8, between 2 and about 7, or between 2 and about 6 amino acids.

In another embodiment, the antigen(s) are chemically cross-linked to the coat protein, for example, by covalent or non-covalent (such as, ionic, hydrophobic, hydrogen bonding, or the like) attachment. The antigen and/or coat protein can be modified to facilitate such cross-linking as is known in the art, for example, by addition of a functional group or chemical moiety to the protein and/or antigen, for example at the C- or N-terminus or at an internal position. Exemplary modifications include the addition of functional groups such as S-acetylmercaptosuccinic anhydride (SAMSA) or S-acetyl thioacetate (SATA), or addition of one or more cysteine residues. Other cross-linking reagents are known in the art and many are commercially available (see, for example, catalogues from Pierce Chemical Co. and Sigma-Aldrich). Examples include, but are not limited to, diamines, such as 1,6-diaminohexane, 1,3-diamino propane and 1,3-diamino ethane; dialdehydes, such as glutaraldehyde; succinimide esters, such as ethylene glycol-bis(succinic acid N-hydroxysuccinimide ester), disuccinimidyl glutarate, disuccinimidyl suberate, N-(g-Maleimidobutyryloxy) sulfosuccinimide ester and ethylene glycol-bis(succinimidylsuccinate); diisocyantes, such as hexamethylenediisocyanate; bis oxiranes, such as 1,4 butanediyl diglycidyl ether; dicarboxylic acids, such as succinyidisalicylate; 3-maleimidopropionic acid N-hydroxysuccinimide ester, and the like. Many of the above-noted cross-linking agents incorporate a spacer that distances the antigen from the VLP. The use of other spacers is also contemplated by the invention. Various spacers are known in the art and include, but are not limited to, 6-aminohexanoic acid; 1,3-diamino propane; 1,3-diamino ethane; and short amino acid sequences, such as polyglycine sequences, of 1 to 5 amino acids.

To facilitate covalent attachment of the one or more antigen to the coat protein of the VLP, the coat protein can be genetically fused to a short peptide or amino acid linker that is exposed in the surface of the VLP and provides an appropriate site for chemical attachment of the antigen. For example, short peptides comprising cysteine residues, or other amino acid residues having side chains that are capable of forming covalent bonds (for example, acidic and basic residues) or that can be readily modified to form covalent bonds as known in the art. The amino acid linker or peptide can be, for example, between one and about 20 amino acids in length. In one embodiment, the coat protein is fused with a short peptide comprising one or more lysine residues, which can be covalently coupled, for example with a cysteine residue in the antigen through the use of a suitable cross-linking agent as described above. In a specific embodiment, the coat protein is fused with a short peptide sequence of glycine and lysine residues. In another embodiment, the peptide comprises the sequence: GGKGG.

In a further embodiment of the present invention, the antigen is attached via an affinity moiety present on the coat protein. In accordance with this embodiment, the PapMV VLP comprises an affinity moiety, such as a peptide, that is exposed on the surface of the VLP following self-assembly, and which is capable of specifically binding to the antigen. The affinity moiety may be genetically fused (in the case of a peptide or protein fragment), or covalently or non-covalently attached to the PapMV or VLP. Binding of the antigen to the affinity moiety should not interfere with the recognition of the antigen by the host's immune system.

Examples of suitable affinity moieties include, but are not limited to, antibodies and antibody fragments (such as Fab fragments, Fab' fragments, Fab'-SH, fragments F(ab')₂ fragments, Fv fragments, diabodies, and single-chain Fv (scFv) molecules), streptavidin (to bind biotin labelled antigens), affinity peptides or protein fragments that specifically bind the antigen.

Suitable peptides or antibodies (including antibody fragments) for use as affinity moieties can be selected by art-known techniques, such as phage or yeast display techniques. The peptides can be naturally occurring, recombinant, synthetic, or a combination of these. For example, the peptide can be a fragment of a naturally occurring protein or polypeptide. The term peptide also encompass peptide analogues, peptide derivatives and peptidomimetic compounds. Such compounds are well known in the art and may have advantages over naturally occurring peptides, including, for example, greater chemical stability, increased resistance to proteolytic degradation, enhanced pharmacological properties (such as, half-life, absorption, potency and efficacy) and/or reduced antigenicity.

### PREPARATION OF THE APS

The present invention provides APSs that comprise PapMV or PapMV VLPs derived from a recombinant PapMV coat protein. The invention further provides recombinant PapMVs VLPs that comprise one or more antigens, or an affinity moiety, in genetic fusion with the coat proteins. These recombinant coat proteins are capable of multimerisation and assembly into VLPs. Methods of genetically fusing the antigens, or affinity peptides for linking to antigens, to the coat protein are known in the art and some are described below and in the Examples. Methods of chemically cross-linking various molecules to proteins are well known in the art and can be employed.

### Papaya Mosaic Virus

PapMV is known in the art and can be obtained, for example, from the American Type Culture Collection (ATCC) as ATCC No. PV-204™. The virus can be maintained on, and purified from, host plants such as papaya (*Carica papaya*) and snapdragon *(Antirrhinum majus)* following standard protocols (see, for example, Erickson, J. W. & Bancroft, J. B., 1978, Virology 90:36-46).

### PapMV VLPs

The recombinant coat proteins for use to prepare the VLPs of the present invention can be readily prepared by standard genetic engineering techniques by the skilled worker provided with the sequence of the wild-type protein. Methods of genetically engineering proteins are well known in the art (see, for example, Ausubel et al. (1994 & updates) Current Protocols in Molecular Biology, John Wiley & Sons, New York), as is the sequence of the wild-type PapMV coat protein (see SEQ ID NOs:1 and 2).

Isolation and cloning of the nucleic acid sequence encoding the wild-type protein can be achieved using standard techniques (see, for example, *Ausubel et al*., *ibid*.). For example, the nucleic acid sequence can be obtained directly from the PapMV by extracting RNA by standard techniques and then synthesizing cDNA from the RNA template (for example, by RT-PCR). PapMV can be purified from infected plant leaves that show mosaic symptoms by standard techniques (see, for example Example I provided herein).

The nucleic acid sequence encoding the coat protein is then inserted directly or after one or more subcloning steps into a suitable expression vector. One skilled in the art will appreciate that the precise vector used is not critical to the instant invention. Examples of suitable vectors include, but are not limited to, plasmids, phagemids, cosmids, bacteriophage, baculoviruses, retroviruses or DNA viruses. The coat protein can then be expressed and purified as described in more detail below.

Alternatively, the nucleic acid sequence encoding the coat protein can be further engineered to introduce one or more mutations, such as those described above, by standard *in vitro* site-directed mutagenesis techniques well-known in the art. Mutations can be introduced by deletion, insertion, substitution, inversion, or a combination thereof, of one or more of the appropriate nucleotides making up the coding sequence. This can be achieved, for example, by PCR based techniques for which primers are designed that incorporate one or more nucleotide mismatches, insertions or deletions. The presence of the mutation can be verified by a number of standard techniques, for example by restriction analysis or by DNA sequencing.

As noted above, the coat proteins can also be engineered to produce fusion proteins comprising one or more antigens or affinity peptides fused to the coat protein. Methods for making fusion proteins are well known to those skilled in the art. DNA sequences encoding a fusion protein can be inserted into a suitable expression vector as noted above.

One of ordinary skill in the art will appreciate that the DNA encoding the coat protein or fusion protein can be altered in various ways without affecting the activity of the encoded protein. For example, variations in DNA sequence may be used to optimize for codon preference in a host cell used to express the protein, or may contain other sequence changes that facilitate expression.

One skilled in the art will understand that the expression vector may further include regulatory elements, such as transcriptional elements, required for efficient transcription of the DNA sequence encoding the coat or fusion protein. Examples of regulatory elements that can be incorporated into the vector include, but are not limited to, promoters, enhancers, terminators, and polyadenylation signals. The present invention, therefore, provides vectors comprising a regulatory element operatively linked to a nucleic acid sequence encoding a genetically engineered coat protein. One skilled in the art will appreciate that selection of suitable regulatory elements is dependent on the host cell chosen for expression of the genetically engineered coat protein and that such regulatory elements may be derived from a variety of sources, including bacterial, fungal, viral, mammalian or insect genes.

In the context of the present invention, the expression vector may additionally contain heterologous nucleic acid sequences that facilitate the purification of the expressed protein. Examples of such heterologous nucleic acid sequences include, but are not limited to, affinity tags such as metal-affinity tags, histidine tags, avidin / streptavidin encoding sequences, glutathione-S-transferase (GST) encoding sequences and biotin encoding sequences. The amino acids corresponding to expression of the nucleic acids can be removed from the expressed coat protein prior to use according to methods known in the art. Alternatively, the amino acids corresponding to expression of heterologous nucleic acid sequences can be retained on the coat protein if they do not interfere with its subsequent assembly into VLPs.

In one embodiment of the present invention, the coat protein is expressed as a histidine tagged protein. The histidine tag can be located at the carboxyl terminus or the amino terminus of the coat protein.

The expression vector can be introduced into a suitable host cell or tissue by one of a variety of methods known in the art. Such methods can be found generally described in Ausubel *et al.* (*ibid*.) and include, for example, stable or transient transfection, lipofection, electroporation, and infection with recombinant viral vectors. One skilled in the art will understand that selection of the appropriate host cell for expression of the coat protein will be dependent upon the vector chosen. Examples of host cells include, but are not limited to, bacterial, yeast, insect, plant and mammalian cells. The precise host cell used is not critical to the invention. The coat proteins can be produced in a prokaryotic host (e.g., *E. coli, A. salmonicida* or *B. subtilis*) or in a eukaryotic host (e.g., *Saccharomyces* or *Pichia;* mammalian cells, e.g., COS, NIH 3T3, CHO, BHK, 293, or HeLa cells; or insect cells). In one embodiment, the coat proteins are expressed in prokaryotic cells.

If desired, the coat proteins can be purified from the host cells by standard techniques known in the art (see, for example, in Current Protocols in Protein Science, ed. Coligan, J.E., et al., Wiley & Sons, New York, NY) and sequenced by standard peptide sequencing techniques using either the intact protein or proteolytic fragments thereof to confirm the identity of the protein.

The recombinant coat proteins of the present invention are capable of multimerisation and assembly into VLPs. In general, assembly takes place in the host cell expressing the coat protein. The VLPs can be isolated from the host cells by standard techniques, such as those described in the Examples section provided herein. In general, the isolate obtained from the host cells contains a mixture of VLPs, discs, less organised forms of the coat protein (for example, monomers and dimers). The VLPs can be separated from the other coat protein components by, for example, ultracentrifugation or gel filtration chromatography (for example, using Superdex G-200) to provide a substantially pure VLP preparation. In this context, by "substantially pure" it is meant that the preparation contains 70% or greater of VLPs. Alternatively, a mixture of the various forms of coat protein can be used in the final vaccine compositions. When such a mixture us employed, the VLP content should be 40% or greater. In one embodiment, preparations containing 50% or more of VLPs are used in the final vaccine compositions. In another embodiment, preparations containing 60% or more of VLPs are used in the final vaccine compositions. In a further embodiment, preparations containing 70% or more of VLPs are used in the final vaccine compositions. In another embodiment, preparations containing 80% or more of VLPs are used in the final vaccine compositions.

The VLPs can be further purified by standard techniques, such as chromatography, to remove contaminating host cell proteins or other compounds, such as LPS. In one embodiment of the present invention, the VLPs are purified to remove LPS.

In one embodiment of the present invention, the coat proteins assemble to provide a recombinant virus in the host cell and can be used to produce infective virus particles which comprise nucleic acid and fusion protein. This can enable the infection of adjacent cells by the infective virus particle and expression of the fusion protein therein. In this embodiment, the host cell used to replicate the virus can be a plant cell, insect cell, mammalian cell or bacterial cell that will allow the virus to replicate. The cell may be a natural host cell for the virus from which the virus-like particle is derived, but this is not necessary. The host cell can be infected initially with virus in particle form (*i.e.* in assembled rods comprising nucleic acid and a protein) or alternatively in nucleic acid form *(i.e.* RNA such as viral RNA; cDNA or run-off transcripts prepared from cDNA) provided that the virus nucleic acid used for initial infection can replicate and cause production of whole virus particles having the chimeric protein.

### Characteristics of Recombinant Coat Proteins

The recombinant coat proteins can be analysed for their ability to multimerize and self-assemble into a VLP by standard techniques. For example, by visualising the purified recombinant protein by electron microscopy (see, for example, Example I). VLP formation may also be determined by ultracentrifugation, and circular dichroism (CD) spectrophotometry may be used to compare the secondary structure of the recombinant proteins with the WT virus (see, for example, Example I).

Stability of the VLPs, and PapMV, can be determined if desired by techniques known in the art, for example, by SDS-PAGE and proteinase K degradation analyses (see Example II). According to one embodiment of the present invention, the PapMV and PapMV VLPs of the invention are stable at elevated temperatures and can be stored easily at room temperature.

### EVALUATION OF EFFICACY

In order to evaluate the efficacy of the APSs of the present invention as vaccines, challenge studies can be conducted. Such studies involve the inoculation of groups of test animals (such as mice) with an APS of the present invention by standard techniques. Control groups comprising non-inoculated animals and/or animals inoculated with a commercially available vaccine, or other positive control, are set up in parallel. After an appropriate period of time post-vaccination, the animals are challenged with an influenza virus. Blood samples collected from the animals pre- and post-inoculation, as well as post-challenge are then analyzed for an antibody response to the virus. Suitable tests for the antibody response include, but are not limited to, Western blot analysis and Enzyme-Linked Immunosorbent Assay (ELISA). The animals can also be monitored for development of other conditions associated with infection with influenza virus including, for example, body temperature, weight, and the like. For certain strains of influenza, survival is also a suitable marker.

Cellular immune response can also be assessed by techniques known in the art, including those described in the Examples presented herein. For example, through processing and cross-presentation of an epitope expressed on a PapMV VLP to specific T lymphocytes by dendritic cells *in vitro* and *in vivo.* Other useful techniques for assessing induction of cellular immunity (T lymphocyte) include monitoring T cell expansion and IFN-γ secretion release, for example, by ELISA to monitor induction of cytokines (see Example III).

The extent of infection can also be assessed by measurement of lung viral titer using standard techniques after sacrifice of the animal.

### Production of stock PapMV or VLP

Stocks of recombinant PapMV or VLP can be prepared by standard techniques. For example, PapMV or a recombinant virus can be propagated in an appropriate host, such as *Carica papaya* or *Antirrhinum majus,* such that sufficient PapMV or recombinant virus can be harvested.

Stocks of PapMV VLPs can be prepared from an appropriate host cell, such as *E. coli* transformed or transfected with an expression vector encoding the recombinant coat protein that makes up the VLP. The host cells are then cultured under conditions that favour the expression of the encoded protein, as is known in the art. The expressed coat protein will multimerise and assemble into VLPs in the host cell and can be isolated from the cells by standard techniques, for example, by rupturing the cells and submitting the cell lysate to one or more chromatographic purification step.

As demonstrated in the Examples provided herein, the PapMV VLPs are stable structures and stocks of the VLPs can, therefore, be stored easily at room temperature or in a refrigerator.

### VACCINE COMPOSITIONS

The present invention provides for compositions suitable for use as influenza vaccines comprising one or more APS of the invention together with one or more non-toxic pharmaceutically acceptable carriers, diluents and/or excipients. If desired, other active ingredients, adjuvants and/or immunopotentiators may be included in the compositions.

The compositions can be formulated for administration by a variety of routes. For example, the compositions can be formulated for oral, topical, rectal, nasal or parenteral administration or for administration by inhalation or spray. The term parenteral as used herein includes subcutaneous injections, intravenous, intramuscular, intrathecal, intrasternal injection or infusion techniques. Intranasal administration to the subject includes administering the pharmaceutical composition to the mucous membranes of the nasal passage or nasal cavity of the subject. In one embodiment of the present invention, the compositions are formulated for topical, rectal or parenteral administration or for administration by inhalation or spray, for example by an intranasal route. In another embodiment, the compositions are formulated for parenteral administration.

The compositions preferably comprise an effective amount of one or more APS of the invention. The term "effective amount" as used herein refers to an amount of the APS required to induce a detectable immune response. The effective amount of APS for a given indication can be estimated initially, for example, either in cell culture assays or in animal models, usually in rodents, rabbits, dogs, pigs or primates. The animal model may also be used to determine the appropriate concentration range and route of administration. Such information can then be used to determine useful doses and routes for administration in the animal to be treated, including humans. In one embodiment of the present invention, the unit dose comprises between about 10µg to about 10mg of protein. In another embodiment, the unit dose comprises between about 10µg to about 5mg of protein. In a further embodiment, the unit dose comprises between about 40µg to about 2 mg of protein. One or more doses may be used to immunise the animal, and these may be administered on the same day or over the course of several days or weeks. In one embodiment of the invention, two or more doses of the composition are administered to the animal to be treated. In another embodiment, three or more doses of the composition are administered to the animal to be treated.

As noted above, the APS of the present invention may comprise a plurality of antigens, and a single APS can thus provide a multivalent vaccine formulation. Multivalent vaccine formulations include bivalent and trivalent formulations in addition to vaccines having higher valencies. Multivalent vaccine compositions that comprise a plurality of APSs, each APS combined with a different antigen are also contemplated. Multivalent vaccines are useful, for example, to provide protection against more than one strain of influenza. One embodiment of the present invention provides a multivalent vaccine. Another embodiment of the invention provides a multivalent vaccine that comprises a plurality of APSs *(i.e.* two or more), each APS comprising a different antigen.

In a specific embodiment of the present invention, there is provided a multivalent vaccine comprising an APS that includes an antigen from the M2 protein and an APS that includes an antigen from a different influenza protein. In another embodiment of the present invention, there is provided a multivalent vaccine comprising an APS that includes a first antigen from the M2 protein and an APS that includes a second antigen from the M2 protein.

Compositions for oral use can be formulated, for example, as tablets, troches, lozenges, aqueous or oily suspensions, dispersible powders or granules, emulsion hard or soft capsules, or syrups or elixirs. Such compositions can be prepared according to standard methods known to the art for the manufacture of pharmaceutical compositions and may contain one or more agents selected from the group of sweetening agents, flavouring agents, colouring agents and preserving agents in order to provide pharmaceutically elegant and palatable preparations. Tablets contain the APS in admixture with suitable non-toxic pharmaceutically acceptable excipients including, for example, inert diluents, such as calcium carbonate, sodium carbonate, lactose, calcium phosphate or sodium phosphate; granulating and disintegrating agents, such as corn starch, or alginic acid; binding agents, such as starch, gelatine or acacia, and lubricating agents, such as magnesium stearate, stearic acid or talc. The tablets can be uncoated, or they may be coated by known techniques in order to delay disintegration and absorption in the gastrointestinal tract and thereby provide a sustained action over a longer period. For example, a time delay material such as glyceryl monosterate or glyceryl distearate may be employed.

Compositions for oral use can also be presented as hard gelatine capsules wherein the APS is mixed with an inert solid diluent, for example, calcium carbonate, calcium phosphate or kaolin, or as soft gelatine capsules wherein the active ingredient is mixed with water or an oil medium such as peanut oil, liquid paraffin or olive oil.

Compositions for nasal administration can include, for example, nasal spray, nasal drops, suspensions, solutions, gels, ointments, creams, and powders. The compositions can be formulated for administration through a suitable commercially available nasal spray device, such as Accuspra™ (Becton Dickinson). Other methods of nasal administration are known in the art.

Compositions formulated as aqueous suspensions contain the APS in admixture with one or more suitable excipients, for example, with suspending agents, such as sodium carboxymethylcellulose, methyl cellulose, hydropropylmethylcellulose, sodium alginate, polyvinylpyrrolidone, hydroxypropyl-β-cyclodextrin, gum tragacanth and gum acacia; dispersing or wetting agents such as a naturally-occurring phosphatide, for example, lecithin, or condensation products of an alkylene oxide with fatty acids, for example, polyoxyethyene stearate, or condensation products of ethylene oxide with long chain aliphatic alcohols, for example, hepta-decaethyleneoxycetanol, or condensation products of ethylene oxide with partial esters derived from fatty acids and a hexitol for example, polyoxyethylene sorbitol monooleate, or condensation products of ethylene oxide with partial esters derived from fatty acids and hexitol anhydrides, for example, polyethylene sorbitan monooleate. The aqueous suspensions may also contain one or more preservatives, for example ethyl, or *n*-propyl p-hydroxy-benzoate, one or more colouring agents, one or more flavouring agents or one or more sweetening agents, such as sucrose or saccharin.

Compositions can be formulated as oily suspensions by suspending the APS in a vegetable oil, for example, arachis oil, olive oil, sesame oil or coconut oil, or in a mineral oil such as liquid paraffin. The oily suspensions may contain a thickening agent, for example, beeswax, hard paraffin or cetyl alcohol. Sweetening agents such as those set forth above, and/or flavouring agents may optionally be added to provide palatable oral preparations. These compositions can be preserved by the addition of an anti-oxidant such as ascorbic acid.

The compositions can be formulated as a dispersible powder or granules, which can subsequently be used to prepare an aqueous suspension by the addition of water. Such dispersible powders or granules provide the APS in admixture with one or more dispersing or wetting agents, suspending agents and/or preservatives. Suitable dispersing or wetting agents and suspending agents are exemplified by those already mentioned above. Additional excipients, for example, sweetening, flavouring and colouring agents, can also be included in these compositions.

Compositions of the invention can also be formulated as oil-in-water emulsions. The oil phase can be a vegetable oil, for example, olive oil or arachis oil, or a mineral oil, for example, liquid paraffin, or it may be a mixture of these oils. Suitable emulsifying agents for inclusion in these compositions include naturally-occurring gums, for example, gum acacia or gum tragacanth; naturally-occurring phosphatides, for example, soy bean, lecithin; or esters or partial esters derived from fatty acids and hexitol, anhydrides, for example, sorbitan monoleate, and condensation products of the said partial esters with ethylene oxide, for example, polyoxyethylene sorbitan monoleate. The emulsions can also optionally contain sweetening and flavouring agents.

Compositions can be formulated as a syrup or elixir by combining the APS with one or more sweetening agents, for example glycerol, propylene glycol, sorbitol or sucrose. Such formulations can also optionally contain one or more demulcents, preservatives, flavouring agents and/or colouring agents.

The compositions can be formulated as a sterile injectable aqueous or oleaginous suspension according to methods known in the art and using suitable one or more dispersing or wetting agents and/or suspending agents, such as those mentioned above. The sterile injectable preparation can be a sterile injectable solution or suspension in a non-toxic parentally acceptable diluent or solvent, for example, as a solution in 1,3-butanediol. Acceptable vehicles and solvents that can be employed include, but are not limited to, water, Ringer's solution, lactated Ringer's solution and isotonic sodium chloride solution. Other examples include, sterile, fixed oils, which are conventionally employed as a solvent or suspending medium, and a variety of bland fixed oils including, for example, synthetic mono- or diglycerides. Fatty acids such as oleic acid can also be used in the preparation of injectables.

Optionally the composition of the present invention may contain preservatives such as antimicrobial agents, anti-oxidants, chelating agents, and inert gases, and/or stabilizers such as a carbohydrate (*e.g.* sorbitol, mannitol, starch, sucrose, glucose, or dextran), a protein (*e.g.* albumin or casein), or a protein-containing agent (*e.g.* bovine serum or skimmed milk) together with a suitable buffer (*e.g.* phosphate bluffer). The pH and exact concentration of the various components of the composition may be adjusted according to well-known parameters.

Further, one or more compounds having adjuvant activity may be optionally added to the vaccine composition. Suitable adjuvants include, for example, alum adjuvants (such as aluminium hydroxide, phosphate or oxide); oil-emulsions (*e.g*. of Bayol F® or Marco152®); saponins, or vitamin-E solubilisate. Virosomes are also known to have adjuvant properties (Adjuvant and Antigen Delivery Properties of Virosomes, Glück, R., et al., 2005, Current Drug Delivery, 2:395-400) and can be used in conjunction with an APS of the invention.

As previously noted and demonstrated herein, PapMV and PapMV VLPs have adjuvant properties. Accordingly, in one embodiment of the invention, the vaccine compositions comprise additional PapMV or PapMV VLPs as an adjuvant. In some embodiments, use of PapMV or PapMV VLPs may provide advantages over commercially available adjuvants in that it has been observed that PapMV or PapMV VLPs do not cause obvious local toxicity when administered by injection (see, for example, Example XI).

Opsonised vaccine compositions are also encompassed by the present invention, for example, vaccine compositions comprising antibodies isolated from animals or humans previously immunised with the vaccine, antigen or PapMV VLPs. Recombinant antibodies based on antibodies isolated from animals or humans previously immunised with the vaccine, antigen or PapMV VLPs could also be used to opsonise the vaccine composition.

Also encompassed by the present invention are vaccine compositions comprising an APS of the present invention in combination with a commercially available influenza vaccine.

Other pharmaceutical compositions and methods of preparing pharmaceutical compositions are known in the art and are described, for example, in *"*Remington: The Science and Practice of Pharmacy" (formerly "Remingtons Pharmaceutical Sciences"); Gennaro, A., Lippincott, Williams & Wilkins, Philadelphia, PA (2000).

### APPLICATIONS & USES

The present invention provides for a number of applications and uses of the APSs described herein. Non-limiting examples include the use of the APS as a vaccine against influenza and/or to potentiate an immune response against an influenza antigen, and the use of the APS to screen for antibodies to an influenza virus. The present invention thus also provides methods for potentiating and/or inducing an immune response to an influenza antigen in an animal. As well, the use of PapMV, VLPs and APSs of the invention for the preparation of medicaments, including vaccines, and/or pharmaceutical compositions is within the scope of the present invention.

The APS of the present invention can be used to induce an immune response to one or more than one strain of influenza virus, depending on the antigens selected for inclusion in the APS. The APS is suitable for use in humans as well as non-human animals, including domestic and farm animals. The administration regime for the APS need not differ from any other generally accepted vaccination programs. A single administration of the APS in an amount sufficient to elicit an effective immune response may be used or, alternatively, other regimes of initial administration of the APS followed by boosting, once or more than once, with antigen alone or with the APS may be used. Similarly, boosting with either the APS or antigen may occur at times that take place well after the initial administration if antibody titers fall below acceptable levels. In one embodiment of the invention, the administration regime for the APS comprises an initial dose of the APS plus a booster dose of the APS. In another embodiment, the administration regime for the APS comprises an initial dose of the APS plus two or more booster doses of the APS. In a further embodiment, the administration regime for the APS comprises an initial dose of the APS plus three or more booster doses of the APS. Appropriate dosing regimens can be readily determined by the skilled practitioner.

When the APS comprises non-conjugated antigen(s), the PapMV or VLP component of the APS can be administered concomitantly with the antigen(s), or it can be administered prior or subsequent to the administration of the antigen, depending on the needs of the human or non-human animal in which an immune response is desired.

One embodiment of the invention provides for the use of a vaccine comprising an APS in which the one or more antigens are in the form of a conventional influenza vaccine. Another embodiment provides for the use of a vaccine comprising the APS in conjunction with conventional influenza vaccines. In accordance with this embodiment, the APS vaccine may be administered concomitantly with the conventional vaccine (for example, by combining the two compositions), it can be administered prior or subsequent to the administration of the conventional vaccine.

One embodiment of the present invention provides for the use of the APS as an influenza vaccine for humans. Another embodiment of the present invention provides for the use of an APS comprising one or more antigens from the H1N1 and/or H3N2 strains of influenza as an influenza vaccine for humans. In a specific embodiment of the present invention, there is provided an APS for use as a human influenza vaccine that comprises at least one M2 antigen. In another embodiment, there is provided an APS comprising at least one M2 antigen that comprises an amino acid sequence substantially identical to SEQ ID NO:6, 7, 8, 9, 10, 11, 12, 13, 14, 15, 16, 17, 18, 40 or 44, or a fragment thereof, for use as a human influenza vaccine. The vaccine can optionally include one or more other APSs that comprise antigens different from those in the first APS, for example, antigens comprising a sequence as set forth in any one of SEQ ID NOs:6, 7, 8, 9, 10, 11, 12, 13, 14, 15, 16, 17, 18, 40 or 44, or antigens from other influenza virus proteins, such as NP or M1.

An alternative embodiment of the present invention provides for the use of the APS as an influenza vaccine for non-humans. Another embodiment provides for the use of an APS comprising one or more antigens from the H3N8, H7N7, H9N2 and/or H5N1 strains of influenza as an influenza vaccine for non-humans. A further embodiment provides for the use of the APS as an influenza vaccine for non-human mammals. Another embodiment provides for the use of the APS as an influenza vaccine for birds. In a further embodiment of the present invention, there is provided an APS for use as an influenza vaccine for mammals and/or birds that comprises at least one antigen comprising an amino acid sequence as set forth in any one of SEQ ID NO:9, 41, 42 or 43, or a fragment thereof. In a specific embodiment of the present invention, there is provided an APS suitable for use as an influenza vaccine for dogs, cats, horses, swine, chickens, geese and crested eagles that comprises at least one antigen comprising an amino acid sequence as set forth in SEQ ID NO:9, or a fragment thereof. The vaccine can optionally include one or more other APSs that comprise antigens derived from variants of the M2e peptide found in other strains of influenza, such as those comprising a sequence as set forth in any one of SEQ ID NOs: 6, 7, 8, 10, 11, 12, 13, 14, 15, 16, 17, 18, 40 or 44. In another embodiment of the present invention, there is provided a vaccine for dogs and/or horses comprising a first APS comprising an antigen including an amino acid sequence as set forth in SEQ ID NO:9, or a fragment thereof, and a second APS comprising an antigen including an amino acid sequence as set forth in SEQ ID NO:10, or a fragment thereof. In a further embodiment, there is provided an APS suitable for use as an influenza vaccine for birds that comprises at least one antigen comprising an amino acid sequence as set forth in SEQ ID NO:41 or 43, or a fragment thereof.

As demonstrated herein, PapMV VLPs are capable of potentiating both a humoral and a CTL response to an antigen. Accordingly, in one embodiment of the present invention, there is provided a vaccine comprising a first APS that includes an influenza antigen that produces a humoral response (such as an antigen from the M2 protein) and a second APS that includes an influenza antigen that produces a CTL response (such as an antigen from the NP or M1 protein).

The present invention also provides for the use of the APS as a screening agent, for example, to screen for antibodies to influenza. The APS can be readily adapted to conventional immunological techniques such as an enzyme-linked immunosorbant assay (ELISA) or Western blotting and is thus useful in diagnostic and research contexts.

### KITS

The present invention additionally provides for kits comprising one or more APS for use as an influenza vaccine. Individual components of the kit would be packaged in separate containers and, associated with such containers, can be a notice in the form prescribed by a governmental agency regulating the manufacture, use or sale of pharmaceuticals or biological products, which notice reflects approval by the agency of manufacture, use or sale. The kit may optionally contain instructions or directions outlining the method of use or administration regimen for the vaccine.

When one or more components of the kit are provided as solutions, for example an aqueous solution, or a sterile aqueous solution, the container means may itself be an inhalant, syringe, pipette, eye dropper, or other such like apparatus, from which the solution may be administered to a subject or applied to and mixed with the other components of the kit.

The components of the kit may also be provided in dried or lyophilised form and the kit can additionally contain a suitable solvent for reconstitution of the lyophilised components. Irrespective of the number or type of containers, the kits of the invention also may comprise an instrument for assisting with the administration of the composition to a patient. Such an instrument may be an inhalant, nasal spray device, syringe, pipette, forceps, measured spoon, eye dropper or similar medically approved delivery vehicle.

Screening kits containing one or more APS of the invention for use in antibody detection are also provided. The kits can be diagnostic kits or kits intended for research purposes. Individual components of the kit would be packaged in separate containers and, associated with such containers, can be a notice in the form prescribed by a governmental agency regulating the manufacture, use or sale of biological products, which notice reflects approval by the agency of manufacture, use or sale of the biological product. The kit may optionally contain instructions or directions outlining the method of use or administration regimen for the vaccine.

To gain a better understanding of the invention described herein, the following examples are set forth. It will be understood that these examples are intended to describe illustrative embodiments of the invention and are not intended to limit the scope of the invention in any way.

### EXAMPLES

### EXAMPLE I: Effect of mutations on RNA binding and self-assembly of PapMV coat protein

PapMV coat protein (CPΔN5; shown in SEQ ID NO:3) harbouring the insertion of an alanine at position 2 and in which 5 amino acids at the N-terminus were deleted from the WT sequence was used to create a series of mutants as outlined in Tremblay, M-H., et al., 2006, FEBS J., 273:14-25. Based on the alignment of the amino acids sequences of 19 potexviruses CPs between amino acid 90 to 169 of PapMV CP which revealed that the amino acid corresponding to position 128 of the PapMV CP is an A in most potexviruses but is an E in PapMV, and the report that the charged residues R104, K133, K137, and R161 are likely to be involved in an interaction with the genomic RNA and play an important role in assembly and packaging of the viral genome (Abouhaidar, & Lai, 1989, J. Gen. Virol. 70, 1871-5), the following mutations were made: E128A; K97A; R104K105R108/A; R118D120K121/A; K133K137/A; D142D145/A; E148A; R161A or E166E168/A.

### Electron microscopy and immunogold labeling

VLPs or viruses were diluted in 10 mM Tris-HCl pH 8 and were absorbed for 3 minutes on a carbon-coated formvar grid. Grids were blocked with 8 mL of BSA (10 mg/mL) for 30 seconds and washed with PBS. Grids were incubated for 30 minutes at room temperature with a rabbit anti-6XH tag antibody (Amersham, Pittsburgh, PA, USA) diluted 1:10 in PBS. Grids were then washed three times with PBS and incubated at room temperature for 30 minutes with donkey anti-rabbit antibodies conjugated with 6 nm gold particles (Jackson Immuno Research, West Baltimore Pike, West Grove, PA, USA) and diluted 1:20 in PBS. Grids were then washed with deionized water and stained as described above.

### Circular Dichroism spectroscopy

CD spectra were recorded on an Olis RSM 1000 (Olis, Conway DriveSuites A & B, Bogart, GA, USA) rapid scanner monochromator at 20°C. For far UV CD (260-190 nm), thermostated quartz cells of 0.1 cm path length were used. Mean residue ellipticity values ([6]mR_{w} in deg x cmz x dmol) were calculated using the equation: [6]MR_{w} = [6]*MRW/(10 x c x 1), where [6] is the ellipticity in degrees, MRW is the average molecular weight of the residues in the protein (108 was used in this study), c is the protein concentration in g/ml and 1 is the path length in cm (Johnson, W. C. (1996) Circular Dichroism Instrumentation in Circular Dichroism and the Conformational Analysis of Biomolecules (Fasman, G. D., ed) pp. 635-652, Kluwer Academic / Plenum Publishers).

Near UV CD spectra were recorded (250-350 nm) at RT in a Jasco Model J-710 instrument (Jasco, Commerce Dr. Easton, MD, USA). Recordings were made using a quartz cuvettes (pathlength 0.1 cm). Spectra were averaged from 10 scans of 0.2 nm steps at a rate of 100 nm/min. Rod and disc samples were respectively at concentration of 1.5 mg/ml and 5.5 mg/ml.

### RNA transcripts and electrophoretic mobility shift assay (EMSA)

The RNA probe was generated by transcription *in vitro* using a RiboMAX™ Large Scale RNA Production System-T7 kit (Promega P1300, Madison, WI, USA) and a clone of 80 nt of the 5'end of PapMV in front of the T7 promoter. The clone was linearised with EcoRI before *in vitro* transcription. The RNA transcript was purified on a G50 Quick Spin Column for DNA/RNA purification (Roche 1273 965). The same method was used to generate a transcript of the 5' 1800 nucleotides of PapMV for the *in vitro* assembly assay. The RNA probe was dephosphorylated using shrimp alkaline phosphatase (Fermentas, Hanover, MD, USA, EFO511) and labelled with gamma ³²P-ATP using T4 polynucleotide kinase (NEB, Ipswich, MA, USA, M0201 S). The probe was then purified using the G-50 Quick Spin Columns as before. Labelled RNA was incubated with recombinant proteins at room temperature for 60 minutes. 165 fmol of RNA were used for each reaction and various amounts of purified recombinant proteins in the *in vitro* assembly buffer, which contained 7.5 U of RNase inhibitor (Amersham Biosciences 27-081601). The final volume of the reaction was 10 µL; 2 µL of loading dye was added to the sample before loading onto a 5% native polyacrylamide gel. Electrophoresis was performed in 0.5X Tris-borate-EDTA buffer for 90 minutes at 10mA. The gel was dried and subjected to autoradiography for 16 hours on Kodak BioMax MS film (Amersham Biosciences V8326886) and developed.

### Purification of PapMV and isolation of discs

PapMV was purified by differential centrifugation from infected papaya leaves that showed mosaic symptoms. Infected leaves (100 g) were ground in 100 mL 50 mM Tris-HCl (pH 8.0) containing 10 mM EDTA in a commercial blender. The ground leaves were filtered through cheesecloth, 1% of Triton X-100 was added to the filtrate, and the filtrate was stirred gently for 10 min. Chloroform was added drop by drop to a volume equivalent to one-quarter of the volume of the filtrate. The solution was stirred for an additional 30 min at 4 °C and centrifuged for 20 min at 10 000 g to remove the precipitate. The supernatant was subjected to high-speed (100 000 g) centrifugation for 120 min. The viral pellet was suspended and subjected to another high-speed centrifugation through a sucrose cushion (30% sucrose) at 100 000 g for 3.5 h. The final viral pellet was suspended in 10 mL of 50 mM Tris (pH 8.0). If color persisted, an additional clarification with chloroform was performed. The purified virus was collected by ultracentrifugation. The isolation of the discs by acetic acid degradation method was performed as described previously (Erickson, et al.,1976, Virology. 72, 514-7).

### Trypsin digest

10 µg of protein was incubated at 37°C in a volume of 50 µl for 120 minutes in a 100mM tris HCl buffer pH 8.5 with 0,2 µg of trypsin (Roche, Indianapolis, IN, USA, 1418475). The reaction was stopped by addition 10 µL of loading dye containing 5% SDS, 5 mM DTT and 40% glycerol. The sample was boiled 5 minutes prior loading on a SDS-PAGE gels. The proteins were visualised by Coomassie blue staining.

### Results

The PapMV CP harbours two M residues at positions 1 and 6 of the CP open reading frame (ORF). It is not clear if both of these initiation codons are used during replication of the virus. However, it has been shown that a large proportion of the CP of the purified virus lacks several amino acids at the N-terminus (Zhang, et al., 1993, J. Mol. Biol. 234, 885-7). To ensure production of only one open reading frame in *E.coli,* the N-terminal 5 amino acids were removed such that M⁶ served as an initiation codon. The introduction of the initiation codon in the NcoI site introduced an extra A that is found in all the constructs. A 6XH tag was added at the C-terminus of the protein to facilitate the purification process. The recombinant protein CPΔN5 was expressed in *E. coli* BL21 (pLysS) and showed a slightly larger molecular weight (MW) than that of WT CP extracted from purified virus. The difference observed between the two proteins is probably caused by the 6XH tag fusion at the C-terminus. The recombinant protein was affinity purified using a Ni²⁺ column and eluted using 1 M imidazole. The yield of the purified recombinant protein was estimated at 40-50 mg/L. Western blot assay using an antibody raised against the WT PapMP CP confirmed that the purified protein was indeed PapMV CP.

Nine different mutants were generated that harbour one, two or three A substitutions. Five mutants R118-D120-K121/A, K133-K137/A, D142-D145/A, R161A and E166-E167-R168/A produced unstable proteins and were undetectable or expressed at very low level. It is likely that mutagenesis in this conserved region affected the native folding of the CP. The mutants K97A, R104K105R108/A, E128A and E148A could be expressed to level similar to CPΔN5 and easily purified using a 6xH tag as shown with the CPΔN5. However, the removal of imidazole during the dialysis made the mutants R104K105R108/A and E148A aggregate and precipitate and it is likely that the mutations affected the folding of the protein.

The construct CPΔN5 self assembled into VLPs in *E. coli* as shown by the electron micrograph of the purified recombinant protein. The VLPs were similar in shape and in diameter to the native virus particles. To analyse and quantify the proportion of the purified protein that was found as VLPs, VLPs and smaller aggregates were separated by ultracentrifugation at 100,000 g for 2 hours. Most of the CPΔN5 proteins (80%) were found in the supernatant. VLPs were found in the pellet and account for 20% of the total purified recombinant protein. However, the purified protein K97A remained in the supernatant after ultracentrifugation. On the contrary, the recombinant protein E128A was found totally in the pellet after ultracentrifugation.

Electron microscopy revealed that E128A VLPs isolated from the high speed pellet are similar to the WT virus. The length of 150 VLPs for each CPΔN5 and E128A was measured and the average length was determined. CPΔN5 VLPs appeared 10 times shorter (50 nm) than the native virus that is 500 nm in length as predicted. However, E128A VLPs are approximately 3 times longer than CPΔN5 VLPs suggesting that this mutant can more efficiently support the initiation and elongation of assembly. Finally, an electron micrograph of the purified K97A protein revealed disorganised aggregates of 15 to 50 nm in diameter. The outline of the aggregates was irregular showing that this protein can not organise itself into VLPs.

The purification of the VLPs using the Ni²+ column was efficient suggesting that the 6XH tag is located at the surface and available for interaction with the affinity column. To confirm this hypothesis, an immunogold labelling experiment was performed on CPΔN5 VLPs using anti-6XHis tag rabbit antiserum followed by a secondary donkey anti-rabbit labelled with gold particles. As expected, the VLPs were decorated with the gold particles, thus demonstrating that the fusion of a peptide (6XH) to the C-terminus is tolerated and exposed to the surface of VLPs. This surface exposure of C-terminally fused peptides demonstrates the suitability of the C-terminus as an appropriate point to which to attach an immunogen.

Circular dichroism (CD) spectrophotometry was used to compare the secondary structure of the recombinant proteins with the WT virus. The secondary structure of CPΔN5 was estimated to be 49% α-helices and 15% random coil. The CD spectra of CPΔN5 VLPs and WT virus showed a slightly different profile (Fig. 2A). The CD signal measured at 208 nm was more pronounced for the WT virus than the CPΔN5 VLPs (Fig. 2A). Interestingly, the CD signal measured with E128A VLPs at 208 nm superimposed with the WT virus (Fig. 2A).

The CD signal of the isolated discs (high speed supernatant of the purified protein) of CPΔN5 was identical to isolated discs from the purified virus using the acetic acid method (Fig. 2B). It is interesting to notice that the CD signal measured with discs in general at 208 nm was less pronounced than with VLPs. This result suggests that the content in α-helices is increased when the discs assemble in VLPs. Finally, the folding of the purified protein of K97A was compared with high speed supernatant of CPΔN5. Both proteins showed an identical CD profile (Fig. 2E).

Spectra between 250 and 350 nm were also obtained to measure the absorption of aromatic residues and tryptophan residues in the protein. A change in the environment of those residues affects the signal recorded and indicates variation in the tertiary structure. The VLPs of CPΔN5 and E128A appeared to be similar (Fig. 2D), indicating that the tertiary structures are the same for both VLPs. The spectra of CPΔN5 discs and K97A were very similar which suggests that both proteins have a similar tertiary structure (Fig. 2E). The slight differences in the intensity of the curves between the samples were probably due to a small variation in protein concentrations.

80% of the purified recombinant CPΔN5 and all the K97A proteins were found as multimers (discs) in the supernatant after ultracentrifugation. To measure the level of multimerisation of these proteins, the high speed supernatant of CPΔN5 and the purified protein K97A were analysed using Superdex™ 200. For CPΔN5, most of the proteins were eluted as a high molecular weight complex of 450 kDa (Fig. 3A) which corresponds to a multimer of approximately 20 subunits (molecular weight of the protein subunit is 23 kDa). The second peak eluting at 81.27 ml was collected and loaded on a Superdex™ 75 column to improve the resolution. The protein eluted as a 39 kDa protein. A sample from this peak was submitted to SDS-PAGE and showed a unique band; smaller than CPΔN5 that corresponds to a degradation product of CPΔN5. It is possible that this degraded protein is unable to form a high molecular complex and remains as a dimer in solution.

The elution profile of K97A can be divided into 3 major peaks (Fig. 3B). The second peak was eluted at 50 ml and overlapped with the CPΔN5 discs (Fig. 3A) which probably corresponds to the disc structure. The first peak eluted between 41 and 43 ml and corresponds to aggregated material that is greater than 700 kDa in size. The elution pattern is wide and shows a shoulder that suggests that this material is not uniform and may correspond to an aggregate of K97A discs agglutinated together by non-specific interactions. The third peak was not analysed further and probably corresponds to a truncated protein as shown for the CPΔN5 construct. These results confirm that K97A is able to form discs with other protein subunits but is unable to assemble into VLPs in *E. coli.*

CPΔN5 discs were isolated from the high-speed supernatant of the purified proteins by affinity chromatography and used for an *in vitro* assembly assay. Discs of a diameter of 17 nm were isolated by gel filtration. 50 ml of CPΔN5 discs at a concentration of 1 mg/ml were incubated with 0.05 mg of RNA for 30 minutes at room temperature. Electron microscopy demonstrated that the RNA and the protein were assembled into VLPs of regular length (150 nm) that correspond to the length of the RNA (5' 1800 nt of PapMV) used for the *in vitro* assembly assay. This result demonstrates clearly that discs of approximately 20 subunits are the building blocks of the VLPs *in vitro.* The purified K97A recombinant protein failed to assemble the RNA in VLPs under these conditions.

The K97A and E128A mutations showed completely opposite effects on the PapMV CP. To evaluate if CPΔN5 and E128A VLPs contain RNA, the 280/260 ratio of the different VLPs was measured on the spectrophotometer and compared with the proteins of the purified virus (Table 2). As expected, the VLPs showed a smaller 280/260 ratio than discs because of their lower level of RNA. The 280/260 ration of the E128A VLPs was comparable to the purified virus. Interestingly, this ratio was 50% higher with CPΔN5 VLPs. The 280/260 ratio of the isolated discs of CPΔN5 and K97A was comparable to the discs extracted with acetic acid of the purified virus.

**Table 2: OD 280/260 for PapMV VLPs**

| **Virus or VLP** | | | **Discs extracted from:** | | |
|---|---|---|---|---|---|
| Purified PapMV | CPΔN5 VLP | E128A VLP | Purified PapMV | CPΔN5 VLP | E128A VLP |
| 0.75 | 1.10 | 0.75 | 1.5 | 1.64 | 1.55 |

To evaluate if the ability to make VLPs was directly related to the affinity of CP for RNA, discs from CPΔN5 and the E128A mutants were isolated and compared to K97A. The high speed supernatant of CPΔN5 was used for isolation of the 450 kDa multimer (discs). Since the E128A mutant makes only VLPs in *E.coli,* these were disrupted using acetic acid treatment and E128A discs were isolated (Abouhaidar & Bancroft, 1978, Virology. 90, 54-9). Different amount of discs were incubated in a volume of 10 µl containing 165 fmol of a ³²P-labelled RNA probe made from a transcript of 80 nucleotides of the 5' non coding region of PapMV. The protein-RNA complex was separated by an electrophoresis mobility shift assay (EMSA). The discs of CPΔN5 interacted with the probe in a cooperative manner and induced a shift with 500 ng (22 pmol) of proteins. This result shows that the CPΔN5 discs, which are free of RNA after isolation, are able to interact with RNA *in vitro* only when a molar ratio of 1,000 (discs/RNA) is reached which, corresponds to a weak affinity for RNA. When a similar experiment was performed with the isolated discs of the mutant E128A and the same probe, the E128A discs bound RNA more efficiently than CPΔN5. As little as 50 ng of protein was sufficient to create a protein RNA complex. Purified K97A proteins in the same conditions failed, even at higher concentration (up to 1500 ng) to induce the formation of a protein RNA complex. The EMSA was repeated with RNAs extracted from CPΔN5 VLPs that were labelled as described previously and the same results were obtained with this RNA that do not contain the PapMV packaging signal.

To evaluate the stability of the VLPs and measure if the assembly of the discs into a rod structure improved the stability of the complex, their resistance to heat was monitored by CD spectrophotometry. CPΔN5 VLPs and the virus both resist to high temperature (over 60°C) before showing any sign of fatigue (Fig. 4A). The purified PapMV was the most stable structure tested and could resist temperatures approaching 100°C. The temperature of inactivation reported for PapMV is 70°C. CPΔN5 VLPs were more sensitive than the WT virus probably due to the presence of the 6xHis tag located at the C-terminus. The E128A VLPs appeared more sensitive to heat and showed sign of fatigue at 42°C (Fig. 4A). Discs were rapidly denatured at 40°C (Fig. 4B). This result suggests that the packing of the discs in the rod structure considerably improves stability.

Treatment of PapMV with trypsin results in a cleavage, presumably at amino acid 198 at the C-terminus. Under these conditions, the remaining protein was resistant to the protease. A similar assay was performed on the purified virion and recombinant VLPs and discs and indicated that PapMV did not seem to be affected by trypsin. Electron microscopy confirmed that treated virus was identical in appearance to untreated virus, however, both the isolated discs from CPΔN5 and the CPΔN5 VLPs were very sensitive to trypsin and several bands of lower molecular weight corresponding to degraded fragments were generated, suggesting that several positively charged residues are exposed and available at the surface of the VLPs. E128A showed similar resistance to trypsin as the WT virus.

### EXAMPLE II: Production and engineering of PapMV gp100 and PapMV Flu VLPs

### Cloning of the PapMV CP gene

The CPΔN5 PapMV coat protein (CP) gene was used (see Example I) and was prepared as described in Tremblay, M-H., et al., 2006, FEBS J., 273:14-25. Briefly, the CP gene was amplified by RT-PCR from isolated viral RNA using the following oligonucleotide primers.

Forward CPΔN5 primer:
5'-AGTCCCATGGATCCAACGTCCAATCTTCTG-3' [SEQ ID NO:19]

Reverse CPΔN5 primer:

The PCR product was digested with *Nco*I and *Bam*HI and inserted into the vector pET-3d to generate the CPΔN5 PapMV VLP clone, in which 5 amino acids at the N-terminus were deleted from the WT sequence. PapMV VLP also harbors the insertion of an alanine at position 2 of the recombinant protein. The amino acid sequence of the CPΔN5 PapMV VLP clone is shown in Fig. 1C [SEQ ID NO:3].

### Cloning and engineering of the PapMV gp100 and PapMV Flu constructs.

9-mer HLA-A*0201 epitopes from the well-defined tumor antigen gp100 (I*M*DQVPFSV; SEQ ID NO:21), and from influenza M1 protein (GILGFVFTL; SEQ ID NO:22) were chosen. The HLA-A*0201 epitopes were flanked on the N- and C-terminal sides by 5 residues from the respective native sequences to favour natural processing by the proteasome (see Fig. 5A).

To generate the PapMV gp100 construct, the following oligonucleotides were used:
Sense gp100 oligonucleotide: and
Antisense gp100 oligonucleotide:

These two oligonucleotides were annealed and cloned into the SpeI and MluI site of the CPΔN5 PapMV CP clone linearized with the same enzymes.

To generate the PapMV FLU construct, the following oligonucleotides were used:
Sense FLU oligonucleotide: and
Antisense FLU oligonucleotide:

These two oligonucleotides were annealed and cloned at the C-terminus of the CPΔN5 PapMV CP as described above for the gp100 construct.

The resulting PapMV gp100 and PapMV FLU constructs were comprised of the PapMV CP gene with the fusion of the respective peptide at their C-terminus followed by a 6xH tag to ease the purification process. The sequences of the PapMV clones were confirmed by DNA sequencing.

### Expression of PapMV, PapMV FL U and PapMV gp100 in E. coli

The *E. coli* expression strain BL21(DE3) RIL (Stratagene, La Jolla, CA) was transformed with the plasmid pET-3d containing one of the constructs described above and maintained in 2xYT medium containing ampicillin (50 µg/mL). Bacterial cells were grown at 37 °C to an optical density of 0.6 at 600 nm and protein expression was induced with 1 mm isopropyl-β-d-thiogalactopyranoside (IPTG). Induction was continued for 16 h at 25 °C. Bacteria were harvested by centrifugation for 15 min at 6000 r.p.m. The pellet was resuspended in ice-cold lysis buffer (50 mm NaH₂PO₄ pH 8.0, 300 mm NaCl, 10 mm imidazole, 20 µm phenylmethanesulfonyl fluoride, 1 mg/mL lysosyme) and bacteria were lysed by one passage through a French Press. The lysate was centrifuged twice for 30 min at 13 000 r.p.m. to eliminate cellular debris. The supernatant was incubated with 1 mL Ni-NTA (Qiagen, Valencia, CA) under gentle agitation for 4 h at 4 °C. Lysates were loaded onto a column and the beads were washed with 3 x 15 mL washing buffer (50 mm NaH₂PO₄ pH 8.0, 300 mm NaCl) containing increasing concentrations of imidazole (10 mm, 20 mm and 50 mm). The beads were then washed with 15 mL working buffer (10 mm Tris/HCl pH 8 or 10 mm sodium phosphate buffer pH 7.2). 2 washing steps were conducted to remove the LPS contaminants, one with 10 mM Tris-HCl, 50 mM imidazole, 0.5% Triton X100 pH 8, and another one with 10 mM Tris-HCl, 50 mM imidazole, 1% Zwittergent pH 8. Proteins were eluted in working buffer containing 1 M imidazole. The eluted proteins were subjected to a high speed ultracentrifugation (100 000 g) for 120 min in a Beckman 50.2 TI rotor. VLPs pellets were resuspended in endotoxin-free PBS (Sigma). Finally, protein solutions were filtered using 0.45 uM filters. The protein concentrations were evaluated by BCA protein kit (Pierce). The level of LPS in the purified proteins were evaluated with the Limulus test under manufacturer's instructions (Cambrex) and was below 0.005 Endotoxin Units (EU)/µg of protein. This procedure yielded more than 20mg of purified VLPs per liter of bacterial culture.

### Electron microscopy and SDS-PAGE

The proteins were diluted in PBS and absorbed for 3 min on a carbon-coated formvar grid. The grids were washed 2 times with deionized water and stained with uranyl acetate 0.1% during 10 min at room temperature. The grids were then observed on an on a Jeol JEM220FS transmission electron microscope. The average length of 100 VLPs was evaluated using the Adobe Photoshop software.

SDS-PAGE analyses were performed as described in the art (Lepage and Lapointe, 2006, Cancer Res. 66:2423-2432) using the mini-protean system from Bio-Rad (Hercules, CA). Proteins were revealed by Coomassie blue staining (Bio-Rad). In some experiments, proteinase K (Invitrogen) was added at a final concentration of 13 µg/ml.

### Results

Electron microscopy analysis of the different PapMV VLPs produced in *E. coli* (Fig. 5B) revealed the typical long rod-shaped structure ranging from 80 to 200 nm in length and a diameter of 15 nm for PapMV VLPs and 16 nm for the engineered PapMV gp100 and Flu VLPs. The PapMV CP was able to spontaneously assemble into VLPs in *E. coli* that are similar in size and shape to the PapMV VLPs. SDS-PAGE analysis was performed on fresh and 7 months old VLPs preparations in PBS at 4°C (Fig. 6). No evidence of degradation was detected on the gel. Furthermore, the preparations were incubated for an additional 7 days at room temperature or at 37°C, without any noticeable degradation. Finally, the PapMV preparations were incubated with proteinase K as a positive control for degradation, which resulted in the rapid degradation of the engineered PapMV VLPs. The PapMV VLP without a fusion peptide was more resistant to proteinase K suggesting that the fusion at the C-terminus probably locally destabilizes this region and increases susceptibility to this enzyme.

### EXAMPLE III: In vitro processing and cross-presentation of the gp100 and influenza M1 epitopes expressed on PapMVs VLPs

### Peptides

The gplOO and the Flu peptides were synthesized by GLBiochem Shangai LTD and resuspended in a DMSO (Sigma).

### Media and cell culture

T lymphocytes, dendritic cells (DC), and CD40-stimulated B lymphocytes (CD40-B) were cultured as described in the art (Lapointe et al., 2003, Can. Res. 63:653-662) in complete medium (Iscove's Modified Dulbecco's Medium; Invitrogen; Carlsbad, CA; and Wisent; St-Bruno, Québec, Canada) supplemented with 7.5% human serum (heat-inactivated, prepared from normal donors), 2 mM L-glutamine, 100 U/ml penicillin/streptomycin and 10 µg/ml gentamicin (the last 3 from Invitrogen and Wisent).

CD40-activated B cells were expanded and cultured from peripheral blood mononuclear cells (PBMC) as described in the art (Lapointe et al., 2003, Cancer Res. 63:653-662; Lepage and Lapointe, 2006, Cancer Res. 66:2423-2432) by addition of 500 ng/ml of a soluble trimeric CD40L (Immunex Corporation; Seatle, WA) and 500 U/ml recombinant human IL-4 (Peprotech; Rocky Hill, NJ).

DCs were generated from PBMC collected by apheresis preparations from normal donors (Lapointe et al., 2000, Eur. J. Immunol. 30:3291-3298), by modifying the original protocol described by Sallusto et al., 1994, J. Exp. Med. 179:1109-1118. Briefly, PBMC were enriched from blood by centrifugation on a lymphocyte separation medium (Wisent). Monocytes were enriched following 2 hours adherence in tissue culture flasks or plates at 37°C (3x10⁷ cells in T-25, 1.5x10⁷ cells/well in 6 well flat bottom plates or 5x10⁶ cells/well in 24 well flat bottom plates, all from Costar, Coming, NY). Adherent cells were washed once with PBS (Wisent) and then cultured in complete medium supplemented with 100 ng/ml of GM-CSF (1,000 U/ml) and 500 ng/ml of IL-4 (1,000 U/ml) (both from Peprotech, Rocky Hill, NJ). GM-CSF and IL-4 were added again on days 3 and 5. PapMV VLPS (prepared as described in Example II) were added on day 6 and harvested at day 7 for recognition and expansion experiments.

The melanoma cell line 1088mel was established at the Surgery Branch (NCI/NIH). SK23, T2, and breast tumor cell lines MDA231 were obtained from the American Type Culture Collection (ATCC; Manassas, VA). All tumor cell lines were cultured in RPMI 1640 medium supplemented with 10% heat-inactivated FBS, 2 mM L-glutamine, 100 U/ml penicillin/streptomycin and 10 µg/ml gentamicin.

### Cross-presentation from PapMV pulsed APC

Different target cells were analyzed for MHC class I presentation of defined epitopes with gp100- or Influenza M1-specific T cells. Gp100-specific CD8⁺ T cell clones were kindly provided by the National Cancer Institute; NIH, Bethesda, MD and were specific to both the native HLA-A*0201-restricted epitope at position 209-217 (ITD QVP FSV; SEQ ID NO:27), and to the modified version with an M at position 210 (IMD QVP FSV; SEQ ID NO:21), which enhanced the stability of the peptide/MHC complex. Gp100-specific T cells were expanded using the rapid expansion protocol described by Dudley et al., 1999, J. Immunother, 22:288-298.

T cell lines specific to the Influenza M1-derived HLA-A*0201-restricted epitope (59-67; GIL GFV FTL; SEQ ID NO:22) were generated as follows. PBMC from HLA-A*02⁺ normal donors were identified by flow cytometry (FACScalibur; BD Biosciences, Mississauga, ON) with a specific antibody (OneLambda, Canoga Park, CA). PBMC prepared as described above, were stimulated in multiple wells of 48 well plates with 1 µM of synthetic FLU peptide in medium described above. Cultures were re-stimulated 7 days later with either peptide pulsed (1 µM for 3 hours followed by 3 washes in PBS) autologous PBMC or CD40-stimulated B lymphocyte cultures. Interleukin (IL)-2 (Chiron, Emeryville, CA) was then added every 2-3 days at 150 IU/ml and cultures were kept between 0.5 and 2X10⁶ cells/ml. Specificity of individual cultures was assessed by interferon (IFN)-γ secretion assays by ELISA with coupled antibodies (Endogen; Woburn, MA) after co-culture with peptide pulsed T2 cells as described in the art (Lapointe et al., 2001, J. Immunol. 167:4758-4764).

To evaluate cross-presentation mediated by PapMV CP, CD40-activated B cells or DC were pulsed with various versions of the modified PapMV CP at 10-50 µg/ml for 20 hours. Cells were harvested, washed twice with PBS, and seeded in complete media (at 4-10X10⁴ cells/well) in 96 well-plates. Gp100- or Influenza M1-specific T cells were added at 2-10X10⁴ cells/well in complete media for 20 hours. Culture supernatants were harvested and interferon (IFN)-γ was evaluated by ELISA as described in the art (Lepage and Lapointe, 2006, Cancer Res. 66:2423-2432). In some experiments, APCs were pre-treated for 1 hour with 50-70 µM chloroquine (Sigma), or 20-25 µg/ml lactacystin or 1.3-3.3 µM MG-132 (the last 2 from Calbiochem, San Diego, CA). Cells were washed with PBS and re-suspended in media containing 1/20 of the original inhibitor concentration. Treated cells were pulsed with the different PapMV variants and analysis of MHC class I mediated presentation was performed as described above.

### T cell expansion

CD40-activated B cells or DC were pulsed with various versions of the PapMV CP (as described in Example II above) for 20 hours. Cells were harvested and washed twice with PBS. Pulsed APC (2-5X10⁵) were co-cultured with autologous PBMC at 2X10⁶ cells in 500 µl of complete media, in single wells of a 48 well-plate. When media turned yellow, 200 µl of medium was removed and 400 µl fresh complete media was added. On day 7 to day 10, freshly PapMV-pulsed APC (5X10⁵; pulsed for 20 hours and washed twice in PBS) were added to individual cultures. IL-2 was then added at 100 IU/ml and every 3 days.

T cell specificity was then assessed on day 15 to 20. Briefly, expanded cells were co-cultured with peptide pulsed T2 cells as described in the art (Lapointe et al., 2001, J. Immunol. 167:4758-4764), or PapMV-pulsed APC. IFN-γ secretion was then evaluated by ELISA with coupled antibodies (Endogen; Woburn, MA) as described in the art (Lepage and Lapointe, 2006, *ibid*.). Alternatively, the frequency of antigen-specific T cells was assessed by ELISPOT assay, using coupled antibodies (MABTECH) according to manufacturer's instructions. Spots were enumerated with an automated counter (CTL Technologies, Cleveland, OH).

### Results

DC are defined in the art as the optimal APC, and it has been demonstrated in the current experiment and in the art that B lymphocytes expanded after CD40 stimulation are efficient APC. These APC were pulsed with the PapMV VLPs, PapMV gp100 and PapMV-Flu and co-cultured with defined T cells specific to MHC class I epitopes from gp100 and Influenza M1 proteins. As presented in Fig. 7A, only APC pulsed with PapMV gp100 were recognized by the gp100-specific T cell clone. Conversely, only PapMV Flu-pulsed APC were recognized by the Influenza M1 specific T cells (Fig. 7B). The specificity of each T cell cultures was confirmed by pulsing CD40-activated B cells with the synthetic peptides corresponding to each epitopes. Also, the addition of PapMV-Flu to the specific T cells failed to stimulate them to secrete IFN-γ, indicating that APC are essential for peptide recognition.

To confirm that HLA-A*02 was the restriction element involved in peptide presentation, similar experiments were performed with APC prepared from 2 additional HLA-A*02 donors, and 2 others negative for this allele. As shown in Fig. 8A, the FLU-specific T-cells were mostly reactive with PapMV-Flu pulsed on HLA-A*02⁺ donors (left panel). The weak reactivity with donor #3 may be explained by the fact that the FLU T-cell line was heterogeneous, and T-cells specific to the peptide, potentially presented by another MHC class I allele, may be present in the culture. Moreover, the gp100-specific T-cell clone was reactive only with PapMV gp100-pulsed HLA-A*02⁺ APC (Fig. 8A; right panel), further confirming that APC expressing the relevant restriction element are necessary for antigenic presentation. Finally, presentation by MHC class I was controlled using antibodies blocking either MHC class I, or class II or HLA-DR presentation, as performed in the art (Lapointe et al., 2003, Cancer Res. 63:2836-2843; Lapointe et al., 2001, J. Immunol. 167:4758-4764). As a control, a melanoma line expressing both HLA-A*0201 and gp100 was co-cultured with the gp100-specific T-cell clone, and only antibody blocking MHC class I presentation abrogated the recognition, as expected (Fig. 8B; right section). Co-culture of gp100-specific T-cell clones with lines either HLA-A*02⁻/gp100⁺, or +/-, or -/- failed to provoke IFN-γ secretion as demonstrated in the art (Dudley et al., J. Immunother. 22:288-298; Lapointe et al., 2001, J. Immunol. 167:4758-4764). When the panel of blocking antibodies was applied to the PapMV Flu-pulsed APC, only the anti-MHC class I decreased the recognition by the specific T cell line (Fig. 8B; left section). These last data demonstrate that the FLU peptide derived from PapMV-Flu was presented by MHC class I, specifically HLA-A*02.

In order to determine whether the processing of the PapMV VLPs was mediated by the proteasome, two different proteasome inhibitors were exploited, namely, lactacystin and MG-132, and their activities controlled by blocking classical MHC class I presentation of the HLA-A*0201 epitope from gp100 by melanoma cells (Fig. 9, right section). When using PapMV Flu-pulsed APC, the presentation of the M1 peptide was unaffected by both inhibitors (Fig. 9, left section). The pre-treatment with chloroquine, which neutralizes the pH of endosomes, had a weak negative effect on the cross-presentation of the FLU epitope, while similar treatment did not change the classical MHC class I presentation of the melanoma cells, as expected. Overall, these data suggests that the MHC class I cross-presentation mediated by the PapMV VLPs is proteasome-independent.

Also evaluated was whether APC pulsed with the PapMV VLPs would have the capacity to expand antigen-specific T lymphocytes from an heterogeneous T cell population from PBMC. DC and CD40-activated B lymphocytes were pulsed with PapMV VLPs and pulsed APC were co-cultured with autologous PBMC according to the protocol described above. The frequency of specific expanded T cells was first evaluated by ELISPOT assay. As shown in Fig. 10A, cells specific to HLA-A*0201 Influenza M1 peptide were generated when PapMV Flu-pulsed APC were used with PBMC. Un-pulsed APC, or the one pulsed with PapMV or PapMV gp100 failed to generate Flu specific T cells, as expected. No gp100-specific T cells were generated, as expected for a healthy normal donor with no melanoma. Expanded T cells were next evaluated for reactivity against various pulsed APC, and IFN-γ secretion seemed equally high when T cells expanded with APC pulsed either with the FLU peptide or PapMV Flu were used to expand T cells (> 5000; Fig. 10B). APC pulsed with either PapMV, PapMV gp100 or PapMV Flu failed to generate T cells specific to the PapMV CP, suggesting that cellular pre-immunity to the PapMV CP is marginal. Finally, expanded T cells were co-cultured with T2 cells pulsed with different amount of the HLA-A*0201 Influenza M1 peptide (Fig. 11). In two independent experiments, highly reactive T cells with high avidity were generated, since T cells had the capacity to recognize T2 cells pulsed with 0.1 and 0.01 nM of the peptide. Secretion was mostly > 100 000 pg/ml, which is very high, and T cells raised with APC pulsed with the Influenza M1 peptide were generally less avid. From these experiments, it is concluded that PapMV-pulsed APC had the capacity to expand specific T cells with high avidity. Interestingly, there is no evidence of pre-existing cellular pre-immunity to the PapMV CP.

### EXAMPLE IV: Production and engineering of PapMV VLPs comprising the H1N1 M2e influenza antigen

### Cloning and engineering of PapMV coat protein

The PapMV CP gene was amplified by RT-PCR from isolated viral RNA using the following primers:
5'-AGTCCCATGGCATCCACACCCAACATAGCCTTC-3'[SEQ ID NO:28], and

The PCR product was cloned as an *Nco*I/*Bam*HI fragment into pET 3D (New England Biolabs) to produce the truncated coat protein CPΔN5. To facilitate the fusion of the M2e peptide at the C-terminus of CPΔN5, SpeI and MluI restriction sites were introduced at the C-terminus of the protein by PCR using SEQ ID NO:28 and the primer:

The PCR product was cloned as an *Nco*I/*Bam*HI fragment into pET 3D (New England Biolabs). The resulting clone, CPΔN5-SM, was used for cloning and fusion of all the other M2e constructs. The amino acid sequence of CPΔN5-SM is provided in Fig. 1F (SEQ ID NO:46) and the nucleotide sequence is provided in Fig. 1M (SEQ ID NO:49).

To generate the PapMVCP-M2e construct, the following oligonucleotides were used:
M2e forward: and
M2e reverse:

These two oligonucleotides were annealed together and digested with SpeI and MluI before ligation into the SpeI/MluI-linearized CPΔN5-SM clone. The DNA sequence of PapMVCP-M2e clone was confirmed and is shown in Fig. 1E [SEQ ID NO:5]. The amino acid sequence encoded by the PapMVCP-M2e clone is shown in Fig. 1D [SEQ ID NO:4] PapMV and PapMV-M2e expression and purification.

Expression and purification of PapMVCP constructs were performed as previously described (Tremblay et al. 2006, FEBS J. 273:14) with minor modifications. Briefly, the bacteria were lysed through a French Press and then loaded onto a Ni2+ column, washed with 10 mM Tris-HCl, 50 mM Imidazole, 0.5% Triton X100, pH8, then with 10 mM Tris-HCl, 50 mM Imidazole, 1% Zwittergent, pH8, to remove endotoxin contamination. To isolate the VLPs, the eluted proteins were subjected to high speed centrifugation (100 000 g) for 120 min in a Beckman 50.2 TI rotor. The VLPs found in the pellet were resuspended in endotoxin-free PBS (Sigma). The PapMV-M2e discs were obtained from the supernatant of the ultracentrifugation. The buffer exchange of the discs was performed by dialysis against endotoxin-free PBS (Sigma). The M2 peptide used for the ELISA was synthesized by GLBiochem Shangai LTD and resuspended in a endotoxin free PBS (Sigma). Protein solutions were filtrated using 0.45 µM filters before use. The amount of protein was evaluated using a BCA protein kit (Pierce). The level of LPS in the purified protein was evaluated with the Limulus test according to the manufacturer's instructions (Cambrex) and was below 0.005 endotoxin units (EU)/µg of protein.

### Electron microscopy

SDS-PAGE and electroblotting were performed as described previously (Tremblay, et al., 2006, *ibid*.). Proteins were diluted in PBS and were absorbed for 3 min on a carbon-coated formvar grid. The grids were washed twice with deionised water and stained with 0.1% uranyl acetate for 10 min at room temperature. The grids were then observed on a Jeol JEM220FS transmission electron microscope. Average VLP length was evaluated by measuring 100 VLPs using Adobe Photoshop software.

### Results

The universal M2e peptide derived from the M2 (ion channel) of Influenza virus strain H1N1 (A/New Caledonia/20/99) was used in this Example. This peptide is also found in more than 85% of influenza virus strains infecting humans. The peptide was fused to the C-terminus of the PapMV CP followed by a 6xH tag (Fig. 12A). The expression of PapMV-M2e in *E. coli* resulted in the production of PapMV-M2e VLPs of about 100nm in length that are similar to the PapMV-VLPs (Fig. 12B) and discs made of 20 subunits of the CP subunits. The VLPs and the discs could be easily purified using a 6xH tag located at the C-terminus of the recombinant protein and a Ni2+ column (Qiagen). The purification of each of those forms can be easily achieved through ultracentrifugation (100,000g for 90 min.) where the VLPs are isolated from the pellet and the discs from the supernatant.

### EXAMPLE V: Immunization of mice with PapMV VLPs comprising the M2e influenza antigen

PapMV VLPs and PapMV-M2e VLPs were prepared as described in Example IV.

### Immunization of mice

Five 4- to 8-week-old Balb/C mice (Charles Rivers Laboratories) were injected subcutaneously with 100 µg of PapMV VLPs, PapMV-M2e VLPs or PapMV-M2e VLPs + IgG. IgG used in the third group, refers to 0.5µl of polyclonal serum isolated from mice vaccinated with PapMV-M2e VLPs. Primary immunization was followed by one booster dose given 2 weeks later. Blood samples were obtained at different time points and stored at -20°C until analysis.

### ELISA quantification

Costar High Binding 96-well plates (Corning, NY, USA) were coated overnight at 4°C with 100 µl/well of the M2 peptide (SSLLTEVETPIRNEWGCRCNDSSD; SEQ ID NO:6) diluted to a concentration of 1 µg/ml in 0.1 M NaHCO₃ buffer pH 9.6. The plates were blocked with PBS/0.1% Tween-20/2% BSA (150 µl/well) for 1 hour at 37°C. After washing three times with PBS/0.1% Tween-20, sera were added in 2-fold serial dilution beginning from 1:50 and incubated for 1 hour at 37°C. Following incubation, the plates were washed three times and incubated with 100 µl of peroxidase-conjugated goat anti-mouse IgG, IgG2a, IgG2b (all from Jackson Immunoresarch), IgG3 (Rockland) at a dilution of 1/10,000 in PBS/0.1% Tween-20/2% BSA for for 1 hour at 37°C. After three washes, the presence of IgG was detected with 100 µl of TMB-S according to the manufacturer's instructions; the reaction was stopped by adding 100 µl of 0.18 mM H₂SO₄ and the OD was read at 450nm. The results are expressed as antibody endpoint titer, determined when the OD value is 3-fold the background value obtained with a 1:50 dilution of serum from PBS mice.

### Virus growth, titration, and protection assay

Influenza virus (A/WSN/33, H1N1 subtype) was grown, purified, and titrated as previously described (Abed et al., 2004, Antivir. Ther. 9 :577-581). Lung virus titers and neutralization titers were determined on Mardin-Darby canine kidney (MDCK) cells as previously described (Abed et al., 2004, *ibid*). Mice were infected intranasally with 100µl of virus diluted in PBS (lethal infection, 3000 PFU/ mouse of live virus or 3LD₅₀). The development of symptoms was monitored at day 2, 5, 6, 7, 8, 9, 10, 12, and 20 after infection.

### Results

To examine the capacity of PapMV-M2e VLPs or discs to induce an immune response, 20 Balb/C mice were injected subcutaneously with 100 µg of the recombinant VLPs or discs. The amount of M2e peptide present in each dose is estimated at 8 µg. A booster dose was given on day 15 after primary immunization. Mice sera were assayed for anti-M2e antibodies 23 days after injection (Fig. 13). The analysis of the immune response reveals that the PapMV-M2e discs are weakly or non-immunogenic. However, PapMV-M2e VLPs were able to trigger a humoral response that reached high titers (>1/1600; 8 on the scale log (2x50) shown in Fig. 13) in 11 of the 20 mice treated. The remaining the mice (9/20) also showed an antibody response to the PapMV-M2e, although the titers were lower. A large variation between the total IgG response was observed (Fig. 13).

To improve the immunogenicity of the VLPs, the VLPs were opsonised with serum from vaccinated mice. The immunoregulatory properties of antibodies have been peviously demonstrated and were showed to play an active role in stimulating the immune response (for review see Brady et al., 2005, Infection and Immunity, 75:671-678). 0.5µl of serum isolated from a pool of 5 vaccinated mice was added to a volume of 200µl of the PapMV-M2e VLPs (containing 100µg of VLPs) that showed high titers and was administered by the subcutaneous route. The improvement was noticeable when compared to VLPs alone (Fig. 13) in that 19 out of 20 mice triggered production of high titers of antibodies.

### Protection assay against challenge with Influenza

Mice were challenged with 3 x 10³ pfu (3LD₅₀) of the influenza H1N1 strain A/WSN/33 at day 28. This strain was adapted to grow in mice and show a high virulence (Francis and Moore, 1940, J. Exp. Med. 72, 717-728). The development of the symptoms was followed on a daily basis. The animals were sacrificed once they had lost more than 25% of their body weight. The results clearly demonstrated that the PapMV-M2e VLPs could provide protection to 45% (9 out of 20) of the mice toward infection (Fig. 14). The opsonisation of the PapMV M2e VLPs with serum isolated from a pool of mice vaccinated with PapMV-M2e VLPs (as described above) significantly improved the protection to challenge such that 80% of the mice were protected. The results suggest that the improvement of the antibody titers observed with opsonised VLPs (Fig. 14) was translated as an improved protection to infection.

The titers in IgG2a and IgG2b of each of the vaccinated mice with the PapMV-M2e VLPs and the PapMV-M2e VLPs + IgG were evaluated. The results were plotted to see a correlation between the level of those antibodies subtyped and the level of protection (Fig. 15). The results clearly demonstrated that mice that show high titers of IgGa and IgGb also show the best protection to the challenge. Although some protected animals were found to show only one of the subtyped IgGs (2 out of 20), the results generally indicate that both IgGa and IgGb antibody isotypes were important in the protection against infection with Influenza.

The titers of IgG and the level of protection were improved in animals that were vaccinated with PapMV-M2e VLPs + IgG. Animals that survived challenge were observed in general to show lighter symptoms of infection when they were vaccinated with the opsonised particles. To confirm this observation, the weight loss of the animals that survived the vaccination with PapMV-M2e VLPs (9 animals) was plotted and compared to the weight of animals treated with the opsonised VLPs (16 animals) (Fig. 16). The graph clearly shows that mice vaccinated with the opsonised particles lost less weight and showed a better fitness that mice vaccinated with VLPs alone.

### EXAMPLE VI: Production and engineering of PapMVCP-E2 and PapMVCP₂₇₋₂₁₅-E2

This Example demonstrates that multimerisation of the PapMV coat protein is essential for the immunogenicity of the PapMV VLPs.

### Cloning and engineering of the PapMV coat protein

The PapMV CPΔN5-SM gene was cloned as described in Example IV. To generate the PapMVCP-E2 construct, the following oligonucleotides were used: and

These two oligonucleotides were annealed together and digested with *Spe*I and *Mlu*I before ligation into the *Spe*I/*Mlu*I-linearized PapMV CPΔN5-SM clone.

The expression vector for the truncated coat protein E2 fusion, PapNWCP₂₇₋₂₁₅-E2, was constructed from the PapMVCP-E2 plasmid by first preparing the following two oligonucleotides (including an *Nco*I restriction site) designed to delete the 26 first amino acids of the PapMV CP. These oligonucleotides were used to PCR amplify the truncated coat protein:
forward primer:
   5'-AGTCCCATGGCCGATCCAACGTCCAATCTTCTG-3' [SEQ ID NO:34], and
reverse primer:
   5'-ACGTCCATGGTATATCTCCTTCTTAAAG-3' [SEQ ID NO:35].

The PCR product was then self-ligated. The expression vector for PapMVCP₂₇₋₂₁₅ was derived from the PapMVCP plasmid following the same procedure as for the construction of the PapMVCP₂₇₋₂₁₅-E2 clone. The sequences of all PapMV clones were confirmed by DNA sequencing.

### PapMVCP-E2 and PapMVCP₂₇₋₂₁₅-E2 expression and purification

Expression and purification of PapMVCP constructs were performed as described in Example IV. The E2 peptide was synthesized by GLBiochem Shangai LTD and resuspended in a endotoxin free PBS (Sigma). Protein solutions were filtered using 0.45 µM filters before use. The amount of protein was evaluated using a BCA protein kit (Pierce). The level of LPS in the purified protein was evaluated with the Limulus test according to the manufacturer's instructions (Cambrex) and was below 0.005 endotoxin units (EU)/µg of protein.

### SDS-PAGE, electroblotting and electron microscopy

SDS-PAGE and electroblotting were performed as described in the previous Examples. Proteins were diluted in PBS and were absorbed for 3 min on a carbon-coated formvar grid. The grids were washed twice with deionised water and stained with 0.1% uranyl acetate for 10 min at room temperature. The grids were then observed on a Jeol JEM220FS transmission electron microscope. Average VLP length was evaluated by measuring 100 VLPs using Adobe Photoshop software.

### Immunization

Five 4- to 8-week-old C3H/HeJ mice (Charles Rivers Laboratories) were injected subcutaneously with 25 µg of PapMVCP-E2, PapMVCP₂₇₋₂₁₅-E2 or the equivalent amount of the E2 peptide (2 µg) or endotoxin-free PBS (Sigma). Primary immunization was followed by one booster dose given 2 weeks later. Blood samples were obtained at different time points and stored at -20°C until analysis. All the experimental protocols were approved by the Laval University animal protection committee.

### ELISA quantification

Costar High Binding 96-well plates (Coming, NY, USA) were coated overnight at 4°C with 100-200 µl/well of P3, P3E2, PapMVCP, PapMVCP₂₇-₂₁₅, or PapMVCP-E2 diluted to a concentration of 1 µg/ml in 0.1 M NaHCO₃ buffer pH 9.6. The plates were blocked with PBS/0.1% Tween-20/2% BSA (150 µl/well) for 1 hour at 37°C. After washing three times with PBS/0.1% Tween-20, sera were added in 2-fold serial dilution beginning from 1:50 and incubated for 1 hour at 37°C. Following incubation, the plates were washed three times and incubated with 100 µl of peroxidase-conjugated goat anti-mouse IgG, IgG1, IgG2a, IgG2b (all from Jackson Immunoresarch), IgG3 (Rockland) at a dilution of 1/10,000 in PBS/0.1% Tween-20/2% BSA for for 1 hour at 37°C. After three washes, the presence of IgG was detected with 100 µl of TMB-S according to the manufacturer's instructions; the reaction was stopped by adding 100 µl of 0.18 mM H₂SO₄ and the OD was read at 450nm. The results are expressed as antibody endpoint titer, determined when the OD value is 3-fold the background value obtained with a 1:50 dilution of serum from PBS mice.

For the determination of antibody levels in human sera, the same conditions were applied, except that the peroxidase-conjugated goat anti-human IgG as secondary antibodies were used at a dilution of 1/80 000. Sera from infected HCV patients were provided by B. Willems (Hopital Saint Luc, CHUM): the results are expressed as antibody endpoint titer, defined as when the OD value is 3-fold the background value obtained with a 1:25 dilution of serum from a pool of sera from 15 non-infected patients.

### Splenocyte restimulation

CD-1 mice (22 weeks old) were immunized with PapMVCP-E2 (25 µg) on days 0, 15, 30 and 45 before being sacrificed on day 65. Spleens were removed and suspended in DMEM (2 x 10⁵ cells/well). Red blood cells were removed with hypertonic ammonium chloride solution. Splenocytes were washed and resuspended in 200 µl of DMEM medium (DMEM supplemented with 10% FBS [HyClone], 100 U/ml penicillin, 100 µg/ml streptomycin, 2 mM L-glutamine, 1 mM sodium pyruvate, and 50 µM β-mercaptoethanol) to a concentration of 2.5 x 10⁵ cells/ml in 96-well flat-bottom microplates (Costar). Samples were incubated four days with 25 µg/ml of PapMVCP or PapMVCP-E2 VLPs. Concavalin A (ConA - 10 µg/ml) and PBS were used as positive and negative controls. Cells culture supernatants were collected and cytokines were measured using the liquid mouse 10 cytokines kit (Qiagen).

### Air Pouch in mice

Air pouches were raised in 10- to 12-week-old CD-1 mice (Charles River Laboratories). Air pouches were raised on the dorsum by subcutaneous injection of 3 ml of sterile air on days 0 and 3. On day 7, one ml of recombinant PapMVCP (1 to 10 µg /ml), LPS (10 µg/ml) or PBS were injected in the air pouches. Six hours after the treatment, the mice were killed by asphyxiation using CO₂. The air pouches were washed once with 1 ml PBS-5 mM EDTA, and then twice with 2 ml of PBS-5 mM EDTA, and the exudates were centrifuged at 500 x g for 5 min at room temperature. Cells were counted with a hematocytometer following acetic blue staining.

### Bone marrow cell extraction and differentiation in APCs

Bone-marrow progenitors cells were obtained from the femurs of BALB/c mice and cultured for 6 days in dendritic cells differentiation bone marrow medium (95% RPMI with 1% penicillin-streptomycin and supplemented with 5% X63-GM-CSF supernatant media culture; the X63-GM247 CSF cell line was provided by B. Ludewig, Research Department, Kantonal Hospital St. Gallen, Switzerland). Medium was partially replaced on day 2 and 4. On day 6, the medium was replaced by medium without LX63 conditioned medium. On day 7, enrichment of APCs was verified by flow cytometry using FITC anti-CD11c and PE-Cy5.5 anti-CD11b surface markers (BD Biosciences). The preparation contained 25% of CD11c+Cd11b+ cells, and more than 80% of CD11b+ cells. This preparation is referred to as "APCs."

### Flow Cytometry

To evaluate the internalization of the PapMVCP-E2 or PapMVCP₂₇₋₂₁₅-E2 in APCs, 1 million bone marrow derived APCs were incubated for 2 hours at 37°C with either 25 µg of PapMVE2 or PapMVCP₂₇₋₂₁₅-E2. Briefly, cells were blocked with PBS 10% FBS and anti-CD16/CD32 (1µg/l million cells) for 15 min at 4°C. After 2 washes with PBS, cells were fixed with PBS/2% paraformaldehyde for 10 min at room temperature. After 2 washes with permeabilization buffer (PBS, 10 % FBS, 0.2 % Triton X-100), cells were incubated for 45 min at 4°C with rabbit polyclonal antibodies diluted 1/200 in permeabilization buffer. After 2 washes with permeabilization buffer, cells were incubated for 45 min at 4°C with the secondary antibodies (anti-rabbit IgG alexa 488 (Molecular Probes)) diluted 1/5000 in permeabilization buffer. After washing with PBS, cells were immediately analysed with an EPICS-XL cytofluorometer. Data analysis was performed using WINMDI2.8. The rabbit polyclonal Ab used for detection was produced in the inventor's facilities: rabbit preimmune serum was used as a negative control.

### Confocal Microscopy

APCs were grown (200,000 cells/well) in a 12-well plates (Coming, NY, USA) containing sterile slides in the bottom following the same differentiation protocol as described previously. For antigen internalization studies, 5 µg of antigen /200 000 cells was used. Fixation, permeabilization, and primary and secondary antibody incubation steps were as described for flow cytometry. Slides were analysed immediately with a Fluoview Fv300 confocal microscope with a X60 oil immersion objective. Fluorescence images were acquired sequentially to avoid non-specific channel interference and by x-z sectioning. Pictures were then digitally processed with Image J software.

### Statistical analysis

Nonparametric Krustal-Wallis and Dunn's multiple comparison tests were used for statistical analysis. A value of P<0.05 was considered statistically significant. Statistical analyses were performed with the program PRISM 3.03.

### Results

The purified recombinant proteins showed the expected molecular weights of 23kDa (PapMVCP₂₇₋₂₁₅-E2) and 26kDa for PapMVCP and PapMVCP-E2 and endotoxin levels were always below 0.005 EU/ µg of protein. Electron microscopy (EM) observations confirmed that the addition of the E2 peptide at the C-terminus of the PapMVCP did not affect the ability of the protein to self-assemble into VLPs that are similar to the recombinant PapMVCP VLPs. As expected, PapMVCP₂₇₋₂₁₅-E2 was unable to form VLPs and remained as a monomeric form as previously shown (Leclerc et al., 1998, J Biol Chem, 273:29015-21). The lengths of the VLPs were variable, with a size range of 201 ± 80 nm. A 201 nm length protein represents 560 copies of the CP presenting the E2 peptide in a repetitive and crystalline fashion.

To test the pro-inflammatory properties of PapMVCP VLPs, the mouse air pouch model was used. Injection of 10 µg of PapMVCP VLPs with very low LP content (< 0.005 EU/µg) failed to induce the recruitment of leukocytes into the pouch of CD1 mice six hours after the treatment. In contrast, injection of LPS at doses of 1,000 and 1 EU was very effective in inducing the recruitment of leukocytes (Fig. 17). This result suggests that PapMVCP VLPs are not pro-inflammatory after 6 hours and that the very low level of LPS in PapMVCP protein samples would not exert any notable immunogenic effects in subsequent experiments.

The capacity of the monomeric (PapMVCP₂₇₋₂₁₅-E2) and the multimeric (PapMVCP-E2) forms to be internalized in bone marrow derived APCs enriched in bone-marrow-derived dendritic cells (BMDDC) was tested. Flow cytometry analysis showed that APCs become efficiently immunolabelled by both the multimeric and the monomeric forms (95.3% for the PapMVCP-E2 VLP and 92.6% for the PapMVCP₂₇-₂₁₅-E2 VLP). To visualize the interaction between the recombinant proteins and the APCs, the treated APCs were observed by confocal microscopy. In both cases, the immunolabelled PapMVCP signal was clearly vesicular, intracytoplasmic and perinuclear. Both recombinant proteins were efficiently internalized by the APCs.

To examine the capacity of PapMVCP VLPs to induce an immune response, C3H/HeJ mice were injected subcutaneously with 25 µg of the recombinant VLPs (PapMVCP-E2) or 25 µg of the monomeric form (PapMVCP₂₇-₂₁₅-E2). The amount of E2 peptide present in each dose is estimated at 2 µg. A booster dose was given on day 15 after primary immunization. Mice sera were assayed for anti-PapMVCP, PapMVCP₂₇₋₂₁₅ and anti-E2 peptide antibodies. Anti-CP IgG was clearly detected in mice immunized with PapMVCP-E2 on day 12, while only a weak level of anti-CP was detected in the sera of mice vaccinated with PapMVCP₂₇-₂₁₅-E2, even after the booster on day 15 (Fig. 18).

### EXAMPLE VII: Adjuvant effect of PapMV

### PapMV purification

PapMV was purified as described in Example I.

### Antigens

LPS-free OVA Grade VI was purchased from Sigma-Aldrich Chemical Co, St Louis, MO. Hen egg white lysozyme (HEL) was purchased from Research Organics Inc. Cleveland, OH. LPS from *E. coli* O111:B4 was purchased from Sigma-Aldrich, St Louis, MO.

### Immunizations

BALB/c mice, 6-8 weeks old, were bred and kept under the animal facilities of the Experimental Medicine Department, Faculty of Medicine, National Autonomous University of Mexico (UNAM), and were cared for in conformity with good laboratory practice guidelines. To study the effects of adjuvant, groups of mice were immunized i.p. on day 0 with 2 mg of OVA or HEL alone or with 30 mg of PapMV, CFA 1:1 (v/v), or 5 mg of LPS from *E. coli* O111:B4 (Sigma-Aldrich). Control mice were injected with saline solution only. Blood samples were collected from the retro-orbital sinus at various times, as indicated in Fig. 19. Individual serum samples were stored at -20 °C until analysis. Three mice were used in each experiment.

### Determination of antibody titers by ELISA

High-binding 96-well polystyrene plates (Corning®, New York, NY) were coated with 1 mg/mL of PapMV, 100 mg/mL of HEL, or 150 mg/mL OVA in 0.1 M carbonate-bicarbonate buffer (pH 9.5). Plates were incubated for 1 h at 37 °C and then overnight at 4 °C. Before use the next morning, plates were washed three times in PBS (pH 7.2) containing 0.05% Tween-20 (PBS-T) (Sigma-Aldrich). Nonspecific binding was blocked with 5% nonfat dry milk diluted in PBS (PBS-M) for 1 h at 37°C. After washing, mice serum was diluted 1:40 in PBS-M, and 2-fold serial dilutions were added to the wells. Plates were incubated for 1 h at 37 °C and then washed four times with PBS-T. Peroxidase-conjugated rabbit anti-mouse IgM (optimal dilution 1:1000) IgG, IgG1, IgG2a, IgG2b antibodies (Zymed, San Francisco, CA) or IgG3 (optimal dilution 1:3000)(Rockland, Gilbertsville, PA) was added, and the plates were incubated for 1 h at 37 °C and washed three times with PBS-T. Orthophenylenediamine (0.5 mg/mL; Sigma-Aldrich) in 0.1 M citrate buffer (pH 5.6) containing 30% hydrogen peroxide was used as the enzyme substrate. The reaction was stopped with 2.5 N H₂SO₄, and the absorbance was determined at 490 nm using an automatic ELISA plate reader (Dynex Technologies MRII, Chantilly, VA, USA) with BIOLINX 2.22 software. Antibody titers are given as -log₂ dilution x 40. A positive titer was defined as 3 SD above the mean value of the negative control.

### Results

The translation of innate immune response into antibody response is observed when adjuvants are co-administered with poorly immunogenic vaccines. Adjuvants are substances capable of strengthening or augmenting the antibody or cellular immune response against an antigen. To determine whether PapMV is capable of acting as an adjuvant to promote a long-lasting antibody response to other antigens, BALB/c mice were immunized with OVA or HEL either alone or together with the following adjuvants: PapMV, CFA, or LPS. The IgG antibody titer specific for OVA or HEL was measured by ELISA at the time points indicated (Figure 19A and B). An adjuvant effect for PapMV was observed in the total IgG response to OVA and HEL in immunized animals. An adjuvant effect induced by PapMV was observed for HEL on day 30 after immunization, when the antibody titer increased 8-fold compared with the antibody titer induced by HEL alone. This difference in antibody titer was maintained until day 120 but had dropped by day 400 (Figure 19A). Although LPS induced an adjuvant effect only in the first 30 days after immunization, CFA showed the strongest adjuvant effect from day 8 to the end of the experiment on day 400 after immunization (Figure 19A). For immunization with OVA, the adjuvant effect on total IgG antibody titers was observed until day 120 after the first immunization, after which antibody titer decreased with time and was 4-fold higher compared with OVA alone on day 400 (Figure 19B). Further analysis was performed on day 20 to identify which IgG subclasses were induced by OVA and OVA coimmunized with adjuvants (where PapMV did not show an adjuvant effect on the total IgG response). PapMV, LPS, and CFA induced OVA-specific IgG2a and IgG2b antibody titers, whereas OVA alone induced only IgG1-specific antibody titers (Figure 19C-E). No adjuvant effect for IgG1 was observed when OVA was coimmunized with any of the adjuvants used.

These results show that PapMV, LPS, and CFA induce an adjuvant effect on the IgG subclass responses to OVA. Moreover, PapMV exhibits adjuvant properties that induce a long-lasting increase in specific antibody titers to model antigens. Taken together, these data demonstrate that PapMV has intrinsic adjuvant properties that may have mediated the translation of the innate response into the observed antigen-specific long-lasting antibody response.

### EXAMPLE VIII: Preparation and engineering of PapMV VLPs comprising affinity peptides

The following Example demonstrates a method of selecting affinity peptides and the engineering of PapMV coat protein such that the selected affinity peptides are expressed on the surface of the VLP formed from the engineered coat protein and can be used to bind a target macromolecule. This concept is readily applicable to the production of VLPs expressing a peptide or antibody fragment on its surface, which can be used to bind to an influenza virus epitope/antigen and thereby form an APS.

### Bacterial strains

*E. coli* strain ER2738, provided with the Ph.D.-7 Phage Display Peptide Library Kit (New England Biolabs, Inc.), was used for the amplification of phage. *E. coli* strain DH5α (Invitrogen) was used for plasmid propagation, and *E. coli* BL21-CodonPlus® (DE3)-RIL (Stratagene) was used for production of recombinant proteins.

### Screening of peptides

Specific peptides against *Plasmodiophora brassicae* resting spores (Horti-Protection, Inc.) were selected using a Ph.D-7 Phage Display Peptide Library Kit (New England Biolabs, Inc.) by an *in vitro* selection process known as "panning" according to the manufacturer's protocol. Briefly, 2x10¹¹ phages were added to 10⁶ resting spores and gently mixed at room temperature for 1 hour. Spores and bound phages were pelleted by centrifugation at 13 000 rpm for 1 min. The spore pellet was washed 10 times with 1 ml of washing buffer each time (50mM Tris pH 7.5, 150mM NaCl). Phages were eluted with 1 ml of 200 mM Glycine-HCl (pH 2.2), by incubating for 10 min at room temperature. The eluted phage was then amplified and taken through additional binding/amplification (panning) cycles to enrich the pool in favor of binding sequences. The wash buffer contained 0.1% of Tween 20 for the first round of panning and was increased to 0.5% for subsequent rounds. To neutralize the supernatant, and to avoid killing the phages, 150 µl of 1M Tris-HCl (pH 9.1) was added. Selected phages were amplified in *E. coli* ER2738 between each panning round. The cycle was repeated 3 times to select peptides with the highest affinity.

To eliminate phages that might not be specific to *P. brassicae* resting spores, two depleting rounds, without amplification between the two rounds, were carried out with a mix of ~10⁷ spores and mycelia fragments of common soil fungi (noted above).

Finally, a fourth round of panning was carried out with resting spores of *P. brassicae,* in the presence of washing buffer containing 0.5% Tween-20. Eluted phages were not amplified before plating. Twenty-seven clones were isolated and amplified, and the sequences of the selected peptides determined.

### Production, purification and characterisation of modified PapMV CPs

Four affinity peptides and a negative control peptide were selected and the corresponding DNA sequence was cloned at the C-terminus of CPΔN5 PapMV coat protein (see Example II) and the monomeric form CPΔN26 (Lecours et al., 2006, Protein Expression and Purification 47:273-280). Restriction sites for *Nco*I and *Bam*H1 at the 5' and 3' ends, respectively, were used to introduce the selected DNA sequences into the vector pET-3D for expression in *E. coli.* Clones were sequenced to verify that the peptides were in frame with the PapMV CP.

Modified PapMV CPs were expressed in *E. coli* BL21 RIL as described previously (Tremblay et al., 2006, FEBS J. 273:14-25; Lecours et al., 2006, ibid.). Imidazole was removed by extensive dialysis. Protein concentrations were measured using the Bradford assay following dilution in 6 M guanidium hydrochloride and 20 mM phosphate buffer, pH 6.5.

For fluorochrome labelling, purified proteins were dialysed to exchange the buffer for PBS (pH 7.4). Proteins were labelled as described in the protocol of Alexa Fluor 488 or Alexa Fluor 633 protein labelling kits (Molecular Probes). The degree of labelling was estimated by measuring absorbance of protein at 280 nm and that of fluorochromes at either 474 nm for Alexa 488, or 632 nm for Alexa 633.

To estimate the mean length of the VLPs produced, labelled proteins were diluted in 0.5% TBST, washed with buffer, and prepared grids (Cu 400 mesh carbon Formvar) were negatively stained with a filtered solution of 2% uranyl acetate. Every VLP (-50) found in a single electron microscopy image was measured.

### Binding of labelled proteins or anti-P. brassicae antibodies to P. brassicae and common soil fungi

Binding was analysed by flow cytometry by adapting the method used for selection of peptides. Essentially, 10⁶ (unless indicated otherwise) spores were first blocked for 1 hr with either 50 µg of CPΔN5 in 0.5% TBST, if testing PapMV CPs, or blocking buffer (0.1 M NaHCO₃, 5 mg/ml BSA) if antibodies were used. Ten µg of Alexalabelled proteins or 1 µl of rat anti-*P*. *brassicae* were added to spores and allowed to bind for 1 hr at room temperature with slight agitation. For antibodies, 5 washes with 1 ml of 0.5% TBST preceded a 1-hour incubation at room temperature with 5 µl PE goat anti-rat IgG (H+L) (Cedarlanes) in blocking solution. All labelled spores were finally washed 5 times with 1 ml of 0.5% TBST and spore pellets were resuspended in 500 µl of 0.5% TBST for analysis. Readings from 10 000 gated spores were collected using a flow cytometer.

Alexa 633-labelled *P. brassicae* resting spores were placed on slides for observation by confocal microscopy; 3x10⁵ spores in 0.5% TBST were centrifuged on slides at 2000 rpm for 10 min. Slides were dried for 30 min and PBS was added to keep the cover-slip in place. Slides were sealed, protected from light and incubated overnight at 4°C.

### Results

Phage display was used to select three peptides against *P. brassicae* resting spores and a negative control peptide, which did not bind to the spores. The sequences of the peptides are as follows:
Peptide A: DPAPRPR (SEQ ID NO:36)
Peptide B: LLNSHAV (SEQ ID NO:37)
Peptide C: NHAHSTP (SEQ ID NO:38)
Negative control peptide: KALGDNG (SEQ ID NO:39)

The selected peptides A, B and C were cloned at the C-terminus of PapMV CP. Two PapMV CP constructs were used as a platform: CPΔN5 (referred to here as "CP"), which was previously shown to self-assemble into VLPs in *E. coli* (Tremblay et al., 2006, *ibid.*) and the monomeric form CPΔN26 (referred to here as "CPm"), which cannot self assemble in bacteria and remains as a monomeric form (Lecours et al., 2006, *ibid*).

Nine different constructs were generated, all with a 6xHis tag at their C-terminus for ease of purification of the recombinant proteins. CP-Neg contains a fusion of the negative control peptide. CP-A, -B and -C correspond to CP constructs in fusion with each of the three peptides selected by phage display, respectively. CP-GlyA has an additional insertion of 3 Gly residues between the C-terminus of the CP and peptide A, CP-AA has a duplication of peptide A at the C-terminus and, finally, CPm-A is the monomeric form of CP in fusion with peptide A.

All constructs were expressed in *E. coli* and the proteins purified by affinity chromatography on a Ni²⁺ column. Up to 30-40 mg of purified protein could be obtained from 500 ml of culture media for all CP constructs. The production of CPm was less efficient but still yielded 5 mg/ml.

Formation of VLPs was monitored by electron microscopy. The recombinant proteins CP, CP-A, -B, -C, -Neg, -GlyA, and -AA self assembled into VLPs in *E. coli.* As predicted, constructs CPm and CPm-A remained as monomeric forms and did not generate VLPs. The length of 50 VLPs for each construct was measured and the average length for all the VLPs estimated at 70 nm. No significant differences could be observed between the VLPs assembled from the different constructs.

The specific binding of the different VLPs to *P. brassicae* resting spores was evaluated by flow cytometry. Labelling of the different recombinant proteins was performed under the same conditions to ensure uniformity of labelling and to allow direct comparison between the different VLPs. The results showed that the labelled CP VLPs alone bound non-specifically to the resting spores, but only slightly more efficiently than CP-Neg VLPs (Fig 20A compare the displacement of the curve to the right compared to the natural autofluorescence of the spores). However, CP-B and -C VLPs both showed improved avidity for resting spores, while the most significant displacement of the curve was seen with CP-A, which exhibited high avidity for resting spores (Fig. 20B).

Binding of CP and CP-A to resting spores was also visualized by confocal microscopy. Because of the innate fluorescence of the spores at the wavelength used for the cytometry assays, CP-A was labelled with a different dye that can be excited in the far red (Alexa 633) to decrease the background in this experiment; resting spores showed no background in the far red. Only very slight fluorescence was observed following incubation with CP VLPs, while spores were clearly decorated with labelled CP-A VLPs. This experiment confirmed that CP-A binds to the surface of the resting spores with improved efficiency compared to CP. Furthermore, this result shows that the specific target of CP-A NLPs is located over the entire surface of the resting spores.

The specificity for *P. brassicae* resting spores of polyclonal antibodies produced in rats with that of CP-peptide VLPs was compared. Resting spores of seven different fungi (*Acremonium* sp., *Alternaria alternata, Cladosporium* sp., *Fusarium solani, Plasmodiophora brassicae, Trichoderma* sp., *Trichotecium roseum, Yerticilium dahliae*) were tested. The polyclonal antibody raised against the fungal spores exhibited strong binding to *P. brassicae* resting spores, however, it also showed nonspecific binding to all spores tested, likely due to the recognition of common features on all the spores tested. In contrast, CP-A VLPs were very specific for *P. brassicae* resting spores out of all the spores tested. CP-B and CP-C VLPs were also very specific, but were bound with less avidity than CP-A VLPs.

To test if CP-A can detect *P. brassicae* resting spores in a mixture of heterologous spores, a flow cytometry analysis on 10⁶ resting spores of *P. brassicae* mixed with 6x10⁶ spores of other soil borne (*Acremonium* sp., *Alternaria alternata, Cladosporium* sp., *Fusarium solani, Plasmodiophora brassicae, Trichoderma* sp., *Trichotecium roseum, Verticilium dahliae*) was performed. When fluorescent CP-A was used for detection of *P. brassicae,* only one peak in was observed in the analysis, indicating very specific detection of *P. brassicae* resting spores in the mixture of heterologous spores.

### Multimerisation of peptides in VLPs is required for improved avidity

Flow cytometry analysis using labelled CPm-A and a CPm control (both based on the monomeric form of the PapMV CP) showed that the monomeric form cannot bind resting spores of *P. brassicae* efficiently. As expected, multimerisation of the peptide at the surface of the VLPs assembled by the CP construct is important to improve the avidity of the peptide (Fig. 20). Thus, as predicted, a monomeric form did not exhibit binding to the target.

### Improving the avidity of CP-A

To improve the freedom of movement of the affinity peptide A at the surface of the CP-A NLPs, and to decrease any steric hindrance caused by the C terminus of the PapMV CP, a stretch of 3 Gly residues was added between the C terminus of CP and affinity peptide A (CP-GlyA). In addition, the A peptide at the C-terminus of CP-A was duplicated to generate CP-AA. Both proteins were produced, purified and used in the flow cytometry binding assay as described above. Signals were compared to those of CP-A. Both CP-GlyA and CP-AA showed an improvement in avidity for resting spores (Fig. 21A-B). The duplication of the peptide A generated the VLPs with the highest avidity (Fig. 21B).

### EXAMPLE IX: Production and engineering of PapMV coat proteins fused to the M2e H1N1 influenza antigen

### PapMVCP and PapMVCP-M2e expression and purification

The PapMVCP-M2e construct was generated as described in Example IV. Expression and purification of the protein were performed as described in Example IV, except for the isolation of VLPs. VLPs were isolated by one of two methods.
*1. Ultracentrifugation:* VLPs were isolated following an ultracentrifugation step using a highly concentrated sucrose phase at the bottom of the tube. The pellet was then resuspended in sterile PBS. Pellet and supernatant (containing a mix of discs and less organized protein forms) were dialysed as described in Example IV.
*2. Macrosep*® *isolation:* The elution buffer containing a mixture of PapMVCP-M2e proteins (VLPs, discs and less organized forms) was concentrated with the aid of a Macrosep® centrifugal device with a 1000 kDa cut off (Pall Corporation, NY).

Each batch of proteins produced was checked systematically for purity by SDS-PAGE. The exact proportion of each PapMV form (VLPs, discs, dimeric, monomeric and degraded forms) was verified by passing the protein solution through a gel filtration column Superdex 200 as previously described (Tremblay et al., 2006, Febs J 273:14-25; Lecours et al., 2006, Protein Expression and Purification, 47:273-280). The column distinguishes High Molecular Weight Proteins (HMWP > 650 kDA) from Low Molecular Weight Proteins (LMWP) which include discs (400-600 kDA) as well as less organized forms (dimeric, monomeric and degraded forms). The compositions designated as PapMVCP and PapMVCP-M2e (prepared by ultracentrifugation) were comprised of 80 and 90% HMWP, respectively. In contrast, the non-ultracentrifuged PapMVCP-M2em composition, concentrated using only the Macrosep® centrifugal device, was composed of 45 % HMWP, 23 % discs and 21 % dimeric and monomeric forms. Western-blotting (Fig. 22A) was performed as described in Example II using polyclonal anti-PapMVCP antibodies. Once purified, protein concentrations were evaluated with a BCA protein kit (Pierce Biotechnology, Rockford, IL).

### Electron microscopy

Proteins were diluted in PBS and absorbed for 3 min on carbon-coated formvar grids. Grids were washed twice with deionized water, stained with 0.1% uranyl acetate for 10 min at room temperature and then observed on a JEOL JEM220FS transmission electron microscope. Average VLP length was determined as described in the previous Examples.

### Results

In this Example, the M2e peptide sequence from influenza virus strain H1N1 A/WSN/33 was fused to the C-terminus of the PapMVCP as shown in Fig. 12A. Purification of PapMVCP and PapMVCP-M2e included an ultracentrifugation step to pellet VLPs and retain smaller molecular weight forms (mainly discs, dimers and monomers) remain in the supernatant. The SDS-PAGE profiles of PapMVCP and PapMVCP-M2e point to high purity of the recombinant proteins, while also revealing degraded protein forms in the supernatant (Fig. 22A). As already noted (Tremblay et al., 2006, *ibid*.; Lecours et al., 2006, *ibid.*), the size of PapMVCP proteins as evaluated by SDS-PAGE (between 25 and 33 kDa) is larger than the size expected by *in silico* prediction (23.1 kDA for PapMVCP and 26.4 kDA for PapMVCP-M2e). Western-blotting (Fig. 22B), using an anti-PapMVCP polyclonal antibody, confirmed the presence of degraded PapMVCP proteins in the preparation. This suggests strongly that a significant portion of the heterogeneous protein mixture does not exhibit the whole M2e epitope, even if the main part of the Low Molecular Weight Proteins (LMWP) is composed of non-degraded discs (approximately 60% as determined by FPLC).

As expected, FPLC indicated a high proportion (90%) of High Molecular Weight Proteins (HMWP with a MW ≥ 650 kDA) in the pellet. As previously demonstrated, PapMVCP expressed in *E. coli* can efficiently self-assemble into VLPs, as numerous rods are seen by electron microscopy (Fig. 22B, left hand panel). Importantly, fusion of the M2e epitope at the C-terminus of the PapMVCP did not impair the ability of the PapMVCP fusion to self-assemble into VLPs as many rods were observed in the pellet after ultracentrifugation (Fig. 22B, right hand panel). No rods but only discs were detectable in the supernatant (Fig. 22B, centre panel). PapMVCP-M2e VLPs have an average length of 101 nm (± 5.6), similar to that for PapMV VLPs (Tremblay et al., 2006, *ibid.*).

To ease purification of PapMVCP-M2e VLPs, another method to enrich the protein extract containing VLPs was tested in which a Macrosep® centrifugal device with a 1000 kDa cut off was used to separate HMWP (the VLPs) from LMWP (discs and less organised forms of protein) rather than pelleting the VLPs by ultracentrifugation. This method was less efficient at enriching the quantity of PapMVCP-M2e HMWP in the purified proteins with a 45 % content of HMWP being observed rather than 80 % with ultracentrifugation. The presence of VLPs in the Macrosep® purified HMWP extract was verified by electron microscopy, and the isolated VLPs on average appeared shorter (78.8 nm ±3.9) than VLPs isolated by ultracentrifugation (p<0.01). This observation suggests that this new method selects a broader size range of VLPs than does ultracentrifugation.

### EXAMPLE X: Specific binding of IgG against PapMV VLPs comprising the M2e influenza antigen by influenza infected cells

### Immunisation of mice

Ten 6- to 8-week-old BALB/c mice (Charles River, Wilmington, MA) were injected subcutaneously with: i) 100 µg of PapMVCP; ii) 100 µg of PapMVCP and 10µg of M2e peptide (Fig. 2 only); iii) 100 µg of PapMVCP-M2e (VLPs or discs), or iv) endotoxin-free PBS (Sigma, St-Louis, MO). Primary immunization was followed by one booster dose of the same amounts given 2 weeks later. Blood samples were obtained at 8 days after the booster shot and stored at -20°C until analysis.

### ELISA quantification

The M2e peptide (SLLTEVETPIRNEWGCRCNDSSD [SEQ ID NO:6]) was synthesized by GLBiochem Shanghai LTD (Shanghai, China) and resuspended in endotoxin free PBS (Sigma, St-Louis, MO). Costar High Binding 96-well plates (eBioscience, San Diego, CA) were coated overnight at 4°C with 100 µl/well of the M2e peptide diluted to a concentration of 1 µg/ml in 0.1 M NaHCO₃ buffer, pH 9.6. Plates were blocked with PBS/0.1% Tween-20/2% BSA (150 µl/well) for 1 h at 37°C. After washing three times with PBS/0.1% Tween-20, sera were added in 2-fold serial dilutions beginning from 1:50 and incubated for 90 min at 37°C. Plates were then washed four times and incubated with 100 µl of peroxidase-conjugated goat anti-mouse IgG, IgG1, IgG2a, IgG2b (all from Jackson Immunoresearch, Baltimore, PA), IgG3 (Rockland, Gilbertsville, PA), IgGA (Serotec, Raleigh, NC) at a dilution of 1/10,000 (for all isotypes except IgA) or 1/2,000 (for IgA) in PBS/0.1% Tween-20/2% BSA for 1 h at 37°C. After four washes, IgG presence was detected with 100 µl of TMB-S according to the manufacturer's instructions. The reaction was stopped by adding 100 µl of 0.18 M H₂SO₄. The OD was read at 450nm. Results are expressed as an antibody endpoint titer, determined when the OD value is 3-fold greater than the background value obtained with a 1:50 dilution of serum from PBS injected mice.

### Binding capacity test of anti-M2 serum on MDCK cells

MDCK cells [1.5 × 10⁶ cells/well in Labtek II chambered cover glass (Nunc International); 4 × 10⁴ cells/well in a 96 well flat-bottom plate] were incubated for 24 h at 37 °C with 5% CO₂ to obtain a confluent cell monolayer. Media was removed and cells were infected with 1 multiplicity of infection (1 MOI) of A/WSN/33 Influenza virus (H1N1) diluted in MEM HEPES Albumin (0.5%) GVF. After 20 h of incubation, media was removed and a 1:40 dilution (in PBS) of a pool of serum from mice injected with PBS, PapMVCP or PapMVCP-M2e, was added to each well. Plates were incubated for 1h at 4°C, then rinsed 2× with PBS. Next, secondary anti-mouse IgG-Alexa 488 (1:100 dilution in PBS) labelled antibody was added to each well for fluorescent visualization of anti-M2 on cells infected with virus. Alternatively, a peroxidase-conjugated goat anti-mouse IgG (diluted 1:500 in PBS) was added. Plates were then incubated for 1 h at 4°C. After incubation, wells were rinsed 4x with PBS. Alexa 488 fluorescent staining was observed immediately under a NIKON Eclipse TE300 microscope with an FITC filter (488 nm). Images were analysed using Image J 1.37v. For the 96 flat-bottom plates, the protocol followed is as described previously for ELISA. All these experiments include the same treatments with non-infected cells to ensure binding specificity.

### Influenza A strain

The influenza A strain used was the A/WSN/33 influenza strain (H1N1), which was derived from a mouse lung-adapted clinical isolate, A/WS/33, obtained by serial passage in neonatal mice and then to brains of adult mice (Stuart-Harris, 1939, Lancet 1:497-499). The LD₅₀ of this strain was previously evaluated as being approximately 10³ plaque forming units (pfu) (Abed et al., 2006, Antivir Ther 11:971-976). In this Example, a low dose of influenza infection corresponds to 1 LD₅₀ and a medium dose of infection corresponds to 4 LD₅₀.

### Virus challenge experiments

Mice were infected intranasally with 35 µl containing 1x10³ (1 LD₅₀) of influenza virus strain A/WSN/33. Mice were monitored daily for clinical signs (loss of body weight, rectal temperature, abnormal behaviour and ruffled fur). Deaths were recorded over a period of 16 days.

### Statistical analysis

Student's, Tukey's and Dunn's tests compared differences (antibody titers, temperature, weight, viral titers in lungs) among groups of mice. Differences among survival curves were analysed by Kaplan-Meier survival analysis. Values of p<0.05 (*), p<0.01 (**), p<0.0001 (***) were considered statistically significant. Statistical analyses were performed with PRISM 3.03.

### Results

To evaluate whether genetic fusion of M2e to PapMVCP facilitates induction of an immune response against this peptide, BALB/c mice were immunized twice at 2-week intervals with 100µg of PapMVCP-M2e, PapMVCP VLPs (without a fused epitope) or PapMVCP VLPs + unfused synthetic M2e peptide. The results are shown in Fig. 23A and indicate that only PapMV-M2e VLPs can trigger a humoral response against M2e and induce class switching to IgG2a, which demonstrates that genetic fusion of the M2e peptide to the VLPs renders the peptide immunogenic. ELISA and confocal microscopy were conducted to determine whether the antibodies induced by vaccination with PapMVCP-M2e VLPs can recognize the M2 protein exhibited by influenza infected cells. Results obtained from both ELISA (Fig. 23B) and confocal microscopy confirmed that these anti-M2e antibodies recognize physiological M2e. Moreover, serum control cases (from mice injected with either PBS or PapMVCP VLPs) did not bind efficiently to either infected or non-infected cells (Fig. 23B).

The comparative immunogenicity of both forms (VLPs and discs) of PapMVCP-M2e protein expressed in *E. coli* was tested. The anti-M2e antibody response in mice injected with either of the two forms was measured after the boost and prior to challenge with H1N1 influenza strain (A/WSN/33). The results are shown in Fig. 24 and demonstrate that, firstly, PapMVCP-M2e VLPs are able to trigger an IgG anti-M2e response which is higher (p<0.01) than the immune response triggered by discs (Fig. 24A) and secondly, VLPs induced an IgG2a class switch while discs did not (Fig. 24A). However, although PapMVCP-M2e VLPs definitively trigger an anti-M2e response, no visible benefits after an influenza challenge were observed for this dosage of VLPs, as evidenced by the lack of significant difference in body weight (Fig. 24B) and in survival rates among the groups.

### EXAMPLE XI: Immunization of mice with PapMV VLPs or discs comprising the M2e influenza antigen: effect of dosing regimen, adjuvantation and opsonisation

### Immunization of mice

Ten 6- to 8-week-old BALB/c mice (Charles River) were injected subcutaneously with either endotoxin-free PBS (Sigma), 100 µg of PapMVCP, or 100 µg of a different form of PapMVCP-M2e (alone, opsonised or adjuvanted) in a 100 µl final injection volume per mouse. Primary immunization was followed by two booster doses of the same amounts given at 2-week intervals after the initial dose.

For PapMVCP-M2e opsonisation, monoclonal IgG2a antibodies (produced in the inventor's facilities) directed against PapMVCP or polyclonal antibodies (from mice injected 2 times with PapMVCP-M2e VLPs) were incubated with PapMVCP-M2e at 37°C for 45 min respecting a 1:10 vol:vol ratio of serum:PapMVCP-M2e. Then, the preparation was diluted in endotoxin-free PBS (Sigma, St-Louis, MO) prior to injection. Preparations containing the same amount of antibodies without PapMVCP-M2e protein served as negative control vaccines.

For adjuvantation, 100µg of PapMVCP-M2e was mixed with either Imject Alum (Pierce, Rockford, IL) following the manufacturer's instructions, or with 100µg of PapMVCP.

For experiments utilising the (non-ultracentrifuged) PapMVCP-M2em, the dose injected corresponded to 80µg of HMWP found in the 100µg of protein used with the ultracentrifuged PamMV-M2e. Since the PapMV-M2em protein contains 45% of HMWP, we injected 200µg of total protein mixture, which correspond approximatly to 80µg of HMWP.

For experiments utilising the (non-ultracentrifuged) PapMVCP-M2em, the dose injected was 200µg of total protein mixture, which corresponded approximately to 80µg of HMWP (as the PapMV-M2em contains approximately 45% of HMWP). This amount corresponded to the 80µg of HMWP found in the 100µg of ultracentrifuged PamMV-M2e. As control (PBS, PapMVCP) and PapMVCP-M2e vaccinated mice are common to experiments depicted in Figs. 25, 26, 27 and 28, data concerning these groups is the same in these four figures. All experimental protocols were approved by the Laval University Animal Protection Committee.

### ELISA quantification

ELISA was conducted as described in Example X.

### Virus challenge experiments

Mice were infected intranasally with influenza virus strain A/WSN/33 as described in Example X, except that 4x10³ pfu (4 LD₅₀) was used for the challenge. Mice were monitored daily for clinical signs (loss of body weight, rectal temperature, abnormal behaviour and ruffled fur). Deaths were recorded over a period of 16 days. For determination of pulmonary anti-M2 antibody levels and viral lung titer at the same time point, groups of 5 mice by condition were sacrificed 7 days post-inoculation. Bronchoalveolar lavage (BAL) was performed with 3 x 1ml of sterile PBS. Lungs were removed aseptically and stored at -80°C in 1 ml of sterile PBS. Later, lungs were homogenized and centrifuged at 2500 rpm/ 4°C for 10 min and supernatants were titrated in MDBK cells using a standard plaque assay as described previously (Abed et al., 2005, Antimicrob Agents Chemother 49:556-559).

### Statistical analysis

Statistical analysis were performed as described in Example X.

### Results

In order to increase the IgG2a anti-M2e response triggered by the PapMVCP-M2e construct, 3 different strategies were implemented: (1) increasing the number of booster injections to two, (2) adding an adjuvant (alum or PapMVCP VLPs) to the preparation, and (3) opsonisation with anti-PapMVCP or anti-PapMVCP-M2e antibodies.

Anti-M2 antibodies generated in response to a dosing regimen that included two booster injections, and in two groups of animals in response to preparations comprising either alum or PapMV VLPs as adjuvant, were isotyped to assess whether there were any differences in isotype profiles among the different formulations (Fig. 25A). PapMVCP-M2e VLPs were shown to trigger a balanced Th1/Th2 antibody response against the fused epitope, as previously demonstrated with a different epitope (see Example VI). In addition, the second boost notably increased IgG2a anti-M2e levels as compared with the prime-single boost experiment described in Example X. The adjuvants were observed to improve the humoral response very differently. Alum drove an expected Th2 humoral response and slightly increased induction of IgG1 anti-M2, whereas PapMVCP VLPs increased, by a factor of four, the level of IgG2a anti-M2 compared with the groups vaccinated with PapMVCP-M2e alone. When infected with four times the lethal dose₅₀ (4LD₅₀) of influenza virus, the mice vaccinated with preparations comprising an adjuvant all showed reduced symptoms of morbidity, with better regulation of body temperature (Fig. 25B) and more stable total body weight (Fig. 25C). These results were most pronounced for mice vaccinated with adjuvanted PapMVCP-M2e VLPs, with the preparations comprising PapMV CP as an adjuvant showing a slightly better improvement than preparations comprising alum. It is noteworthy that in these experiments, alum induced granulomas at the injection site in the mice. In contrast, adjuvantation with PapMVCP VLPs did not generate any obvious local toxic effect.

During the course of infection, survival rate for half (5/10) of the mice in each group was analysed. The remaining mice (5/10) were sacrificed to determine the viral load in the lungs and the anti-M2 response in bronchoalveolar lavages. Use of either adjuvant increased survival rate (4/5 with alum and 5/5 with PapMVCP VLPs versus 2/5 without adjuvant, see Fig. 25D). These results were confirmed by the viral load measured in the lungs, with mice vaccinated with adjuvanted VLPs showing reduced lung viral titers to those vaccinated with non-adjuvanted VLPs, when compared to the controls (Fig. 25E). Anti-M2e antibodies were detected in the bronchoalveolar lavages of all PapMV-M2 vaccinated mice, with increased levels of IgG2a anti-M2e induced by PapMVCP-M2e VLPs adjuvanted with PapMVCP VLPs (Fig. 25F).

Overall, the above results show that the PapMV platform can trigger a humoral protective response to influenza, and that both increased dose of PapMVCP-M2e VLPs and the use of adjuvants can improve protection against influenza challenge. In this regard, it is worth noting that two injections of PapMVCP fused to the HCV E2 epitope (see Example VI) were sufficient to trigger a significant IgG2a anti-E2 (1/3 200) antibody level. This suggests that the nature of the fused epitope influences to some extent the global immunogenicity directed to the fused epitope and that the fusion of a different influenza antigen to PapMV coat protein may thus require a lower number of doses in order to trigger a sufficiently protective response.

In an additional experiment, either IgG2a monoclonal anti-PapMVCP (mAb) antibodies or mouse polyclonal anti-PapMVCP-M2e (pAb) antibodies were used to form immune complexes (ICs) *in vitro.* The ICs were named IC [mAb/PapMVCP-M2e] and IC [pAb/PapMVCP-M2e], respectively. Mice immunized with ICs did not show significantly increased levels of anti-M2e antibodies compared with mice immunized with PapMV-M2 VLPs, for all isotypes considered (Fig. 26A). During the course of influenza infection, mice immunized with ICs showed similar morbidity symptoms (temperature, level of body weight loss) as mice vaccinated with PapMVCP-M2e VLPs alone (Figs. 26B and C), and only slight improvements compared with control groups of mice injected with: PBS, PapMVCP VLPs, monoclonal anti-PapMVCP, or polyclonal anti-PapMVCP-M2e. These trends were confirmed fully by survival rates (Fig. 26D) and lung viral load (Fig. 26E) which both demonstrated an absence of benefit for opsonized PapMVCP-M2e VLPs. In addition, the IgG2a antibody (anti-M2) levels detected in bronchoalveolar lavages were slightly higher in mice immunized with IC [mAb/PapMVCP-M2e] compared with other PapMVCP-M2e vaccinated groups (Fig. 26F).

A further experiment was performed in order to determine the efficacy of the preparation of PapMV-M2e VLPs purified by a Macrosep® device (PapMVCP-M2em), which contained 45% macro-particles ≥600 kDa together with 55% discs (see example IX). In determining the amount of PapMVCP-M2em to use for the immunization, only the amount of HMWP in the preparation was considered. As 100µg of the ultracentrifuged PapMVCP-M2e proteins was shown to contain 80µg of HMWP as determined by gel filtration, mice were injected with 200µg of PapMVCP-M2em, which corresponded to a content of 80µg of HMWP. To test the immunogenicity of this preparation, 10 mice were injected three times with: PBS, 100µg of PapMVCP VLPs or 200µg PapMVCP-M2em and then infected with a low dose of influenza (1LD₅₀). The level of IgG2a anti-M2e antibodies was dramatically decreased in mice immunized with PapMVCP-M2em (p<0.0001) (<1/200 IgG2a, Fig. 27A) compared to the levels of IgG2a antibodies generated after three injections of ultracentrifuged PapMVCP-M2e VLPs (1/8000 IgG2a, Fig. 27A). Mice vaccinated with PapMVCP-M2em also showed worsened morbidity symptoms compared with control groups (Figs. 27B and C) and had an insignificant (p=0.19) increase in survival (Fig. 27D).

The effect of adjuvantation and opsonization of PapMVCP-M2em were tested. For all IgG isotypes, the anti-M2e antibody titers were increased from 30-fold for IgG1 to 80-fold for IgG2a (Fig. 28A), with a Th2 antibody profile (IgG1) using alum as an adjuvant, and a Th1 antibody profile (IgG2a and IgG2b) using PapMVCP VLPs as an adjuvant. Adjuvantation of the PapMVCP-M2em mix with either alum or PapMVCP VLPs also dramatically improved the control of internal body temperature (Fig. 28B), body weight (Fig. 28C) and survival rate (p<0.0001) from 1/10 to 8/10 and 9/10 respectively (Fig. 28D) after a medium dose influenza challenge.

Opsonization of PapMVCP-M2em mix with mAb or pAb improved the immunogenicity of the vaccine (Fig. 29A) but with a less impressive magnitude than adjuvantation. Anti-M2 IgG1 and IgG2a antibody levels were increased 20-fold for IgG1 to 50-fold for IgG2a. Opsonization with mAb or pAb did significantly increase the control of internal body temperature (Fig. 29B), body weight (Fig. 29C) and survival rates (p<0.001, from 1/10 to 6/10 and 7/10 respectively, Fig. 29D) after a medium dose influenza challenge. These results suggest that opsonization may be a good option for weakly immunogenic ACSs.

### EXAMPLE XII: Assessment of Immunogenicity of PapMV VLPs comprising the M2e influenza antigen in Ferrets

### PapMV VLPs

The PapMVCP-M2e construct described in Example IX was used for all experiments.

### Ferrets and Immunization Schedule

Male ferrets 16 weeks or older were used for all experiments. The animals were vaccinated against rabies, and free of ELISA-detectable antibodies against canine distemper and influenza A subtypes H1N1 and H2N3. At the day of the first immunization, a serum sample was collected, which served as prebleed. Two consecutive experiments were performed. For the first experiment, four animals were injected subcutaneously with 25 µg of PapMV-M2e VLPs. A second and third injection at the same concentration were performed at three-week intervals. Vaccinated animals, as well as two non-vaccinated control ferrets, were challenged three weeks after the last boost by intranasal inoculation of 10⁵ tissue culture infectious doses of the vaccine reassortant PR8, which carries the hemagglutinin and neuraminidase glycoproteins of the influenza virus strain H1N1 New Caledonia/99. Following infection, body temperature was determined daily for seven days, and body weight was measured on days two, four, and either six or seven. Serum samples were collected at days three and seven, and antibody titers were determined by ELISA using either infected cells or the M2e peptide as antigen.

The overall study design of the second experiment was similar to the first with the following exceptions: the PapMV-M2e dose was increased 10-fold for the last boost (*i.e.* 250 µg), the influenza strain H1N1 USSR/77 was used as challenge virus, the PapMV ELISA was performed using limited dilution, rather than OD at a dilution of 1:50, and nasal wash titers were measured at the same days as the clinical parameters.

### Results

At the time of challenge, three of the four vaccinated animals in the first group had developed antibodies that recognized the M2e peptide as well as virus-infected cells (Fig. 30A and B). These antibodies increased further within seven days after infection. The control animals reached similar titers against virus-infected cells three days after infection and then followed the kinetics of the vaccinated animals. However, they failed to develop antibodies that cross-reacted with the M2e peptide. Aside from a slightly higher temperature in animals without antibodies on the first day after infection, no discernable differences in clinical signs were observed between vaccinated and control animals (Fig. 30C and D).

In the second group, the amount of PapMV-M2e VLPs injected for the last boost was increased by 10-fold. At the time of challenge, three weeks after the last boost, all vaccinated animals had developed antibodies against the peptides that cross-reacted with virus-infected cells (Fig. 31A and B). Due to changes in the execution of the peptide ELISA from OD to limited dilution, these values cannot be compared directly, however, similar levels of antibodies recognizing virus-infected cells were detected for both vaccination regimens (compare Fig. 30A and 31A). There was no difference between vaccinated and control animals in respect to body temperature and weight over the course of the challenge infection. Two of the vaccinated animals experienced a slightly reduced and shortened period of virus shedding in the nasal wash fluid, while the other two animals followed the kinetics of the control ferrets. Interestingly, the vac2 individual that had the lowest antibody titer against the peptide fared best in the challenge (Fig. 31, black squares). In contrast, vac3, which had exhausted its cross-reactive antibody supply by day 3, experienced the most severe course of disease (Fig. 31, black circles).

This study was designed as a pilot study with the dual aim of assessing the potential of the PapMV-M2e VLPs as vaccine candidates and establishing a ferret animal model. The results demonstrate that the subcutaneous injection of PapMV-M2e elicits a humoral immune response in ferrets, which also recognizes virus-infected cells. The disease caused by the challenge viruses used, however, was too mild to clearly separate vaccinated and control animals. Additional studies that include a group vaccinated with a licensed vaccine as gold standard and use a more virulent challenge virus are anticipated to confirm the ability of the PapMV-M2e VLPs to provide a protective effect in ferrets.

Additional studies conducted using the above H1N1 construct, as well as a construct comprising the H9N2 M2e peptide (see Example XIII), showed that after an initial injection followed by one booster injection, the treated ferrets had already raised antibodies against the respective VLPs, and antibodies against the M2e peptides are anticipated to be detected after a second booster injection. In these experiments, ferrets were also injected with formulations comprising each construct in combination with either PapMV or PapMV VLPs as adjuvant. For the H1N1 constructs, the effect of PapMV as an adjuvant was slightly more pronounced that that of the PapMV VLPs.

### EXAMPLE XIII: Development of an ACS comprising the M2e epitope from the Influenza A H9N2 strain

### Cloning and preparation of H9N2 PapMV-M2e virus like particles (VLPs)

PapMV coat protein comprising the M2e peptide from the H9N2 influenza strain fused at the C-terminus of the protein was prepared as described in Example IX but using the following oligonucleotides to create the M2e coding sequence:
Oligo H9N2 forward:
Oligo H9N2 reverse:

The amino acid sequence of the final construct (H9N2 PapMV-M2e) is shown in Fig. 1G [SEQ ID NO:47]. Another version of the construct was also prepared that included a glycine-leucine spacer (GGGLLLTS) between the PapMV CP and the M2e peptide was also constructed. In brief, the DNA of the PapMV-M2e clone was linearised with SpeI. The oligonucleotides shown below [SEQ ID NOs: 52 & 53] were annealed and inserted into the SpeI site between the 6xH tag and the C-terminus of the PapMV CP.

Oligo H9N2 spe1-GLY forward:
5'-CTAGTGGTGGCGGTCTGCTGCTGA-3' [SEQ ID NO: 52]

Oligo H9N2 spe1-GLY reverse:
5'-CTAGTCAGCAGCAGACCGCCACCA-3' [SEQ ID NO: 53]

The amino acid sequence of the final construct comprising the spacer (H9N2 PapMV-3GLM2e) is shown in Fig. 1H [SEQ ID NO:48].

The resulting constructs were expressed in *E.coli* as described in Example IX and the H9N2 PapMV-M2e and H9N2 PapMV-3GLM2e proteins were purified by ultracentrifugation as described in Example IX.

### SDS-PAGE, FPLC and Electron Microscopy

Analysis of the expressed proteins was conducted as described in Example IX.

### Results

The C-terminal sequences of PapMV coat protein, H9N2 PapMV-M2e and H9N2 PapMV-3GLM2e are shown in Fig. 32A. The SDS-PAGE profiles of PapMV, H9N2 PapMV-M2e and H9N2 PapMV-3GLM2e show the high purity of the recombinant proteins (Fig. 32B). As expected, the FPLC profiles showed a unique peak that corresponded to VLPs for both proteins and indicated a high proportion of VLPs (90% VLPs, with a MW ≥ 650 kDA) in the pellet for the H9N2 PapMV-3GLM2e and a similar result for the H9N2 PapMV-M2e VLPs (80% VLPs) (Fig. 32C). As already demonstrated, PapMVCP expressed in *E. coli* can efficiently self-assemble into VLPs, as numerous rods are seen by electron microscopy (Fig. 32D, left-hand panel). Importantly, fusion of the H9N2 M2e epitope at the C-terminus of the PapMVCP did not impair the ability of the protein to self-assemble in VLPs as many rods were observed in the pellet after ultracentrifugation (Fig. 32D centre panel (H9N2 PapMV-3GLM2e) and right-hand panel (H9N2 PapMV-M2e)).

### EXAMPLE XIV: Immunogenicity of an ACS comprising the M2e epitope from the Influenza A H9N2 strain

### Immunogenicity of H9N2 PapMV-M2e VLPs in Balb/C mice

Groups of 10 mice were injected subcutaneously three times with 100µg of purified H9N2 PapMV-M2e VLPs, H9N2 PapMV-M2e VLPs adjuvanted with an equal volume of alum, or H9N2 PapMV-M2e VLPs adjuvanted with an additional 100µg of PapMV VLPs. Separate groups of mice were also injected with total protein, *i.e.* the total purified protein isolated from *E. coli* without ultracentrifugation and containing at least 20% discs. The final volume injected in the mice in all cases was 500 µl.

ELISA performed with the antisera that were collected after the three injections revealed that the H9N2 PapMV-M2e VLPs were immunogenic in mice. No advantage in using PapMV VLPs or alum as an adjuvant was observed in this instance. A variation in the immune response between the mice was observed (Fig. 33A), which is likely due to the fact that the repertoire of mice does not include memory B cells that can recognise the H9N2 M2e peptide efficiently. This result is rather intriguing because it has been demonstrated that the H1N1 M2e peptide fused to the surface of PapMV VLPs was immunogenic in all the treated Balb/C mice (see Example XI) and the H1N1 M2e peptide harbours only 3 amino acid differences compared to the H9N2 peptide.

The levels of IgG directed toward the H9N2 PapMV-M2e VLPs were measured as described in the previous Examples to verify that the VLPs were immunogenic. As expected, very high titers of IgG toward the H9N2 PapMV VLPs (*i.e.* the vaccine platform) were detected, even though the treated mice did not react efficiently to the H9N2 M2e peptide (Fig. 33B). This result confirms that the H9N2 PapMV-M2e VLPs are immunogenic but, because of the limited immune repertoire of the Balb/C mice, not all the individual mice had the capacity to develop an immune response to the H9N2 M2e peptide.

It is likely, however, that the H9N2 PapMV-M2e VLPs will be immunogenic in chickens, the natural host for H9N2 strains of influenza, because the immune system of birds has evolved to maintain their repertoire of B cells towards influenza virus.

### H9N2 PapMV-M2e VLPs induce a long lasting memory response

The sera of two mice that produced high levels of antibodies toward the H9N2 M2e peptide and that were kept 140 days after the third injection were analysed. The antibody response was seen to be maintained during this entire period indicating that the H9N2 PapMV-M2e VLPs are able to induce a memory response in mice efficiently (Fig. 34A). Furthermore, these mice were also shown to produce prolonged and abundant amounts of IgG1 (Fig. 34B) and IgG2a (Fig. 34C) isotypes. This result indicates that the H9N2 PapMV-M2e VLPs can induce the class switch of B cells toward different isotypes efficiently and suggests a balanced Th1/Th2 response in the mice.

### Cross-reactivity of the H9N2-M2e antiserum with other M2e peptides

Antisera obtained from mice that reacted strongly toward the H9N2 PapMV-M2e VLPs was analysed by ELISA to determine whether the sequences of other M2e peptides were recognised. Five different M2e peptides, including the H9N2 peptide, were tested (see Table 3).

**Table 3: M2e Peptides Tested for Cross-Reactivity**

| **Peptide Sequence*** | **Influenza Strain** | **SEQ ID NO** |
|---|---|---|
| SLLTEVETPIRNEWGCRCNDSSD | Human H1N1 A/USRR/90/77 | 6 |
| HSLLTEVETPTRNEWECRCSDSSD | Avian H5N1 A/Vietnam/1196/04 | 41 |
| MSLLTEVETPTRNGWECKCSDSSD | H3N8, Horse-Dog A/equine/Massachussetts/213/2003 | 42 |
| MSLLTEVETPTRNGWGCRCSDSSD | H9N2, A/chicken/Osaka/aq69/2001(H9N2) | 43 |
| SLLTEVETPTRNEWGCRCSDSSD | Mutant H1N1 I/T | 62 |

| | | |
|---|---|---|
| * The differences between the sequences shown and the reference H1N1 M2e peptide [SEQ ID NO:6] are underlined. | | |

The ELISA results demonstrated that the H9N2 sera reacted most strongly with the H9N2 peptide, as expected, followed by the H3N8 peptide and weakly with the H5N1, the H1N1 and the mutant I/T peptides (Fig. 35). This result suggests that immunisation of animals with the H9N2 PapMV-M2e VLPs could lead to protection against infection with both H9N2 and H3N8 strains of influenza.

### EXAMPLE XV: Development of an ACS comprising the M2e epitope from the Influenza A H3N8 strain

### Cloning and preparation of H3N8 PapMV-M2e virus like particles (VLPs)

PapMV coat protein comprising the M2e peptide from the H3N8 influenza strain fused at the C-terminus of the protein was prepared as described in Example IX but using the following oligonucleotides to create the M2e coding sequence:
Oligo M2-H3N8 forward:
Oligo M2-H3N8 reverse:

The nucleotide sequence of the final construct is shown in Fig. 1I [SEQ ID NO:56], and the encoded amino acid sequence is shown in Fig. 1J [SEQ ID NO:57].

The resulting constructs were expressed in *E.coli* as described in Example IX and the H3N8 PapMV-M2e protein was purified by ultracentrifugation as described in Example IX.

### SDS-PAGE, FPLC and Electron Microscopy

Analysis of the expressed protein was conducted as described in Example IX.

### Results

The C-terminal sequences of PapMV coat protein and H3N8 PapMV-M2e are shown in Fig. 36A. The SDS-PAGE profiles of PapMV and H3N8 PapMV-M2e show the high purity of the recombinant proteins (Fig. 36B). As expected, the FPLC profiles of the ultracentrifuged proteins resuspended in PBS showed a unique peak that corresponded to VLPs for both proteins (Fig. 36C). The H3N8 PapMV-M2e VLP peak was estimated to be composed of 92% of VLPs. Fusion of the H3N8 M2e epitope at the C-terminus of the PapMVCP did not impair the ability of the protein to self-assemble in VLPs as many rods were observed in the pellet after ultracentrifugation (Fig. 36D; right-hand panel).

### EXAMPLE XVI: Development of an ACS comprising the M2e epitope from the Influenza A H5N1 strain

### Cloning and preparation of H5N1 PapMV-M2e virus like particles (VLPs)

PapMV coat protein comprising the M2e peptide from the H5N1 influenza strain fused at the C-terminus of the protein was prepared as described in Example IX but using the following oligonucleotides to create the M2e coding sequence:
Oligo M2-H5N1 forward:
Oligo M2-H5N1 reverse:

The nucleotide sequence of the final construct is shown in Fig. 1K [SEQ ID NO:58], and the encoded amino acid sequence is shown in Fig. 1L [SEQ ID NO:59].

The resulting constructs were expressed in *E.coli* as described in Example IX and the H5N1 PapMV-M2e protein was purified by ultracentrifugation as described in Example IX.

### SDS-PAGE and Electron Microscopy

Analysis of the expressed protein was conducted as described in Example IX.

### Results

The C-terminal sequences of PapMV coat protein and H5N1 PapMV-M2e are shown in Fig. 37A. The SDS-PAGE profiles of PapMV and H5N1 PapMV-M2e show the high purity of the recombinant proteins (Fig. 37B). Fusion of the H5N1 M2e epitope at the C-terminus of the PapMVCP did not impair the ability of the protein to self-assemble into VLPs as many rods were observed in the pellet after ultracentrifugation (Fig. 37C; right-hand panel).

### EXAMPLE XVII: Preparation of the influenza NP protein

The coding sequence of the NP protein from the influenza virus strain A/WSN/33 (see Fig. 41 A; SEQ ID NO:63) was PCR-amplified using the following primers:
Forward: 5'-GACTCC ATG GCG ACC AAA GGC ACC AAA CGA-3' [SEQ ID NO:65]
Reverse: 5'-GATC CTC GAG TTA GTG GTG GTG GTG GTG GTG ATT GTC GTA CTC CTC-3' [SEQ ID NO:66]

The amplified sequence was cloned into the pET-24d(+) vector (Novagen) linearised with NcoI and XhoI. The amino acid sequence of the NP protein is provided in Fig. 41B [SEQ ID NO:64]. The encoded sequence included a 6xHis tag to facilitate purification.

The recombinant NP protein was prepared from *E. coli* BL21 (DE3) RIL (Stratagene) transformed with the pET-24d(+) plasmid containing the NP coding sequence (described above). Cells were grown in 2 X YT containing 30 µg/mL kanamycin. Protein expression was induced with 1 mM IPTG when the culture reached an OD₆₀₀ of 0.6. The culture was incubated for a further 16h at 22°C after induction and then cells were collected by centrifugation.

Cells were lysed using a French Press and the His-tagged NP protein was isolated using Ni-NTA beads (Qiagen) and an elution buffer containing 50 mM NaH₂PO₄, 300 mM NaCl, 500 mM imidazole (pH8.0). The eluted protein was dialysed extensively against 10 mM NaH₂PO₄, pH7.0 to remove the imidazole. Finally, the NP protein was purified using a HiTrap™ Heparin HP column (GE Healthcare) and a step gradient of increasing concentrations of NaCl (up to 2 M) in 10 mM NaH₂PO₄.

### EXAMPLE XVIII: Adjuvantation of a commercial influenza vaccine with PapMV improves immunogenicity

### PapMV

Wild-type PapMV was isolated from plants as described in Example I.

### Immunisation of Mice

Two groups of 5 mice were immunized subcutaneously once with either 9 µg of the commercial Fluviral® vaccine (GlaxoSmithKline) in a final volume of 350 µl (group 1) or with 9 µg of the Fluviral® vaccine adjuvanted with 100 µg of wild-type PapMV virus. Blood samples were collected on days 5, 10 and 14 after immunisation.

### ELISA

ELISA analysis was performed using the protocol outlined in the previous Examples but using plates coated with either the Fluviral® vaccine or recombinant NP protein (produced in *E. coli* and purified as described in Example XVII). For the determination of the amount of IgG2a against the NP protein (as shown in Fig, 40B), the coating of the plates with the NP protein was performed at 37°C for 2 hours rather than at 4°C overnight.

### Results

ELISAs performed to determine total IgG in the sera show that no IgG directed against the Fluviral® vaccine could be detected 5 days after immunisation (Fig 38A). By day 10, however, it can be seen that adjuvantation with PapMV improved the IgG titers by between one and two logs, corresponding to a factor of 2-4 X, over the IgG titers obtained with the Fluviral® vaccine alone (Fig. 38B) and that this increase was maintained or slightly improved at day 14 (Fig. 38C).

The respective amounts of IgG2a and the IgG1 antibody isotypes in the sera against the Fluviral® vaccine were also analysed (Fig. 39). The sera of the mice treated with the Fluviral® vaccine adjuvanted with PapMV showed approximately 32 times higher amounts of IgG2a (Fig. 39A), suggesting that the PapMV adjuvant is driving the immune response toward a TH1 response. As such, it is expected that a cellular response will be triggered toward the influenza epitope contained in the Fluviral® vaccine.

The immune response of the mice (total IgG) toward the NP protein was also analysed by ELISA. The NP protein is one component of the influenza virus and is thus contained in the Fluviral® vaccine. The sera of mice treated with the Fluviral® vaccine adjuvanted with PapMV can be seen to show an increased amount of IgG towards the NP protein (Fig. 40A). Analysis of the amount of IgG2a against the NP protein in the sera of the vaccinated mice shows that the sera of mice treated with the Fluvial® vaccine adjuvanted with PapMV contained higher amounts of IgG2a aginst the NP protein (Fig. 40B), again demonstrating that the PapMV adjuvant is driving the immune response toward a TH1 response. The relatively low titers measured in this instance are the result of using an accelerated coating protocol for the ELISA plates, however, the results clearly show an increase in measured IgG2a in the sera of mice treated with the adjuvanted Fluviral® vaccine.

## Claims

1. An antigen-presenting system comprising one or more influenza virus antigens in combination with a papaya mosaic virus (PapMV) or a VLP comprising PapMV coat protein, wherein said one or more influenza virus antigens are not conjugated to said PapMV or VLP.

2. The antigen-presenting system of claim 1, wherein said VLP comprises modified PapMV coat protein, said modified PapMV coat protein being capable of self-assembly to form said VLP.

3. The antigen-presenting system of claim 1 or 2, wherein said one or more influenza virus antigens are comprised by a commercially available influenza vaccine preparation.

4. The antigen-presenting system of any one of claims 1, 2 or 3, wherein at least one of said influenza virus antigens is derived from the M2 protein.

5. The antigen-presenting system of any one of claims 1, 2 or 3, wherein at least one of said influenza virus antigens is derived from the nucleoprotein (NP).

6. The antigen-presenting system of any one of claims 1, 2, 3, 4 or 5, wherein said antigen-presenting system comprises a VLP, said VLP comprising PapMV coat protein having an amino acid sequence substantially identical to the sequence as set forth in any one of SEQ ID NOs:1, 3 or 46.

7. An influenza vaccine composition comprising one or more of the antigen-presenting systems of any one of claims 1, 2, 3, 4, 5 or 6.

8. An antigen-presenting system according to any one of claims 1, 2, 3, 4, 5 or 6 for use in inducing an immune response against an influenza virus in an animal.

9. The antigen-presenting system for use according to claim 8, wherein said immune response comprises a humoral immune response.

10. The antigen-presenting system for use according to claim 8 or 9, wherein said immune response comprises a cellular immune response.

11. The antigen-presenting system for use according to any one of claims 8, 9 or 10, wherein said animal is a human.

12. The antigen-presenting system for use according to any one of claims 8, 9 or 10, wherein said animal is a non-human animal.

13. A composition comprising papaya mosaic virus (PapMV) or a VLP comprising PapMV coat protein for use to improve the efficacy of a commercially available influenza vaccine in inducing an immune response in an animal against an influenza virus, wherein said composition and said influenza vaccine are administered concomitantly to said animal.

14. The composition for use according to claim 13, wherein said immune response comprises a response against NP protein.

15. The composition for use according to claim 13 or 14, wherein said immune response is effective against more than one strain of influenza virus.

16. The composition for use according to any one of claims 13, 14 or 15, wherein said composition comprises a VLP comprising PapMV coat protein.

17. The composition for use according to claim 16, wherein said VLP comprises modified PapMV coat protein, said modified PapMV coat protein being capable of self-assembly to form said VLP.

18. The composition for use according to claim 16 or 17, wherein said VLP comprises PapMV coat protein having an amino acid sequence substantially identical to the sequence as set forth in any one of SEQ ID NOs:1, 3 or 46.

19. A composition comprising:
one or more influenza virus antigens in combination with a papaya mosaic virus (PapMV) or a VLP comprising PapMV coat protein, and
an adjuvant,
wherein said one or more influenza virus antigens are peptide antigens that are conjugated to the coat protein of said PapMV or VLP, and
wherein said adjuvant comprises additional PapMV or PapMV VLPs.

20. The composition according to claim 19, wherein said VLP comprises modified PapMV coat protein, said modified PapMV coat protein being capable of self-assembly to form said VLP.

21. The composition according to claim 19 or 20, wherein said one or more influenza virus antigens are genetically fused or covalently attached to said coat protein of the VLP.

22. The composition according to claim 19 or 20, wherein:
(a) the one or more influenza virus antigens are genetically fused at or proximal to the C-terminus of said coat protein, and/or
(b) at least one of said influenza virus antigens is an M2e peptide, or a fragment thereof.

23. The composition according to any one of claims 19, 20, 21 or 22, wherein said composition comprises a VLP comprising PapMV coat protein having an amino acid sequence substantially identical to the sequence as set forth in any one of SEQ ID Nos:1, 3 or 46.

24. The composition according to claim 22, wherein said composition comprises a VLP comprising PapMV coat protein having an amino acid sequence substantially identical to the sequence as set forth in any one of SEQ ID Nos:4, 47 or 48.

25. A composition according to any one of claims 19, 20, 21 22, 23 or 24 for use in inducing an immune response against an influenza virus in an animal.

26. The composition for use according to claim 25, wherein said immune response comprises a humoral immune response, a cellular immune response, or both.

27. The composition for use according to claim 25 or 26, wherein said animal is a human.

28. A polypeptide comprising a papaya mosaic virus (PapMV) coat protein fused to one or more influenza virus peptide antigens for use in the preparation of a composition according to claim 21, wherein said polypeptide is capable of self-assembly to form a VLP.

29. The polypeptide of claim 28 for the use according to claim 28, wherein:
(a) the peptide antigens are fused at or proximal to the C-terminus of said papaya mosaic virus coat protein, and/or
(b) at least one of said peptide antigens is an M2e peptide, or a fragment thereof.

30. The polypeptide of claim 29 for the use according to claim 28, wherein said polypeptide comprises an amino acid sequence substantially identical to the sequence as set forth in any one of SEQ ID NOs:4, 47 or 48.

31. A polynucleotide encoding the polypeptide of any one of claims 28, 29 or 30.

## Patentansprüche

1. Antigen-präsentierendes System, das ein oder mehrere Influenzavirus-Antigene zusammen mit einem Papaya Mosaic Virus (PapMV) oder einem VLP, das PapMV-Hüllprotein umfasst, umfasst, worin das genannte eine oder die genannten mehreren Influenzavirus-Antigene nicht zum genannten PapMV oder VLP konjugiert sind.

2. Antigen-präsentierendes System nach Anspruch 1, worin das genannte VLP modifiziertes PapMV-Hüllprotein umfasst, wobei das genannte modifizierte PapMV-Hüllprotein zur Selbstorganisation fähig ist, um das genannte VLP zu bilden.

3. Antigen-präsentierendes System nach Anspruch 1 oder 2, worin das genannte eine oder die genannten mehreren Influenzavirus-Antigene von einem kommerziell erhältlichen Influenza-Impfstoffpräparat umfasst werden.

4. Antigen-präsentierendes System nach einem der Ansprüche 1, 2 oder 3, worin mindestens eines der genannten Influenzavirus-Antigene von dem M2-Protein abstammt.

5. Antigen-präsentierendes System nach einem der Ansprüche 1, 2 oder 3, worin mindestens eines der genannten Influenzavirus-Antigene von dem Nucleoprotein (NP) abstammt.

6. Antigen-präsentierendes System nach einem der Ansprüche 1, 2, 3, 4 oder 5, worin das genannte Antigen-präsentierende System ein VLP umfasst, wobei das genannte VLP PapMV-Hüllprotein umfasst, das eine Aminosäuresequenz aufweist, die im Wesentlichen identisch mit der Sequenz ist, die in einer der SEQ.-ID Nr. 1, 3 oder 46 dargelegt ist.

7. Influenza-Impfstoffzusammensetzung, die ein oder mehrere der Antigenpräsentierenden Systeme nach einem der Ansprüche 1, 2, 3, 4, 5 oder 6 umfasst.

8. Antigen-präsentierendes System nach einem der Ansprüche 1, 2, 3, 4, 5 oder 6 für die Verwendung bei der Auslösung einer Immunantwort gegen ein Influenzavirus bei einem Tier.

9. Antigen-präsentierendes System für die Verwendung nach Anspruch 8, worin die genannte Immunantwort eine humorale Immunantwort umfasst.

10. Antigen-präsentierendes System für die Verwendung nach Anspruch 8 oder 9, worin die genannte Immunantwort eine zelluläre Immunantwort umfasst.

11. Antigen-präsentierendes System für die Verwendung nach einem der Ansprüche 8, 9 oder 10, worin das genannte Tier ein Mensch ist.

12. Antigen-präsentierendes System für die Verwendung nach einem der Ansprüche 8, 9 oder 10, worin das genannte Tier ein nichtmenschliches Tier ist.

13. Zusammensetzung, die ein Papaya Mosaic Virus (PapMV) oder ein VLP, das PapMV-Hüllprotein umfasst, umfasst, für die Verwendung zur Verbesserung der Wirksamkeit eines kommerziell erhältlichen Influenza-Impfstoffs bei der Auslösung einer Immunantwort bei einem Tier gegen ein Influenzavirus, worin die genannte Zusammensetzung und der genannte Influenza-Impfstoff gleichzeitig an das genannte Tier verabreicht werden.

14. Zusammensetzung für die Verwendung nach Anspruch 13, worin die genannte Immunantwort eine Antwort gegen NP-Protein umfasst.

15. Zusammensetzung für die Verwendung nach Anspruch 13 oder 14, worin die genannte Immunantwort gegen mehr als einen Stamm von Influenzavirus wirksam ist.

16. Zusammensetzung für die Verwendung nach einem der Ansprüche 13, 14 oder 15, worin die genannte Zusammensetzung ein VLP umfasst, das PapMV-Hüllprotein umfasst.

17. Zusammensetzung für die Verwendung nach Anspruch 16, worin das genannte VLP modifiziertes PapMV-Hüllprotein umfasst, wobei das genannte modifizierte PapMV-Hüllprotein zur Selbstorganisation fähig ist, um das genannte VLP zu bilden.

18. Zusammensetzung für die Verwendung nach Anspruch 16 oder 17, worin das genannte VLP PapMV-Hüllprotein umfasst, das eine Aminosäuresequenz aufweist, die im Wesentlichen identisch mit der Sequenz ist, die in einer der SEQ.-ID Nr. 1, 3 oder 46 dargelegt ist.

19. Zusammensetzung, die Folgendes umfasst:
ein oder mehrere Influenzavirus-Antigene zusammen mit einem Papaya Mosaic Virus (PapMV) oder einem VLP, das PapMV-Hüllprotein umfasst, und
einen Hilfsstoff,
worin das genannte eine oder die genannten mehreren Influenzavirus-Antigene Peptidantigene sind, die zu dem Hüllprotein des genannten PapMV oder VLP konjugiert sind, und
worin der genannte Hilfsstoff zusätzliches PapMV oder zusätzliche PapMV-VLPs umfasst.

20. Zusammensetzung nach Anspruch 19, worin das genannte VLP modifiziertes PapMV-Hüllprotein umfasst, wobei das genannte modifizierte PapMV-Hüllprotein zur Selbstorganisation fähig ist, um das genannte VLP zu bilden.

21. Zusammensetzung nach Anspruch 19 oder 20, worin das genannte eine oder die genannten mehreren Influenzavirus-Antigene mit dem genannten Hüllprotein des VLP genetisch fusioniert oder kovalent verknüpft sind.

22. Zusammensetzung nach Anspruch 19 oder 20, worin:
(a) das eine oder die mehreren Influenzavirus-Antigene am oder in der Nähe des C-Terminus des genannten Hüllproteins genetisch fusioniert sind, und/oder
(b) mindestens eines der genannten Influenzavirus-Antigene ein M2e-Peptid oder ein Fragment davon ist.

23. Zusammensetzung nach einem der Ansprüche 19, 20, 21 oder 22, worin die genannte Zusammensetzung ein VLP umfasst, das PapMV-Hüllprotein umfasst, das eine Aminosäuresequenz aufweist, die im Wesentlichen identisch mit der Sequenz ist, die in einer der SEQ.-ID Nr. 1, 3 oder 46 dargelegt ist.

24. Zusammensetzung nach Anspruch 22, worin die genannte Zusammensetzung ein VLP umfasst, das PapMV-Hüllprotein umfasst, das eine Aminosäuresequenz aufweist, die im Wesentlichen identisch mit der Sequenz ist, die in einer der SEQ.-ID Nr. 4, 47 oder 48 dargelegt ist.

25. Zusammensetzung nach einem der Ansprüche 19, 20, 21, 22, 23 oder 24 für die Verwendung bei der Auslösung einer Immunantwort gegen ein Influenzavirus bei einem Tier.

26. Zusammensetzung für die Verwendung nach Anspruch 25, worin die genannte Immunantwort eine humorale Immunantwort, eine zelluläre Immunantwort oder beides umfasst.

27. Zusammensetzung für die Verwendung nach Anspruch 25 oder 26, worin das genannte Tier ein Mensch ist.

28. Polypeptid, das ein Papaya Mosaic Virus (PapMV)-Hüllprotein umfasst, das mit einem oder mehreren Influenzavirus-Peptidantigenen fusioniert ist, für die Verwendung bei der Herstellung einer Zusammensetzung nach Anspruch 21, worin das genannte Polypeptid zur Selbstorganisation fähig ist, um ein VLP zu bilden.

29. Polypeptid nach Anspruch 28 für die Verwendung nach Anspruch 28, worin:
(a) die Peptidantigene am oder in der Nähe des C-Terminus des genannten Papaya Mosaic Virus-Hüllproteins fusioniert sind, und/oder
(b) mindestens eines der genannten Peptidantigene ein M2e-Peptid oder ein Fragment davon ist.

30. Polypeptid nach Anspruch 29 für die Verwendung nach Anspruch 28, worin das genannte Polypeptid eine Aminosäuresequenz umfasst, die im Wesentlichen identisch mit der Sequenz ist, die in einer der SEQ.-ID Nr. 4, 47 oder 48 dargelegt ist.

31. Polynukleotid, das das Polypeptid nach einem der Ansprüche 28, 29 oder 30 kodiert.

## Revendications

1. Système de présentation d'antigène, comprenant un ou plusieurs antigènes d'influenzavirus en combinaison avec un virus de la mosaïque de la papaye (PapMV) ou une PPV comprenant une protéine de la capside du PapMV, dans lequel lesdits un ou plusieurs antigènes d'influenzavirus ne sont pas conjugués audit PapMV ou à ladite PPV.

2. Système de présentation d'antigène selon la revendication 1, dans lequel ladite PPV comprend une protéine de la capside du PapMV modifiée, ladite protéine de la capside du PapMV modifiée étant capable d'auto-assemblage pour former ladite PPV.

3. Système de présentation d'antigène selon la revendication 1 ou 2, dans lequel lesdits un ou plusieurs antigènes d'influenzavirus sont constitués par une préparation de vaccin antigrippal disponible dans le commerce.

4. Système de présentation d'antigène selon l'une quelconque des revendications 1, 2 ou 3, dans lequel au moins un parmi lesdits antigènes d'influenzavirus est dérivé de la protéine M2.

5. Système de présentation d'antigène selon l'une quelconque des revendications 1, 2 ou 3, dans lequel au moins un parmi lesdits antigènes d'influenzavirus est dérivé de la nucléoprotéine (NP).

6. Système de présentation d'antigène selon l'une quelconque des revendications 1, 2, 3, 4 ou 5, dans lequel ledit système de présentation d'antigène comprend une PPV, ladite PPV comprenant une protéine de la capside du PapMV ayant une séquence d'acides aminés sensiblement identique à la séquence telle qu'exposée selon l'une quelconque parmi SEQ ID n° 1, 3 ou 46.

7. Composition de vaccin antigrippal, comprenant un ou plusieurs parmi les systèmes de présentation d'antigène selon l'une quelconque des revendications 1, 2, 3, 4, 5 ou 6.

8. Système de présentation d'antigène selon l'une quelconque des revendications 1, 2, 3, 4, 5, ou 6, pour une utilisation dans l'induction d'une réponse immunitaire contre un influenzavirus chez un animal.

9. Système de présentation d'antigène pour une utilisation selon la revendication 8, dans lequel ladite réponse immunitaire comprend une réponse immunitaire humorale.

10. Système de présentation d'antigène pour une utilisation selon la revendication 8 ou 9, dans lequel ladite réponse immunitaire comprend une réponse immunitaire cellulaire.

11. Système de présentation d'antigène pour une utilisation selon l'une quelconque des revendications 8, 9 ou 10, dans lequel ledit animal est un être humain.

12. Système de présentation d'antigène pour une utilisation selon l'une quelconque des revendications 8, 9 ou 10, dans lequel ledit animal est un animal non humain.

13. Composition comprenant un virus de la mosaïque de la papaye (PapMV) ou une PPV comprenant une protéine de la capside du PapMV, pour une utilisation destinée à améliorer l'efficacité d'un vaccin antigrippal disponible dans le commerce pour l'induction d'une réponse immunitaire chez un animal contre un influenzavirus, dans laquelle ladite composition et ledit vaccin antigrippal sont administrés de manière concomitante audit animal.

14. Composition pour une utilisation selon la revendication 13, dans laquelle ladite réponse immunitaire comprend une réponse contre la protéine NP.

15. Composition pour une utilisation selon la revendication 13 ou 14, dans laquelle ladite réponse immunitaire est efficace contre plus d'une souche d'influenzavirus.

16. Composition pour une utilisation selon l'une quelconque des revendications 13, 14 ou 15, dans laquelle ladite composition comprend une PPV comprenant une protéine de la capside du PapMV.

17. Composition pour une utilisation selon la revendication 16, dans laquelle ladite PPV comprend une protéine de la capside du PapMV modifiée, ladite protéine de la capside du PapMV modifiée étant capable d'auto-assemblage pour former ladite PPV.

18. Composition pour une utilisation selon la revendication 16 ou 17, dans laquelle ladite PPV comprend une protéine de la capside du PapMV ayant une séquence d'acides aminés sensiblement identique à la séquence telle qu'exposée selon l'une quelconque parmi SEQ ID n° 1, 3 ou 46.

19. Composition comprenant :
un ou plusieurs antigènes d'influenzavirus en combinaison avec un virus de la mosaïque de la papaye (PapMV) ou une PPV comprenant une protéine de la capside du PapMV, et
un adjuvant,
dans laquelle lesdits un ou plusieurs antigènes d'influenzavirus sont des antigènes peptidiques qui sont conjugués à la protéine de la capside dudit PapMV ou de ladite PPV, et
dans laquelle ledit adjuvant comprend des PapMV ou des PPV de PapMV supplémentaires.

20. Composition selon la revendication 19, dans laquelle ladite PPV comprend une protéine de la capside du de PapMV modifiée, ladite protéine de la capside du PapMV modifiée étant capable d'auto-assemblage pour former ladite PPV.

21. Composition selon la revendication 19 ou 20, dans laquelle lesdits un ou plusieurs antigènes d'influenzavirus sont génétiquement fusionnés ou fixés de manière covalente à ladite protéine de la capside de la PPV.

22. Composition selon la revendication 19 ou 20, dans laquelle
(a) les un ou plusieurs antigènes d'influenzavirus sont génétiquement fusionnés au niveau ou à proximité de l'extrémité C-terminale de ladite protéine de la capside, et/ou
(b) au moins un parmi lesdits antigènes d'influenzavirus est un peptide M2e, ou un fragment de celui-ci.

23. Composition selon l'une quelconque des revendications 19, 20, 21 ou 22, dans laquelle ladite composition comprend une PPV comprenant une protéine de la capside du PapMV ayant une séquence d'acides aminés sensiblement identique à la séquence telle qu'exposée selon l'une quelconque parmi SEQ ID n° 1, 3 ou 46.

24. Composition selon la revendication 22, dans laquelle ladite composition comprend une PPV comprenant une protéine de la capside du PapMV ayant une séquence d'acides aminés sensiblement identique à la séquence telle qu'exposée selon l'une quelconque parmi SEQ ID n° 4, 47 ou 48.

25. Composition selon l'une quelconque des revendications 19, 20, 21, 22, 23 ou 24, pour une utilisation dans l'induction d'une réponse immunitaire contre un influenzavirus chez un animal.

26. Composition pour une utilisation selon la revendication 25, dans laquelle ladite réponse immunitaire comprend une réponse immunitaire humorale, une réponse immunitaire cellulaire, ou les deux.

27. Composition pour une utilisation selon la revendication 25 ou 26, dans laquelle ledit animal est un être humain.

28. Polypeptide comprenant une protéine de la capside du virus de la mosaïque de la papaye (PapMV) fusionnée à un ou plusieurs antigènes peptidiques d'influenzavirus, pour une utilisation dans la préparation d'une composition selon la revendication 21, dans lequel ledit polypeptide est capable d'auto-assemblage pour former une PPV.

29. Polypeptide selon la revendication 28, pour l'utilisation selon la revendication 28, dans lequel
(a) les antigènes peptidiques sont fusionnés au niveau ou à proximité de l'extrémité C-terminale de ladite protéine de la capside du virus de la mosaïque de la papaye, et/ou
(b) au moins un parmi lesdits antigènes peptidiques est un peptide M2e, ou un fragment de celui-ci.

30. Polypeptide selon la revendication 29, pour l'utilisation selon la revendication 28, dans lequel ledit polypeptide comprend une séquence d'acides aminés sensiblement identique à la séquence telle qu'exposée selon l'une quelconque parmi SEQ ID n° 4, 47 ou 48.

31. Polynucléotide codant pour le polypeptide selon l'une quelconque des revendications 28, 29 ou 30.
